(19) Europäisches Patentamt
European Patent Office
Office européen des brevets

(11) EP 3 019 481 B1

(12) EUROPÄISCHE PATENTSCHRIFT

(45) Veröffentlichungstag und Bekanntmachung des Hinweises auf die Patenterteilung:
**06.03.2019 Patentblatt 2019/10**

(21) Anmeldenummer: **14734844.5**

(22) Anmeldetag: **04.07.2014**

(51) Int Cl.:
***C07D 239/557*** (2006.01) ***A01N 43/54*** (2006.01)
***A01N 43/707*** (2006.01) ***C07D 253/075*** (2006.01)
***A01P 7/00*** (2006.01)

(86) Internationale Anmeldenummer:
**PCT/EP2014/064352**

(87) Internationale Veröffentlichungsnummer:
**WO 2015/004028 (15.01.2015 Gazette 2015/02)**

# (54) SECHSGLIEDRIGE C-N-VERKNÜPFTE ARYLSULFID- UND ARYLSULFOXID- DERIVATE ALS SCHÄDLINGSBEKÄMPFUNGSMITTEL

SIX-MEMBERED C-N LINKED ARYLSULFIDE AND ARYLSULFOXIDE DERIVATIVES AS PESTICIDES

DÉRIVÉS D'ARYLSULFURE ET D'ARYLSULFOXYDE HEXAGONAUX LIÉS C-N COMME MOYENS DE LUTTE CONTRE LES PARASITES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.07.2013 EP 13175501**

(43) Veröffentlichungstag der Anmeldung:
**18.05.2016 Patentblatt 2016/20**

(73) Patentinhaber: **Bayer CropScience Aktiengesellschaft**
**40789 Monheim am Rhein (DE)**

(72) Erfinder:
- **ALIG, Bernd**
**53639 Königswinter (DE)**
- **CEREZO-GALVEZ, Silvia**
**40764 Langenfeld (DE)**
- **FISCHER, Reiner**
**40789 Monheim (DE)**
- **KÖHLER, Adeline**
**40764 Langenfeld (DE)**
- **HAHN, Julia Johanna**
**40597 Düsseldorf (DE)**
- **ILG, Kerstin**
**50670 Köln (DE)**
- **LÖSEL, Peter**
**51371 Leverkusen (DE)**
- **MALSAM, Olga**
**51503 Rösrath (DE)**
- **PORTZ, Daniela**
**52391 Vettweiß (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 1 803 712 DE-A1- 19 536 842**

EP 3 019 481 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Anmeldung betrifft neue heterocyclische Verbindungen, Verfahren zu Ihrer Herstellung und ihre Verwendung zur Bekämpfung von tierischen Schädlingen, zu denen Arthropoden und insbesondere Insekten zählen.

2-Aryl-1,2,4-triazine-3,5-dione (auch sogenannte 1-Aryl-6-azauracile) sind bereits in der Literatur mit herbizider, antiprotozoischer, anthelmintischer, antiinfektiver und insektizider Wirkung bekannt, beispielsweise in WO 8600072, WO 9730980 und WO 2005/112941.

Strukturell teilweise ähnliche 3-Triazolylphenylsulfid-Derivate, die insektizid, akarizid oder nematizid wirken, sind ferner aus EP 1803712 A1 bekannt.

2-Aryl-1,2,4-triazine-3,5-dithione sind in WO 9730980 als Herbizide offenbart. 6-Aryl-3-thioxo-5-(thi)oxo-1,2,4-triazine sind beispielsweise in WO 9959983 als Herbizide offenbart.

Gleich 1,3-disubstituierte 1,3,5-Triazin-2,4-dione sind beispielsweise in DE 2543497 und DE 2724673 mit insektizider Wirkung beschrieben.

Ferner sind verschiedene substituierte 1-Aryl- und 1-Pyridyl-uracile bekannt, die eine fungizide (GB 2021098) oder eine herbizide (DE 19528305) Wirkung aufweisen, oder die allgemein als Schädlingsbelkämpfungsmittel (DE 19536842) beschrieben sind.

Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I)

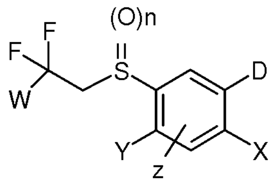
(I),

worin gilt:

D steht für eine Substruktur der Formel (I-A-1) oder (I-A-9)

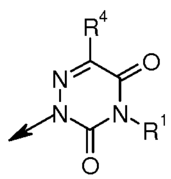
(I-A-1)

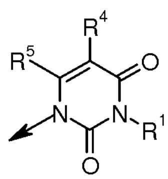
(I-A-9)

,

worin der Stickstoff mit dem Sechsring gemäß Formel (I) verbunden ist und der Pfeil die Bindung zu diesem Sechsring bedeutet,

$R^1$ steht für Wasserstoff;
oder für $(C_1-C_6)$Alkyl, $(C_3-C_8)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl, Phenyl$(C_1-C_3)$alkyl oder Pyridyl$(C_1-C_3)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Ha-

lo($C_1$-$C_6$)alkylsulfonyl oder gegebenenfalls durch Methyl, Fluor, Chlor, Cyano substituiertes Cyclopropyl substituiert sein können und wobei das in Pyridyl($C_1$-$C_3$)alkyl vorhandene N-Atom auch als N-Oxid vorliegen kann;
oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridizanyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können und wobei das in Pyridyl vorhandene N-Atom auch als N-Oxid vorliegen kann;

$R^4$ und $R^5$ stehen unabhängig voneinander
für Wasserstoff, Halogen, ($C_1$-$C_4$)Alkyl, ($C_1$-$C_4$)Halogenalkyl, Cyano($C_1$-$C_6$)alkyl, ($C_3$-$C_6$)Cycloalkyl, Cyano oder Carboxyl;

$R^{11}$ steht für Wasserstoff, ($C_1$-$C_4$)Alkyl, ($C_1$-$C_4$)Halogenalkyl, Cyano($C_1$-$C_6$)alkyl, oder ($C_3$-$C_6$)Cycloalkyl; wobei $R^{11}$ insbesondere für Wasserstoff, Methyl, Ethyl, *tert*-Butyl, Trifluoromethyl, Methoxy, Trifluoromethoxy oder Cyclopropyl steht;

W steht für Wasserstoff oder Fluor;

n steht für die Zahl 0 oder 1;

X und Y stehen unabhängig voneinander für Wasserstoff, Fluor, Chlor, Brom, Methyl, Ethyl, Trifluoromethyl, Difluoromethyl, Difluoromethoxy, Trifluoromethoxy, Cyclopropyl, Cyano, Amino, Hydroxy oder Nitro;

Z steht für Wasserstoff.

Weiterhin wurde gefunden, dass die neuen Verbindungen der Formel (I) eine gute Wirksamkeit als Schädlingsbekämpfungsmittel, beispielsweise gegen Arthropoden und insbesondere Insekten und Akariden, darüber hinaus in der Regel insbesondere gegenüber Kulturpflanzen sehr gut pflanzenverträglich sind und/oder über günstige toxikologische und/oder umweltrelevante Eigenschaften verfügen.

Bevorzugte Substituenten bzw. Bereiche der in den Verbindungen der Formel (I) aufgeführten Reste werden im Folgenden erläutert.

D steht für eine Substruktur der Formel (I-A-1) oder (I-A-9);

$R^1$ steht für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, $CH_2CCH$, Cyclopropylmethyl, Trifluoroethyl, (2,2)-Difluoroethyl, $CH_2CN$, $CH_2OCH_3$, $CH_2CH_2OCH_3$, Benzyl, Cyclopropyl, Cyclobutyl, Phenyl, 2-Fluorophenylmethyl, 3-Fluorophenylmethyl, 4-Fluorophenylmethyl, 2-Pyridylmethyl, N-Oxid-2-pyridylmethyl, 3-Pyridylmethyl, N-Oxid-3-pyridylmethyl, 4-Pyridylmethyl oder N-Oxid-4-pyridylmethyl;

$R^4$ und $R^5$ stehen unabhängig voneinander
für Wasserstoff, Methyl, Ethyl, *tert*-Butyl, Trifluoromethyl, Methoxy, Trifluoromethoxy, Cyclopropyl, Cyano oder Carboxyl;

W steht für Wasserstoff oder Fluor;

n steht für die Zahl 0 oder 1;

X steht für Wasserstoff, Chlor, Fluor, Methyl oder Ethyl;

Y steht für Chlor, Brom, Cyano, Methyl, Ethyl, Trifluoromethyl, Fluor oder Methoxy;
Z steht für Wasserstoff.

Weiter bevorzugt innerhalb dieser bevorzugten Substituenten bzw. Bereiche ist, dass X und Y für folgende

Kombinationen (Y,X) stehen: (Me, F), (Me,H), (Me,Cl), (Me,Me), (Et, Et), (Cl,Cl), (Cl,F), (CN,F), (MeO,F), (MeO,H), (Cl,H), (Me,H), (Br,H), (Br,F), (CN,H), (F,F), ($CF_3$,H).

Ganz besonders bevorzugte Substituenten bzw. Bereiche der in den Verbindungen der Formel (I) aufgeführten Reste werden im Folgenden erläutert.

D steht für eine Substruktur der Formel (I-A-1) oder (I-A-9);

$R^1$ steht für Wasserstoff, Methyl, Trifluoroethyl;

$R^4$ und $R^5$ stehen unabhängig voneinander für Wasserstoff, Cyano oder Methyl;

W steht für Wasserstoff oder Fluor;

n steht für die Zahl 0 oder 1;

X steht für Chlor, Fluor, Methyl oder Ethyl;

Y steht für Chlor, Methyl oder Ethyl;

Z steht für Wasserstoff.

Weiter bevorzugt innerhalb dieser ganz besonders bevorzugten Substituenten bzw. Bereiche ist, dass X und Y für folgende Kombinationen (Y,X) stehen: (Me, F), (Me,Me), (Cl,Cl), (Et,Et).

Ebenfalls ganz besonders bevorzugte Substituenten bzw. Bereiche der in den Verbindungen der Formel (I) aufgeführten Reste werden im Folgenden erläutert.

D steht für eine Substruktur der Formel (I-A-1) oder (I-A-9);

$R^1$ steht für Wasserstoff, Methyl, Ethyl, n-Propyl, $CH(CH_3)_2$, $CH_2CH(CH_3)_2$, $CH_2CH=CH_2$, $CH_2CCH$, Cyclopropylmethyl, Trifluoroethyl, (2,2)-Difluoroethyl, $CH_2CN$, $CH_2OCH_3$, Benzyl, 4-Fluorophenylmethyl, 3-Pyridylmethyl oder N-Oxid-3-pyridylmethyl;

$R^4$ und $R^5$ stehen unabhängig voneinander für Wasserstoff, Cyano oder Methyl;

W steht für Wasserstoff oder Fluor;

n steht für die Zahl 0 oder 1;

X steht für Wasserstoff, Chlor, Fluor, Methyl oder Ethyl;

Y steht für Chlor, Methyl oder Ethyl;

Z steht für Wasserstoff.

Weiter bevorzugt innerhalb dieser ganz besonders bevorzugten Substituenten bzw. Bereiche ist, dass X und Y für folgende Kombinationen (Y,X) stehen: (Me, F), (Me,Me), (Cl,Cl), (Et,Et), (Me,H), (Cl,H).

Wenn in obigen Definitionen in Ringen Schwefel und/oder Stickstoff vorkommen, wie beispielsweise in Ausdrücken wie "in welchen die Ringe mindestens ein Heteroatom aus der Reihe Schwefel, Sauerstoff (wobei Sauerstoffatome nicht unmittelbar benachbart sein dürfen) und Stickstoff enthalten können" oder "in welchen ein oder zwei Ringglieder jeweils durch ein Heteroatom aus der Reihe Schwefel, Sauerstoff (wobei Sauerstoffatome nicht unmittelbar benachbart sein dürfen) und Stickstoff ersetzt sein können", dann kann, sofern nichts anderes angegeben ist, der Schwefel auch als SO oder $SO_2$ vorliegen, der Stickstoff, sofern er nicht als -N= vorliegt, neben NH auch als N-Alkyl (insbesondere N-$C_1$-$C_6$-Alkyl) vorliegen.

In den vorstehenden Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom, sowie

Hetaryl (gleichbedeutend mit Heteroaryl, auch als Teil einer größeren Einheit, wie beispielsweise Hetarylalkyl) ausgewählt aus der Reihe Pyridyl, Pyrimidinyl, Pyridazinyl und Pyrazinyl, bevorzugt Pyridyl.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte (die Verbindungen der Formel (I) mit den aufgeführten Substrukturen (I-A-1) und (I-A-9)) und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt, wobei jede vorstehend als bevorzugt beschriebene Ausgestaltung eine eigenständige Kombination darstellt.

Erfindungsgemäß ganz besonders bevorzugt werden Verbindungen der Formel (I), in welchen eine Kombination der vorstehend als ganz besonders bevorzugt aufgeführten Bedeutungen vorliegt, wobei jede vorstehend als ganz bevorzugt beschriebene Ausgestaltung eine eigenständige Kombination darstellt.

In einer weiteren bevorzugten Ausgestaltung betrifft die Erfindung Verbindungen der Formel (I), in der D für eine Substruktur der Formel (I-A-1) steht:

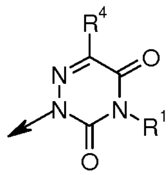

(I-A-1)

In einer weiteren bevorzugten Ausgestaltung betrifft die Erfindung Verbindungen der Formel (I), in der D für eine Substruktur der Formel (I-A-9) steht:

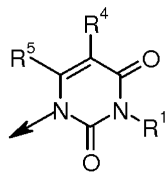

(I-A-9)

In den durch die Strukturen (I-A-1) und (I-A-9) definierten Verbindungen der Formel (I) haben die Reste bzw. Strukturelemente $R^1$, $R^4$, $R^5$, W, n, X, Y und Z die weiter oben genannten Bedeutungen.

Insbesondere stehen X und Y für folgende Kombinationen (Y,X): (Me, F), (Me,H), (Me,Cl), (Me,Me), (Et, Et), (Cl,Cl), (Cl,F), (CN,F), (MeO,F), (MeO,H), (Cl,H), (Br,H), (Br,F), (CN,H), (F,F), ($CF_3$,H), wobei die folgenden Kombinationen (Y,X) besonders bevorzugt sind: (Me, F), (Me,Me), (Cl,Cl), (Et,Et), (Me,H), (Cl,H).

Bevorzugt innerhalb der Ausführungsformen, in denen D für eine Substruktur der Formel (I-A-1) oder (I-A-9) steht, werden diejenigen Verbindungen, in welchen eine Kombination der vorstehend als bevorzugt aufgeführten Bedeutungen vorliegt, wobei jede vorstehend als bevorzugt beschriebene Ausgestaltung eine eigenständige Kombination darstellt.

Ganz besonders bevorzugt innerhalb der Ausführungsformen, in denen D für eine Substruktur der Formel (I-A-1) oder (I-A-9) steht, werden diejenigen Verbindungen, in welchen eine Kombination der vorstehend als ganz besonders bevorzugt aufgeführten Bedeutungen vorliegt, wobei jede vorstehend als ganz bevorzugt beschriebene Ausgestaltung eine eigenständige Kombination darstellt. Hierin steht insbesondere bei Ausführungsformen, in welchen D für eine Substruktur der Formel (I-A-1) steht,

$R^1$ für Wasserstoff, Methyl oder Trifluoroethyl;

$R^4$ für Wasserstoff;

W für Fluor;

n für die Zahl 0 oder 1;

X für Chlor, Fluor oder Methyl;

Y für Chlor oder Methyl;
wobei X und Y insbesondere für folgende Kombinationen (Y,X) stehen: (Me, F), (Me,Me), (Cl,Cl);

Z für Wasserstoff,

oder

$R^1$ für Wasserstoff, Methyl, Ethyl, $CH(CH_3)_2$, $CH_2CH(CH_3)_2$, Cyclopropylmethyl, Trifluoroethyl, $CH_2CN$, Benzyl, 3-Pyridylmethyl oder N-Oxid-3-pyridylmethyl;

$R^4$ für Wasserstoff;

W für Fluor;

n für die Zahl 0 oder 1;

X für Wasserstoff, Chlor, Fluor oder Methyl;

Y für Chlor oder Methyl;
wobei X und Y insbesondere für folgende Kombinationen (Y,X) stehen: (Me, F), (Me,Me), (Cl,Cl), (Me,H), (Cl,H);

Z für Wasserstoff,

und bei Ausführungsform, in welchen D für eine Substruktur der Formel (I-A-9) steht,

$R^1$ für Wasserstoff;
$R^4$ für Cyano;
$R^5$ für Methyl;
W für Fluor;
n für die Zahl 0 oder 1;
X für Fluor oder Ethyl;
Y für Methyl oder Ethyl;
wobei X und Y insbesondere für folgende Kombinationen (Y,X) stehen: (Me, F), (Et,Et);
Z für Wasserstoff,

oder

$R^1$ für Wasserstoff, Methyl, Ethyl, n-Propyl, $CH(CH_3)_2$, $CH_2CH(CH_3)_2$, $CH_2CH=CH_2$, $CH_2CCH$, Cyclopropylmethyl, Trifluoroethyl, (2,2)-Difluoroethyl, $CH_2CN$, $CH_2OCH_3$ oder 4-Fluorophenylmethyl;
$R^4$ für Wasserstoff oder Cyano;
$R^5$ für Wasserstoff oder Methyl;
W für Fluor;
n für die Zahl 0 oder 1;
X für Wasserstoff, Fluor, Methyl oder Ethyl;
Y für Methyl oder Ethyl;
wobei X und Y insbesondere für folgende Kombinationen (Y,X) stehen: (Me, F), (Et,Et), (Me,Me), (Me,H);
Z für Wasserstoff.

Die erfindungsgemäßen Verbindungen der Formel (I) und deren Säureadditionssalze und Metallsalzkomplexe besitzen gute Wirksamkeit, insbesondere zur Bekämpfung von tierischen Schädlingen, zu denen Arthropoden und

insbesondere Insekten und Akariden zählen.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten und Akariden zählen. Eine individuelle Ausgestaltung der Erfindung ist daher auf das Vorliegen des R-Enantiomers gerichtet bzw. auf ein Gemisch, welches mehrheitlich das R-Enantiomer umfasst, vorzugsweise wobei das Verhältnis von R- zu S-Enantiomer mindestens 60:40 und zunehmend bevorzugt mindestens 70:30, 75:25, 80:20, 85:15 und 90:10 beträgt. Eine weitere individuelle Ausgestaltung der Erfindung ist daher auf das Vorliegen des S-Enantiomers gerichtet bzw. auf ein Gemisch, welches mehrheitlich das S-Enantiomer umfasst, vorzugsweise wobei das Verhältnis von S-zu R-Enantiomer mindestens 60:40 und zunehmend bevorzugt mindestens 70:30, 75:25, 80:20, 85:15 und 90:10 beträgt.

Die erfindungsgemäßen Verbindungen der Formel (I) können in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die erfindungsgemäßen Verbindungen sind durch die Formel (I) allgemein definiert und die beinhaltet auch alle möglichen Rotamere und Gemische davon.

Die erfindungsgemässen Verbindungen der Formel (I) können nach üblichen, dem Fachmann bekannten Methoden hergestellt werden. Verschiedene Herstellungsmethoden, die gleichfalls Gegenstand der Erfindung sind, sind in den folgenden Verfahren V1, V2, V3, V3', Va1, Va2, Va3, Va4 und Va5 beschrieben.

## Herstellverfahren

Die Verbindungen der allgemeinen Formel (I) lassen sich in Verbindungen mit n=0 (Ia) und n=1 (Ib) unterteilen und können prinzipiell nach den allgemeinen Verfahren V1, V2, V3 und V3' hergestellt werden.

Unter **Verfahren V1** lassen sich alle Methoden zusammenfassen, die -meistens in einem mehrstufigen Prozesseinen Aufbau des 5-gliedrigen Ringes ermöglichen, insbesondere ausgehend aus den Anilinen der allgemeinen Formel (IVa), nach folgendem Schema,

VERFAHREN V1

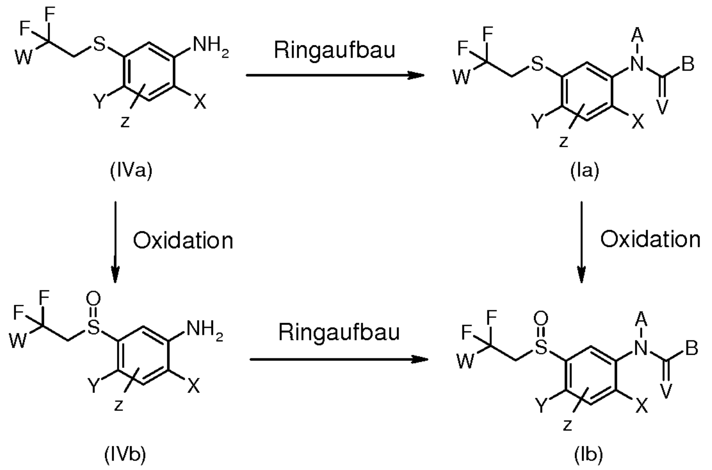

(IVa) (Ia) (IVb) (Ib)

wobei X, Y und Z die oben angegebene Bedeutung haben und A, B und V zusammen mit den Atomen, an die sie gebunden sind, für eine Substruktur D wie oben angegeben stehen.

Durch Oxidation der Thioether der allgemeinen Formel (Ia) nach literaturbekannten Methoden können die Sulfoxide der allgemeinen Formel (Ib) erhalten werden.

Alternativ können einige der in Verfahren V1 beschriebenen Methoden auch ausgehend von Sulfoxiden der allgemeinen Formel (IVb) zu den Sulfoxiden der allgemeinen Formel (Ib) angewandt werden. Die Sulfoxide der Formel (IVb) können aus den Sulfiden (IVa) nach literaturbekannten Methoden hergestellt werden.

Alternativ können die Verbindungen der allgemeinen Formel (Ia) nach **Verfahren V2** in hergestellt werden, nach folgendem Schema,

VERFAHREN V2

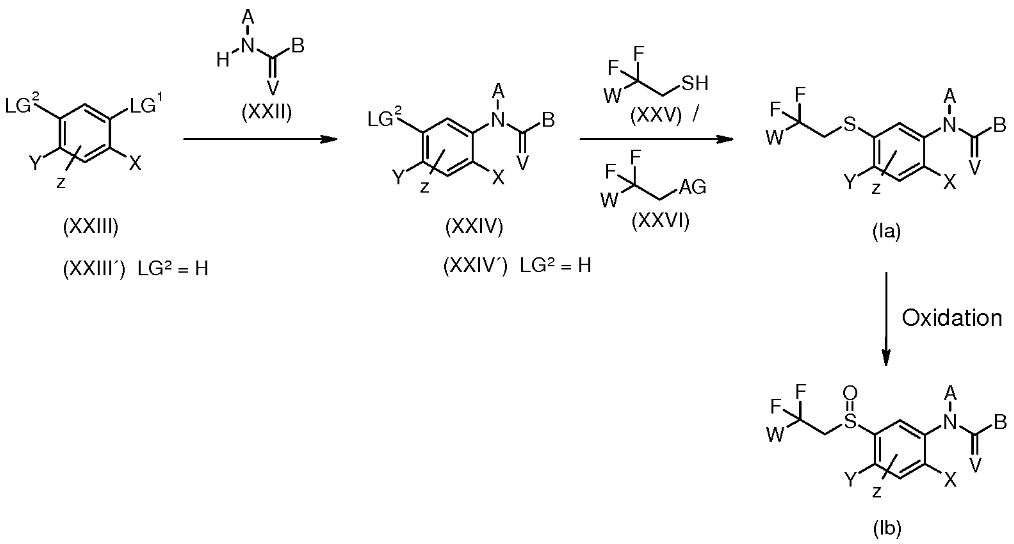

(XXII) (XXIII) (XXIII´) $LG^2 = H$ (XXIV) (XXIV´) $LG^2 = H$ (XXV) / (XXVI) (Ia) (Ib)

wobei Z und W die oben angegebene Bedeutung haben und A, B und V zusammen mit den Atomen, an die sie gebunden sind, für eine Substruktur D wie oben angegeben stehen, X steht für Wasserstoff oder eine elektronenziehende Gruppe (insbesondere Nitro, Chlor, Fluor, Cyano), Y stellt elektronenziehende Substituenten (insbesondere Nitro, Chlor, Fluor, Cyano) dar, $LG^1$ steht für typische Abgangsgruppen in nucleophilen Substitutionsreaktionen (insbesondere Fluorid, Chlorid) und $LG^2$ kann für Wasserstoff oder für typische Abgangsgruppen in nucleophilen Substitutionsreaktionen (insbesondere Fluorid, Chlorid) stehen.

Die Umsetzung von Verbindungen der allgemeinen Formel (XXIII) mit heterocyclischen Verbindungen der allgemeinen Formel (XXII), meistens unter basischen Reaktionsbedingungen wie zum Beispiel in DE 4431218 für Pyrimidin(thi)one, WO 2009/012275 und WO 2008/155034 für Pyridone oder DE 19528305 für Uracile beschrieben, liefert die Verbindungen der allgemeinen Formel (XXIV). Durch erneute nukleophile aromatische Substitution mit Thiolen der

allgemeinen Formel (XXV) können die Thioether der Formel (Ia) hergestellt werden. Geeignete Reaktionsbedingungen für solche Reaktionen werden in WO 2007/131680 und WO 2008/086226 beschrieben.

Durch Oxidation der Thioether der allgemeinen Formel (Ia) nach literaturbekannten Methoden können die Sulfoxide der allgemeinen Formel (Ib) erhalten werden.

Verbindungen der Formel (XXIII) in denen $LG^2$ für Wasserstoff steht werden als (XXIII') genannt und können auf ähnlicher Weise wie oben dargestellt mit Verbindungen der Formel (XXII) umgesetzt werden, in diesem Fall zu Verbindungen der Formel (XXIV').

Verbindungen der Formel (XXIV') sind zum Teil kommerziell erhältlich.

Die Verbindungen der Formel (XXIV') können in einem mehrstufigen Prozess zu den erfindungsgemäßen Verbindungen (Ia) umgesetzt werden. Zu den benötigten Schritten gehören Chlorsulfonierung, Reduktion und Alkylierung mit Haloalkylelektrophilen der Formel (XXVI), alle möglich nach literaturbekannten Methoden. Die Chlorsulfonierung der Verbindungen (XXIV') mit Chlorsulfonsäure liefert die entsprechenden Sulfonylchloride und diese können in deren Disulfide mit literaturbekannten Methoden wie zum Beispiel Eisen in Salzsäure oder Iodid überführt werden. Die Umsetzung der Disulfide mit Haloalkylelektrophilen der Formel (XXVI) liefert die Sulfide (Ia).

Durch Oxidation der Thioether der allgemeinen Formel (Ia) nach literaturbekannten Methoden können die Sulfoxide der allgemeinen Formel (Ib) erhalten werden.

Verfahren V2 eignet sich insbesondere zur Darstellung der Ausführungsform I-A, bei denen X für Wasserstoff oder elektronenziehende Substituenten (insbesondere Nitro, Chlorid, Fluorid, Cyano) und Y für elektronenziehende Substituenten (insbesondere Nitro, Chlorid, Fluorid, Cyano) stehen.

Alternativ können die Verbindungen der allgemeinen Formel (Ia) nach **Verfahren V3 und V3'** hergestellt werden, wie im folgendem Schema dargestellt,

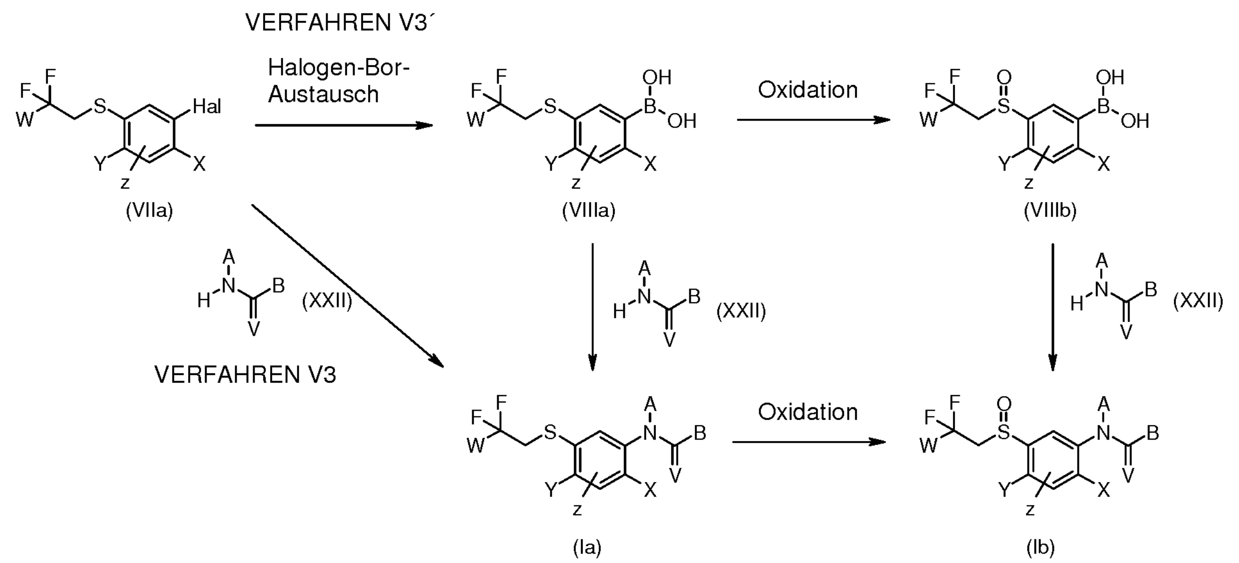


(VIIa) (VIIIa) (VIIIb) (Ia) (Ib)

wobei X, Y, Z und W die oben angegebene Bedeutung haben und A, B und V zusammen mit den Atomen, an die sie gebunden sind, für eine Substruktur D wie oben angegeben stehen, und Hal steht für Halogen (bevorzugt Chlor, Brom Iod).

Laut Verfahren V3 können Verbindungen der allgemeinen Formel (Ia) nach literaturbekannten Methoden durch Umsetzung von Arylhalogeniden der allgemeinen Formel (VIIa) mit heterocyclischen Verbindungen der allgemeinen Formel (XXII) hergestellt werden. Bevorzugt findet die Umsetzung durch Übergangsmetall-Katalyse oder -Vermittlung statt. Beispielhafte Reaktionsbedingungen sind in der Literatur zahlreich bekannt, zum Beispiel in WO 2006/117657 A1, in US 2010/99725 A1 oder in WO 2010/47956 A1. Bevorzugt werden Kupfer oder Kupfersalze, zum Beispiel Kupfer(I)-iodid, Kupfer(I)-oxid, Kupfer(I)-triflat oder Kupfer(II)-triflat, als Katalysator verwendet, häufig in Gegenwart eines Liganden, zum Beispiel Diamin-Liganden wie *N,N'*-Dimethylethylendiamin, *N,N*-Dimethylethylendiamin oder *trans-N,N'*-Dimethyl-1,2-cyclohexandiamin. Eine Übersicht findet sich zum Beispiel in Chem. Sci. 2010, Vol. 1, 13-31. Alternativ können 1,3-Diketone, wie z. B. 2,4-Pentandion, 2,2,6,6-Tetramethyl-3,5-heptandion oder Dibenzoylmethan, Aminosäuren, wie z.B. L-Prolin oder Glycin oder andere Verbindungen wie 8-Hydroxychinolin (Tetrahedron Lett. 2009, Vol. 50, 7293-7296), Dibenzylidenaceton, Bipyridin oder Phenanthrolin Verwendung finden. In der Regel wird die Umsetzung unter Anwesenheit einer Base, häufig Carbonat- oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid durchgeführt. Desweiteren können Additive wie z. B. Kaliumiodid, Cäsiumfluorid oder andere Salze Einsatz finden.

Alternativ lassen sich derartige Umsetzungen unter Palladiumkatalyse durchführen, etwa durch Einsatz von Katalysatoren wie zum Beispiel Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl, 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid.

Verbindungen der allgemeinen Formel (Ia) können alternativ nach Verfahren V3' durch Umsetzung von Boronsäuren der allgemeinen Formel (VIIIa) mit heterocyclischen Verbindungen der allgemeinen Formel (XXII) hergestellt werden.

In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel mit Molekularsieb) statt. Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N,N*-Dimethylformamid, Tetrahydrofuran, Dioxan, Acetonitril, Essigester oder Toluol verwendet. In der Literatur werden zahlreiche Beispiele beschrieben, unter anderem in US 6673810 oder Synthesis 2001, 6, 829-856 (für Uracile). Zusammenfassende Übersichten finden sich zum Beispiel in Synthesis 2011, No. 6, 829-856 oder in Tetrahedron 2012, Vol. 68, 7735-7754. Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Durch Oxidation der Thioether der allgemeinen Formel (Ia) nach literaturbekannten Methoden können die Sulfoxide der allgemeinen Formel (Ib) erhalten werden. Alternativ kann die oxidative übergangsmetallvermittelte Kohlenstoff-Stickstoff-Kupplung zu Arylsulfoxiden der allgemeinen Formel (Ib) ausgehend von Boronsäure-Sulfoxiden der allgemeinen Formel (VIIIb), welche durch Oxidation der Boronsäuren (VIIIa), zum Beispiel mit Natriumperiodat, oder analoger Derivate zugänglich sind, ermöglicht werden.

<u>Verfahren Va1</u> **(für I-A-I, $V^1$=$V^2$=O)**

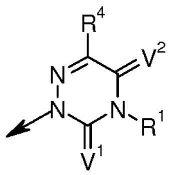

(I-A-I)

6-Azauracile der allgemeinen Formel (I-A-I) und damit auch erfindungsgemäße Verbindungen der Formel (I-A-1) können in (I-A-Ia) (für n=0) und (I-A-Ib) (für n=1) unterteilt und beispielsweise nach **Verfahren Va1** hergestellt werden, wie im folgenden Schema,

VERFAHREN Va1

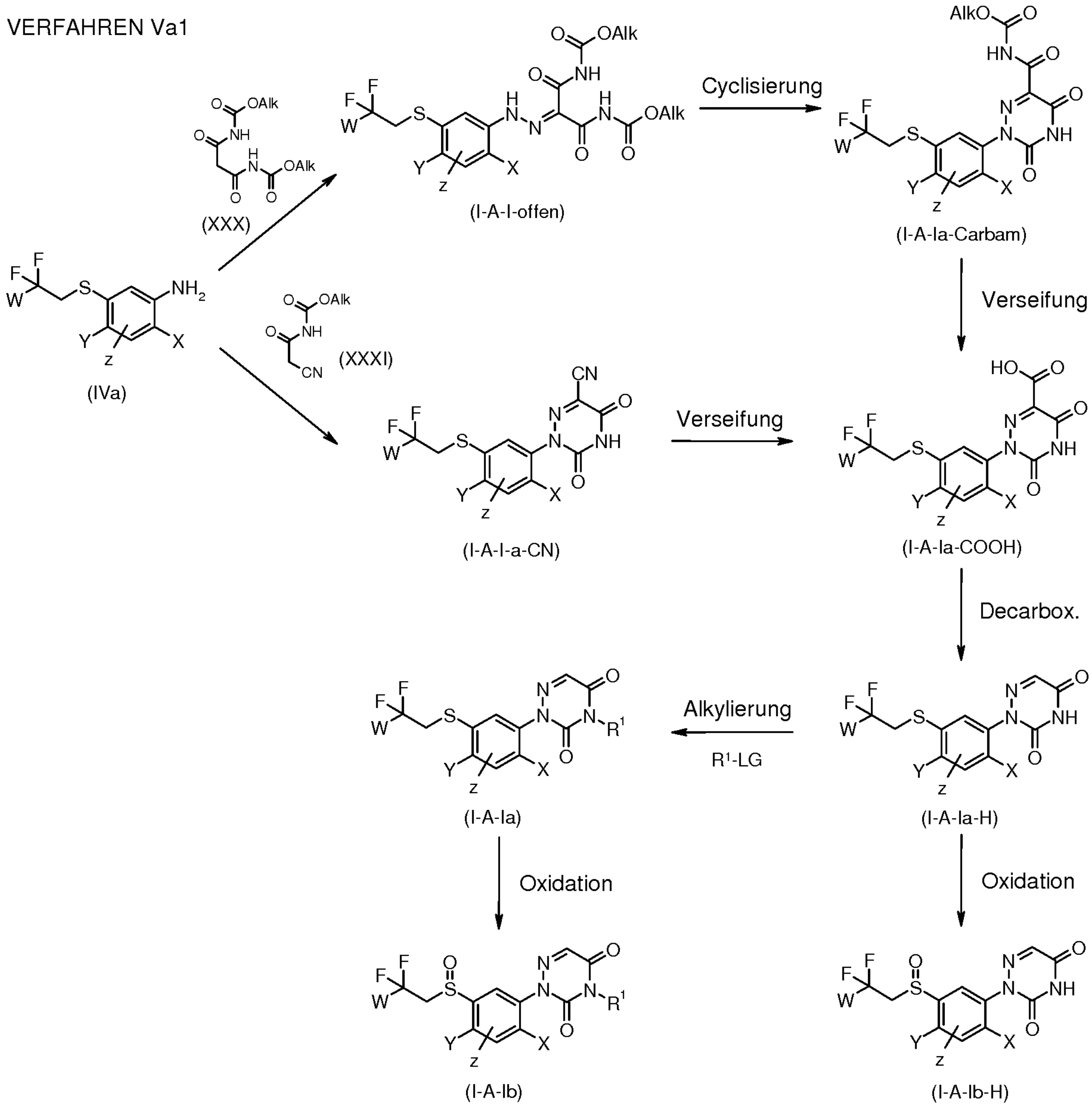


wobei X, Y, Z, W und $R^1$ die oben angegebene Bedeutung haben, Alk für eine kleine Alkylgruppe (insbesondere für Methyl und Ethyl) und LG für eine typische Abgangsgruppe in nucleophilen Substitutionsreaktionen (insbesondere Bromid, Iodid, Triflat, Mesylat) oder in Mitsunobus Reaktionen (insbesondere Hydroxy) steht.

Verbindungen der allgemeinen Formel (I-A-Ia) und (I-A-Ia-H), wo für $R^1$ = Wasserstoff steht, können nach Verfahren Va1 in einem mehrstufigen Prozess aus den Anilinen (IVa) hergestellt werden.

Als erster Syntheseschritt können Aniline der Formel (IVa) durch Diazotierung (beispielsweise durch Umsetzung mit Natriumnitrit in wässriger Salzsäure) und anschließend Reaktion mit Malonyldiurethane der Formel (XXX) zu den Hydrazonen der Formel (I-A-I-offen) umgewandelt werden, zum Beispiel in einer wässrigen Natriumacetatlösung. Diese Intermediate (I-A-I-offen) lassen sich dann, zum Beispiel durch Behandlung mit Natriumacetat in Essigsäure unter Hitzeeinwirkung, in die 6-Azauracile der Formel (I-A-Ia-Carbam) umsetzen. Durch Verseifung dieser, beispielsweise in wässriger Salzsäure, kann man die Carbonsäuren (I-A-I-COOH) erhalten.

Alternativ können 6-Azauracil-5-carbonsäuren (I-A-I-COOH) aus den entsprechenden Nitrilen (I-A-I-CN) durch Verseifung erhalten werden. Diese lassen sich aus den Anilinen (IVa) herstellen, zum Beispiel wenn sie nach der Diazotierungsreaktion mit *N*-Cyanacetylurethan (XXXI), mit Alk = Ethyl, umgesetzt werden, unter ähnlichen Reaktionsbedingungen wie die oben genannten für die Reaktion mit Malonyldiurethanen.

Zahlreiche Vorschriften zu Synthese und Cyclisierungen von Hydrazonen finden sich in der Literatur, zum Beispiel in Monatshefte Chem. 1968, 99(3), 1009-1013, Coll. Czech. Chem. Comm. 1979, 44(8), 2438-2442 und Pharmazie 1980, 35(12), 744-745, alle von J. Slouka. Beispiele von Reaktionsbedingungen für Verseifungen der Carbamate in (I-A-Ia-Carbam) oder Nitrile in (I-A-Ia-CN) finden sich in WO 9800072 oder US 7015230.

Zur Darstellung der 6-Azauracile der Formel (I-A-Ia-H) können Carbonsäuren (I-A-I-COOH) decarboxyliert werden, beispielsweise unter Hitzeeinwirkung in einem inerten, hoch-siedenden Lösungsmittel wie z.B. Diphenylether nach Heterocycles 1978, 9(10), 1387-1390, oder Mercaptoessigsäure nach J. Med. Chem. 1979, 22(12), 1483-1487.

Die erfindungsgemäßen 3-substituierten 6-Azauracile der Formel (I-A-Ia) können aus den unsubstituierten (I-A-Ia-H) durch Reaktion mit geeigneten Mitteln hergestellt werden. Zum Beispiel für die *N*-Alkylierung können Alkylierungsmittel der Formel $R^1$-LG in Anwesenheit einer geeigneten Base, gegebenenfalls in einem inerten Lösungs- oder Verdünnungsmittel, eingesetzt werden. Zahlreiche Vorschriften finden sich in der Literatur, zum Beispiel in WO 9800072 für Alkylhalogenide (LG = Halogen), in WO 2003/042191 mit Diazomethyltrimethylsilan für $R^1$ = Methyl oder in WO 2003/011841 für Alkohole (LG = OH) in Mitsunobus Reaktion, beschrieben.

Durch Oxidation der Thioether der allgemeinen Formel (I-A-Ia) bzw. (I-A-Ia-H) nach literaturbekannten Methoden können die Sulfoxide der allgemeinen Formel (I-A-Ib) bzw. (I-A-Ib-H) erhalten werden.

**Verfahren Va3 (für I-A-III)**

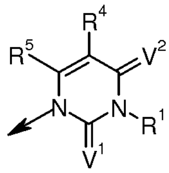

(I-A-III)

Die Pyrimidin-2,4(*1H,3H*)-(di)(thi)one (auch (Thio)Uracile genannt) der allgemeinen Formel (I) und damit auch erfindungsgemäße Verbindungen der Formel (I-A-9) können in (I-A-IIIa) (für n=0) und (I-A-IIIb) (für n=1) unterteilt und beispielsweise durch das in den folgenden Schema dargestellte **Verfahren Va3** erhalten werden,

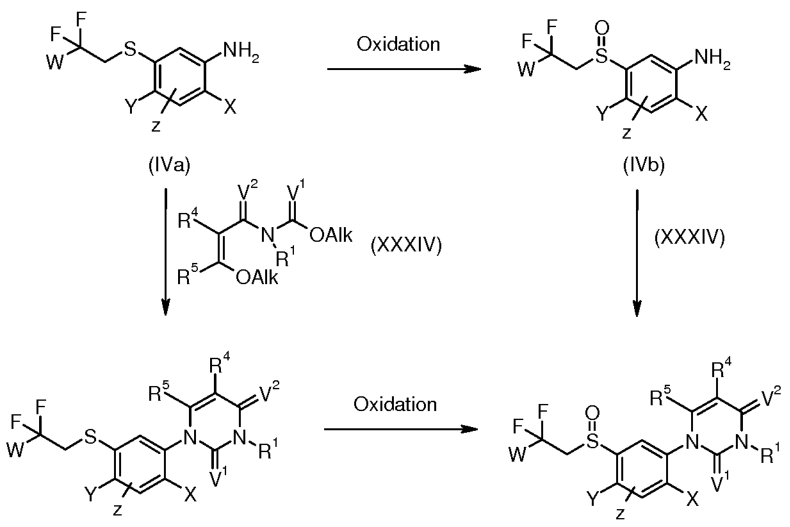


(IVa) (IVb) (I-A-IIIa) (I-A-IIIb)

wobei $R^1$, $R^4$, $R^5$, W, X, Y und Z die oben angegebene Bedeutung haben, $V^1$ und $V^2$ für Sauerstoff stehen, und Alk für eine kleine Alkylgruppe (insbesondere Methyl oder Ethyl) steht. Aniline der Formel (IVa) können mit Carbamaten der allgemeinen Formel (XXXIV), ohne Zwischenisolierung der intermediär entstehenden 3-Anilino-2-cyanalk-2-enoyl-(thio)carbamate, zu den erfindungsgemäßen Verbindungen der Formel (I-A-IIIa) umgesetzt werden.

Durch Oxidation der Thioether der allgemeinen Formel (I-A-IIIa) nach literaturbekannten Methoden können die erfindungsgemäßen Sulfoxide der allgemeinen Formel (I-A-IIIb) erhalten werden.

Alternativ können die Aniline der allgemeinen Formel (IVb), die aus deren Thioethervorstufen (IVa) nach bekannten Oxidationsmethoden hergestellt werden können, durch die Umsetzung mit Carbamaten der allgemeinen Formel (III) zu den erfindungsgemäßen Verbindungen der Formel (I-A-IIIb) umgesetzt werden.

Das im Verfahren Va3 benutzte Synthesekonzept - die Umsetzung von Aminen mit Carbamaten zu substituierten

Uracilen - gegebenenfalls mit der vorherigen Isolierung von offenkettigen Zwischenstufen und deren Cyclisierung - ist in zahlreichen Publikationen vorbeschrieben, beispielsweise in GB 2021098 oder DE 19536842. In Liebigs Ann. Chem. 1982, 1, 182-185 wird ein Verfahren beschrieben, das die gleichzeitige Umsetzung von Anilinen mit (Cyanacetyl)harnstoff und Carbonsäureorthoestern zur Herstellung von Acryloylharnstoffen beschreibt, deren anschließende thermische Cyclisierung Uracile liefert.

Die als Ausgangsprodukte benötigten Verbindungen der Formel (III), in der $V^1$ für Sauerstoff und $V^2$ für Sauerstoff oder Schwefel steht, sind kommerziell erhältlich oder können nach Literaturvorschriften, z.B. analog US 20040072746 oder Tetrahedron Lett. 1970, 45, 3957-3960, hergestellt werden.

Die Reaktion der Aniline der Formel (IVa) bzw. (IVb) mit Carbamaten der allgemeinen Formel (III) kann in Substanz oder vorzugsweise in einem Lösungsmittel, das bei den vorherrschenden Reaktionsbedingungen inert ist, durchgeführt werden, gegebenenfalls in Gegenwart einer Säure. Bevorzugt werden aliphatische Alkohole, wie Beispielsweise Methanol, Ethanol, Isopropanol, Pentanol; Ether wie beispielsweise Diisopropylether, Dioxan, Tetrahydrofuran, 1,2-Dimethoxyethan; Nitrile, wie beispielsweise Acetonitril oder Propionitril; oder Amide, wie beispielsweise N,N-Dimethylformamid (DMF), N,N-Dimethylacetamid oder N-Methylpyrrolidon; oder Mischungen dieser mit Wasser; oder aromatische Kohlenwasserstoffe wie Toluol, Chlorbenzol, Dichlorbenzole, Xylole; oder Dimethylsulfoxid. Die Reaktion kann im Vakuum, bei Normaldruck oder unter Überdruck und bei Temperaturen von -20 bis 250 °C durchgeführt werden, vorzugsweise erfolgt die Reaktion bei Normaldruck und Temperaturen von 30 bis 180 °C.

**Verfahren Va4 (für I-A-III mit $V^1$=$V^2$=O)**

Die erfindungsgemäßen Pyrimidin-2,4(1H,3H)-dione (auch Uracile genannt) der allgemeinen Formel (I) können in (I-A-9a) (für n=0) und (I-A-9b) (für n=1) unterteilt und beispielsweise durch das im folgenden Schema dargestellte **Verfahren Va4** erhalten werden,

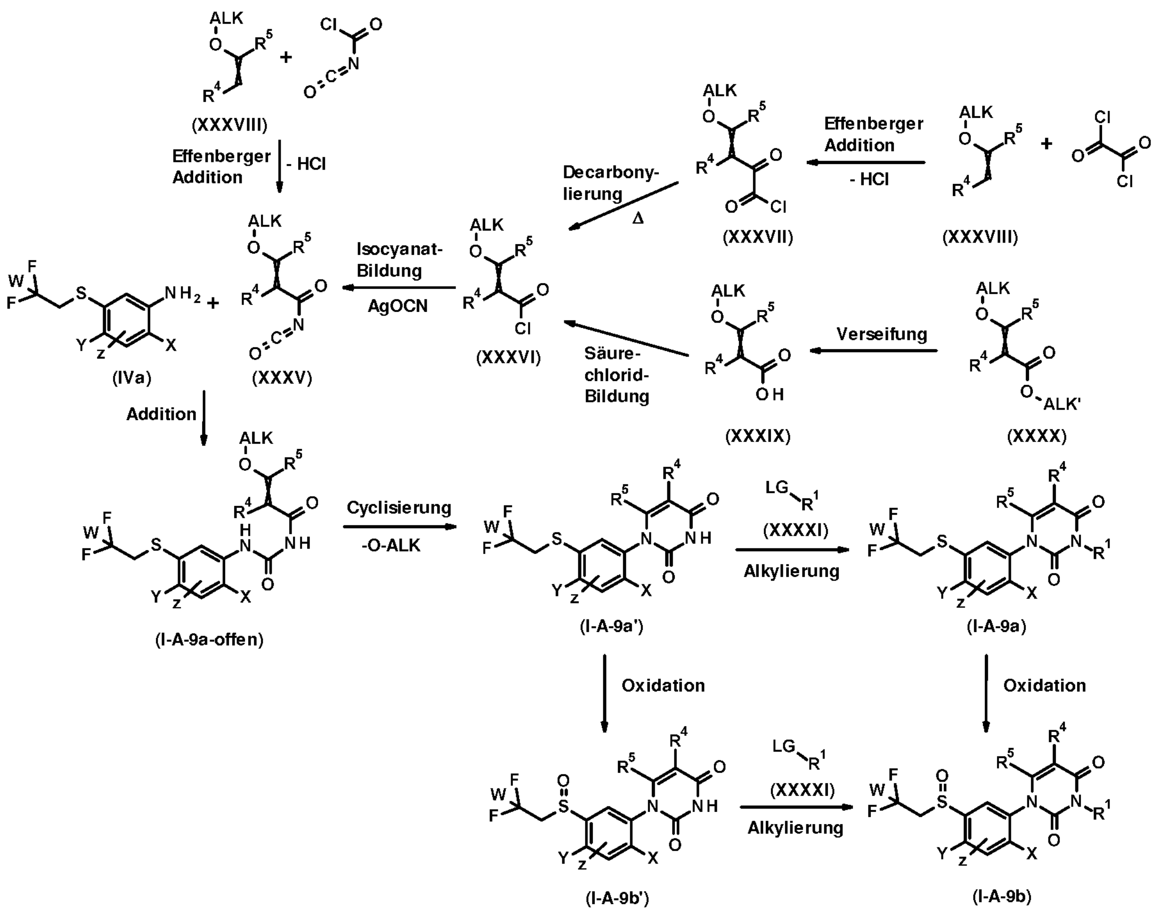


wobei $R^1$, $R^4$, $R^5$, W, X, Y und Z die oben angegebene Bedeutung haben und ALK für eine Alkylgruppe (insbesondere Methyl oder Ethyl) steht.

Aniline der Formel (IVa) können mit ggf. substituierten 3-Alkoxyacryloylisocyanaten der allgemeinen Formel (XXXV) zu den entsprechenden ggf. substituierten 3-Alkoxy-N-(arylcarbamoyl)acrylamiden der allgemeinen Formel (I-A-9a-offen) umgesetzt werden. Die ggf. substituierten 3-Alkoxyacryloylisocyanaten der allgemeinen Formel (XXXV) sind auf verschiedenen, in der Literatur beschriebenen Syntheserouten erhältlich: So können zum Beispiel ggf. substituierte 3-Alkoxyacryloylchloride der allgemeinen Formel (XXXVI) mit Silbercyanat umgesetzt werden wie etwa in Synthesis 2001, 2, 239-242 beschrieben ist. Die Herstellung derartiger ggf. substituierter 3-Alkoxyacryloylchloride der allgemeinen Formel (XXXVI) kann zum Beispiel durch Umsetzung von ggf. substituierten, kommerziell erhältlichen oder literaturbekannten Alkylvinylethern der allgemeinen Formel (XXXVIII) mit Oxalylchlorid zu den entsprechenden $\alpha$-Ketosäurechloriden der allgemeinen Formel (XXXVII) und anschließender Decarbonylierung bei erhöhten Temperaturen realisiert werden (siehe zum Beispiel Synthesis 1993, 1079-1080 oder J. Amer. Chem. Soc. 2011, 133, 41, 16418-16421). Eine weitere Möglichkeit zur Darstellung von ggf. substituierten 3-Alkoxyacryloylchloriden der allgemeinen Formel (XXXVI) ist die Verseifung kommerziell erhältlicher oder literaturbekannter 3-Alkoxyacrylsäureester der allgemeinen Formel (XXXX) zu den entsprechenden 3-Alkoxyacrylsäuren der allgemeinen Formel (XXXIX) und anschließender Säurechloridbildung, zum Beispiel mit Oxalylchlorid oder Thionylchlorid wie etwa in J. Heterocyclic Chem. 1999, 36, 293-295 beschrieben wird.

Alternativ können ggf. substituierte 3-Alkoxyacryloylisocyanate der allgemeinen Formel (XXXV) durch direkte Umsetzung von *N*-(Chlorcarbonyl)-isocyanat (siehe zum Beispiel Angew. Chem. 1977, 89, 789-796) mit ggf. substituierten, kommerziell erhältlichen oder literaturbekannten Alkylvinylethern der allgemeinen Formel (XXXVIII) unter Basenzusatz hergestellt werden, wie z. B. in Tetrahedron 2006, 62, 906-914 oder in Nucleosides Nucleotides 1995, 14, 2039-2049 beschrieben ist. Vorzugsweise werden die dargestellten 3-Alkoxyacryloylisocyanate der allgemeinen Formel (XXXV) nicht isoliert, sondern unmittelbar mit Anilinen der Formel (IVa) zu 3-Alkoxy-N-(arylcarbamoyl)acrylamiden der allgemeinen Formel (I-A-9a-offen) umgesetzt.

Ggf. substituierte 3-Alkoxy-N-(arylcarbamoyl)acrylamiden der allgemeinen Formel (I-A-9a-offen) können sowohl isoliert werden als auch ohne Zwischenisolierung zu den entsprechenden 1-Arylpyrimidin-2,4(1H,3H)-dionen der allgemeinen Formel (I-A-9a') unter Basen- oder Säureeinwirkung cyclisiert werden (siehe zum Beispiel WO2009/039127 oder J. Org. Chem. 2005, 70, 20, 7925-7335), vorzugsweise unter sauren Reaktionsbedingungen und Hitzeeinwirkung.

Anschließende *N*-Alkylierung mit klassischen Alkylierungsmitteln der allgemeinen Formel (XXXXI), wobei LG für klassische Abgangsgruppen wie zum Beispiel Chloride, Bromide, Iodide, Tosylate, Triflate oder Mesylate steht, ggf. in Anwesenheit einer geeigneten Base und ggf. unter Einsatz von inerten Lösungsmitteln liefert die entsprechenden Pyrimidin-2,4(1H,3H)-dione der allgemeinen Formel (I-A-9a) (siehe zum Beispiel Tetrahedron 2007, 63, 2859-2864).

Durch Oxidation der Thioether der allgemeinen Formel (I-A-9a) nach literaturbekannten Methoden können die erfindungsgemäßen Sulfoxide der allgemeinen Formel (I-A-9b) erhalten werden.

Alternativ können die Arylpyrimidin-2,4(1H,3H)-dion-Thioether der allgemeinen Formel (I-A-9a') zunächst nach literaturbekannten Methoden zu den erfindungsgemäßen Arylpyrimidin-2,4(1H,3H)-dion-Sulfoxiden der allgemeinen Formel (I-A-9b') oxidiert und anschließend zu den erfindungsgemäßen Arylpyrimidin-2,4(1H,3H)-dion der allgemeinen Formel (I-A-9b) alkyliert werden.

**Verfahren Va5 (für I-A-III mit $V^1$=O und $V^2$=O)**

Die erfindungsgemäßen Verbindungen der allgemeinen Formel (I) können in (I-A-9a) (für n=0) und (I-A-9b) (für n=1) unterteilt und beispielsweise durch das im folgenden Schema dargestellte Verfahren **Va5** erhalten werden,

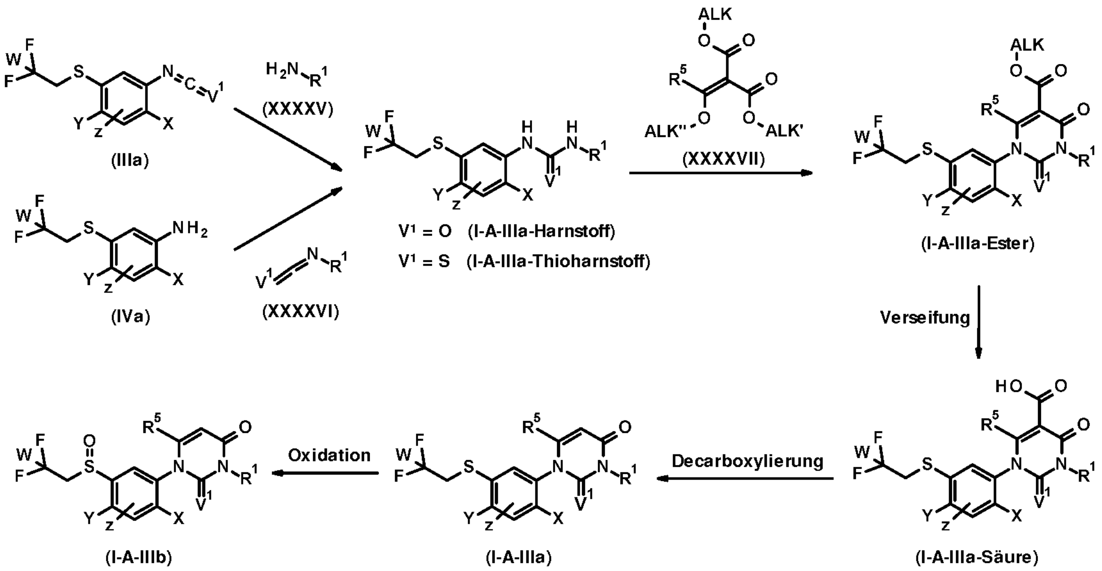


wobei $V^1$ für Sauerstoff steht, $R^1$, $R^5$, W, X, Y und Z die oben angegebene Bedeutung haben und ALK, ALK' sowie ALK" für Alkylgruppen (insbesondere Methyl oder Ethyl) stehen.

Harnstoffe der allgemeinen Formel (I-A-IIIa-Harnstoff) bzw. Thioharnstoffe der allgemeinen Formel (I-A-IIIa-Thioharnstoff) können zum einen aus Isocyanaten bzw. Isothiocyanaten der allgemeinen Formel (IIIa) ($V^1$=O bzw. S) durch Umsetzung mit Aminen der allgemeinen Formel (XXXXV) dargestellt werden, zum anderen durch Reaktion von Anilinen der Formel (IVa) mit entsprechenden Iso(thio)cyanaten der allgemeinen Formel (XXXXVI) synthetisiert werden. Cyclisierung der Harnstoffe bzw. Thioharnstoffe (I-A-IIIa-Harnstoff bzw. -Thioharnstoff) durch Umsetzung mit kommerziell erhältlichen oder literaturbekannten, ggf. substituierten (Alkoxymethylen)malonsäureestern der allgemeinen Formel (XXXXVII) führt zu ggf. substituierten 1-Aryl-2,4-dioxo- oder 1-Aryl-4-oxo-2-thioxo-1,2,3,4-tetrahydropyrimidin-5-carbonsäureestern der allgemeinen Formel (I-A-IIIa-Ester), anschließende Verseifung ergibt die entsprechenden Carbonsäurederivate der allgemeinen Formel (I-A-9a-Säure), wie zum Beispiel in WO2013/074633 beschrieben ist.

Zur Darstellung der Uracile der Formel (I-A-IIIa) ohne Substituenten in 5-Position können Carbonsäuren der allgemeinen Formel (I-A-IIIa-Säure) decarboxyliert werden, beispielsweise unter Hitzeeinwirkung in einem inerten, hochsiedenden Lösungsmittel wie zum Beispiel Diphenylether oder Mercaptoessigsäure in Analogie zu den in Heterocycles 1978, 9(10), 1387-1390 oder J. Med. Chem. 1979, 22(12), 1483-1487 beschriebenen Methoden.

Durch Oxidation der Thioether der allgemeinen Formel (I-A-IIIa) nach literaturbekannten Methoden können die erfindungsgemäßen Sulfoxide der allgemeinen Formel (I-A-IIIb) erhalten werden.

Thionierung:

Ein weiteres allgemeines Verfahren zur Herstellung der erfindungsgemäßen Verbindungen der allgemeinen Formel (Ia) oder (Ib), in denen V bzw. $V^1$ und $V^2$ unabhängig voneinander Schwefel sein können, besteht in der Umwandlung der Carbonylgruppe(n) entsprechender Vorstufen in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Pyridin, Xylol oder Cumol. Diese Variante ist in zahlreichen Publikationen beschrieben, z.B. in J. Amer. Chem. Soc. 1956, 1938-1941, Chem. Pharm. Bull. 1962, 10, 647-652, US 3007927 (für 1,2,4-Triazin(di)thione), DE 2554866 oder WO 2000026194 (für Pyrimidinthione).

Oxidation:

Verbindungen der allgemeinen Formel (Ib) lassen sich durch Oxidation nach literaturbekannten Verfahren aus Verbindungen der allgemeinen Formel (Ia) herstellen, beispielsweise durch ein Oxidationsmittel in einem geeignetem Lösungs- und Verdünnungsmittel. Als Oxidationsmittel eignen sich zum Beispiel verdünnte Salpetersäure, Wasserstoffperoxid und Peroxycarbonsäuren, wie etwa meta-Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum

Beispiel mit Titanium und Vanadium als meistbenutzten Katalysatorquellen, in Form von Ti(O$^i$Pr$_4$) und VO(acac)$_2$, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC-Säule getrennt werden.

Alternativ können Verbindungen der allgemeinen Formel (Ib) durch ähnliche wie die hier genannten Methoden in einer anderen Reihenfolge hergestellt werden.

## Erläuterung der Ausgangsstoffe und Zwischenprodukte

### *Aniline der allgemeinen Formel (IVa) und (IVb)*

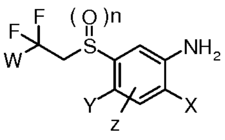

(IVa) n=0
(IVb) n=1

Aniline der Formel (IVa) sind teilweise literaturbekannt, z.B. aus JP 2007/284356, oder können anhand literaturbekannter Verfahren synthetisiert werden, insbesondere nach den in den Herstellbeispielen genannten Bedingungen.

Die Verbindungen der Formel (IVb) sind neu und lassen sich durch Oxidation, insbesondere nach den in den Herstellbeispielen genannten Bedingungen, herstellen.

Die Aniline der allgemeinen Formel (IVa) können vorzugsweise wie im folgenden Schema hergestellt werden,

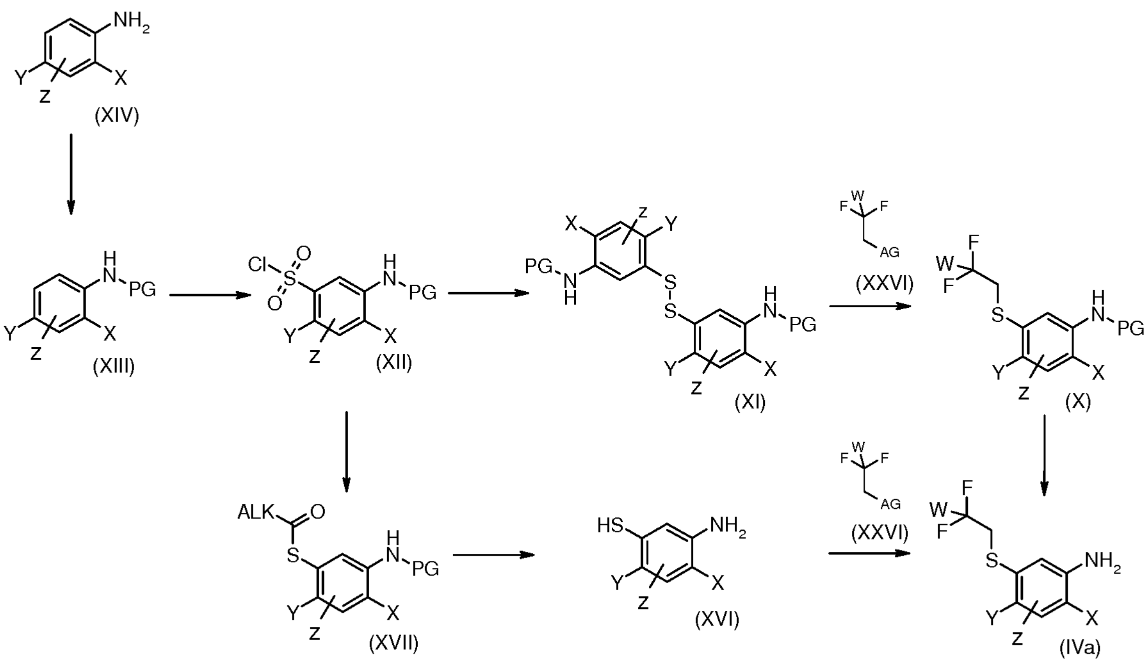


,

wobei X, Y, Z und W die oben angegebenen Bedeutungen haben, AG für eine Abgangsgruppe und PG für eine Schutzgruppe steht.

Aniline der Formel (XIV) sind entweder kommerziell erhältlich oder können durch bekannte Methoden hergestellt werden. Sie können mit einer geeigneten Schutzgruppe, wie z.B. einer Acetylgruppe, zu Verbindungen der Formel (XIII) geschützt werden. Beispielsweise in Gegenwart von Säuren, Säureanhydriden oder Säurechloriden können die Aniline

(XIV) in die entsprechenden Anilide (XIII) überführt werden. Die Chlorsulfonierung der geschützen Aniline (XIII) mit Chlorsulfonsäure liefert die entsprechenden Sulfonylchloride (XII). Die Reduktion der Sulfonylchloride (XII) in die Disulfide (XI) ist mit literaturbekannten Methoden wie zum Beispiel Eisen in Salzsäure oder Iodid möglich. Die Umsetzung der Disulfide (XI) mit Haloalkylelektrophilen der Formel (XXVI), wobei AG für eine Abgangsgruppe wie zum Beispiel Chlor, Brom, Tosylat, Mesylat oder Triflat steht, liefert die Sulfide (X). Die Schutzgruppe kann durch geeignete literaturbekannte Methoden abgespalten werden, so dass man Aniline der Formel (IVa) erhält.

Anstatt der Reduktion zum Disulfid (XI), kann das Sulfonylchlorid (XII) mit einem geeigneten Reduktionsmittel wie zum Beispiel Iod/Phosphor zum Alkylthioat (XVII) reduziert und anschließend durch eine geeignete Methode, wie zum Beispiel die Umsetzung mit Kaliumhydroxidlösung, zu Thiolen der Formel (XVI) entschützt werden. Die Umsetzung der Thiole (XVI) mit Haloalkylelektrophilen der Formel (XXVI) liefert die Sulfide (IVa).

Die Verbindungen der Formel (X), (XI), (XII), (XIII), (XVI) und (XVII) sind neu und lassen sich insbesondere nach den in den Herstellbeispielen genannten Bedingungen herstellen.

Ebenfalls bevorzugt können die Thioether der Formel (IVa) alternativ nach folgendem Schema hergestellt werden,

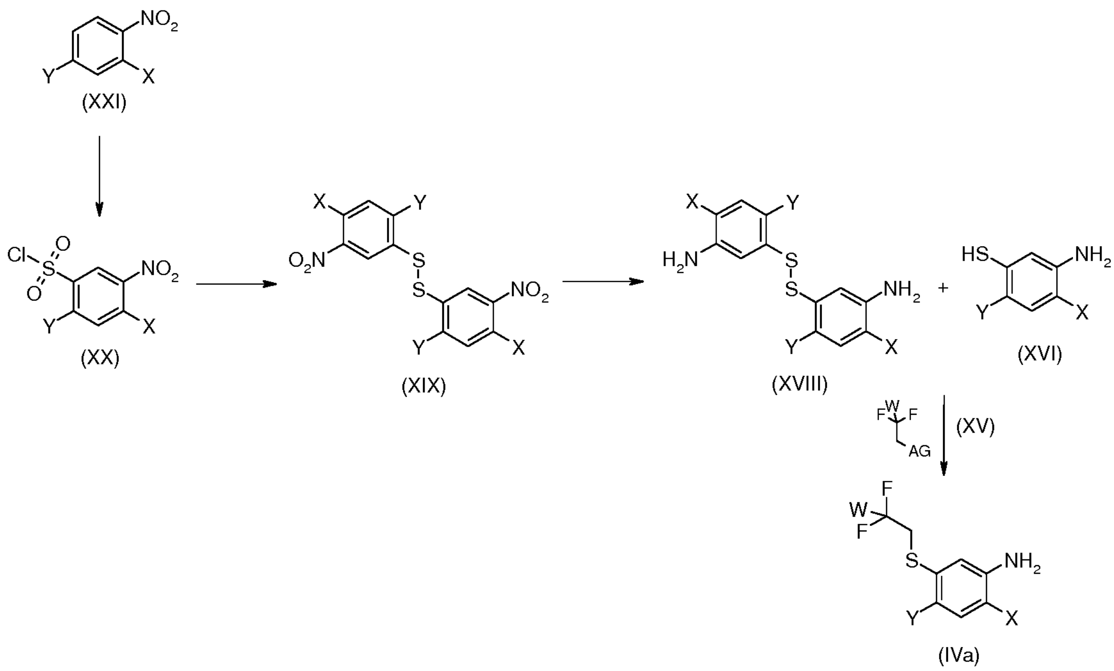

wobei X, Y, Z und W die oben angegebenen Bedeutungen haben, AG für eine Abgangsgruppe und PG für eine Schutzgruppe steht.

Die Chlorsulfonierung der Nitroaromaten der Formel (XXI) mit Chlorsulfonsäure liefert die entsprechenden Sulfonylchloride (XX). Die Reduktion der Sulfonylchloride (XX) in die Bis(nitroaryl)disulfide (XIX) ist mit literaturbekannten Methoden, wie zum Beispiel Iodid, möglich. Die Reduktion der Disulfide (XXI) in die Disulfanediyldianiline (XIX), die teilweise als Gemisch mit den entsprechenden Aminoarylthiolen (XVI) entstehen, ist mit allgemein bekannten Reduktionsmittel, wie zum Beispiel Wasserstoff, gegebenenfalls mit Hilfe heterogener Katalysatoren, wie zum Beispiel Raney-Nickel, Platin auf Aktivkohle oder Palladium auf Aktivkohle, möglich. Die Umsetzung der Disulfide (XVIII) bzw. Thiophenole (XVI) mit Haloalkylelektrophilen der Formel (XV), wobei AG für eine Abgangsgruppe wie zum Beispiel Chlor, Brom, Iod, Tosylat, Mesylat oder Triflat steht, liefert die 3-[(2,2,2-Trifluorethyl)sulfanyl]aniline der Formel (IVa).

Die Verbindungen der Formel (XVI), (XVIII), (XIX) und (XX) sind neu und lassen sich insbesondere nach den in den Herstellbeispielen genannten Bedingungen herstellen.

***Isocyanate (V=O) und Isothiocyanate (V=S) der allgemeinen Formel (IIIa)***

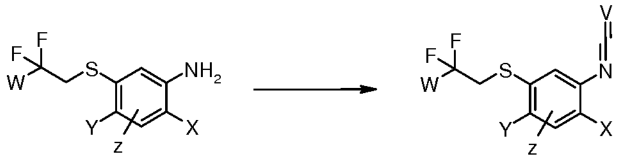

(IVa) (IIIa) V = O V = S

Die Syntheseintermediate (IIIa) (Isocyanate für V=O, Isothiocyanate für V=S) sind literaturbekannt oder können aus den Anilinen der allgemeinen Formel (IVa) nach literaturbekannten Methoden hergestellt werden. Isocyanate und Isothiocyanate der allgemeinen Formel (IIIa) sind aus JP2011/042611 bekannt oder können nach literaturbekannten Methoden hergestellt werden, insbesondere nach den in den Herstellbeispielen genannten Bedingungen.

***Halogenide der allgemeinen Formel (VIIa)***

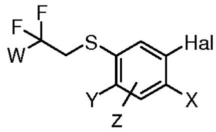

(VIIa)

in welcher X, Y, Z und W die oben angegebenen Bedeutungen haben und Hal für Chlor, Brom oder Iod steht, sind literaturbekannt aus WO 2007/034755, JP 2007/081019, JP 2007/284385, JP 2008/260706, JP 2008/308448, JP 2009/023910 oder WO 2012/176856 oder können anhand literaturbekannter Verfahren synthetisiert werden, die gegebenenfalls geringfügig modifiziert sein können, insbesondere wie in den konkreten Synthesebeipielen beschrieben.

Als Ausgangsstoffe für die Synthese der Iodide der allgemeinen Formel (VIIa) eignen sich Bromide derselben Formel, zum Beispiel in Halogenaustauschreaktionen nach literaturbekannten Methoden gegebenenfalls unter Metallkatalyse (s. H. Suzuki, Chem. Let. 1985, 3, 411-412; S. L. Buchwald, J. Amer. Chem. Soc. 2002, 124 (50), 14844-14845), insbesondere nach den in den Synthesebeispielen genannten Bedingungen. Ebenso ist die Synthese möglich ausgehend von Anilinen der Formel (IVa) unter Sandmeyers Reaktionsbedingungen, wie von E. B. Merkushev in Synthesis 1988, 12, 923-937, beschrieben.

***Boronsäuren der allgemeinen Formel (VIIIa) und (VIIIb)***

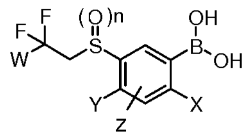

(VIIIa) n=0
(VIIIb) n=1

in welcher X, Y, Z und W die oben angegebenen Bedeutungen haben, sind literaturbekannt, z.B. aus WO2007/034755, JP2007/284385, JP2009/023910 und WO2012/176856 oder können anhand literaturbekannter Verfahren synthetisiert werden, insbesondere wie in den konkreten Synthesebeipielen beschrieben.

*Hydrazine der allgemeinen Formel (Va)*

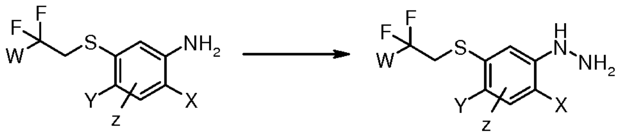

(IVa) (Va)

Hydrazine der allgemeinen Formel (Va) sind teilweise literaturbekannt, zum Beispiel aus EP 1803712 A1 und WO 2006043635, oder können anhand literaturbekannter Verfahren synthetisiert werden, wie zum Beispiel in J. Med. Chem. 2003, 46, 4405-4418 beschrieben.

*Heterocyclischen Verbindungen der Formel (XXII)*

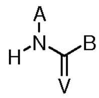

(XXII)

in welcher A und B gemeinsam mit V und den Atomen, an die sie gebunden sind, für eine Substruktur D wie vorstehend angegeben stehen, sind kommerziell erhältlich, literaturbekannt oder können anhand literaturbekannter Verfahren synthetisiert werden. Als Beispiele werden die unterschiedlichen heterocyclischen Verbindungen in ihre Subklassen unterteilt und dort genannt.

***1,2,4-Triazin(di)(thi)one der Formel (XXII-A-I)***

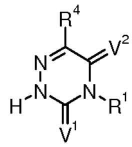

(XXII-A-I)

1,2,4-Triazin(di)(thi)one der Formel (XXII-A-I), in denen $V^1$ und $V^2$ für Sauerstoff stehen, sind kommerziell erhältlich, literaturbekannt oder können anhand literaturbekannter Verfahren (analog der unten zitierten Literaturstellen) synthetisiert werden. Als Beispiele seien folgende Verbindungen genannt: 1,2,4-Triazin-3,5*(2H,4H)*-dion (kommerziell erhältlich, Chem. Berichte 1947, 180, 494-502), 4-Methyl-1,2,4-triazin-3,5*(2H,4H)*-dion (kommerziell erhältlich, Bull. Soc. Chim. Fr. 1959, 1793-1798), 4-Ethyl-1,2,4-triazin-3,5*(2H,4H)*-dion (Coll. Czech. Chem. Comm. 1961, 26, 986-997), 3,4-Dihydro-3-thioxo-1,2,4-triazin-5(2H)-on (kommerziell erhältlich, J. Amer. Chem. Soc. 1938-1941), 3,4-Dihydro-4-methyl-3-thioxo-1,2,4-triazin-5(2H)-on (Coll. Czech. Chem. Comm. 1961, 26, 986-997), 1,2,4-Triazin-3,5*(2H,4H)*dithion (kommerziell erhältlich, US 3007927), 4-Methyl-1,2,4-triazin-3,5*(2H,4H)*dithion (kommerziell erhältlich, Coll. Czech. Chem. Comm. 1961, 26, 986-997), 4,5-Dihydro-5-thioxo-1,2,4-triazin-3*(2H)*-on (kommerziell erhältlich, Chem. Pharm. Bull 1962, 10, 647-652), 4,5-Dihydro-4-methyl-5-thioxo-1,2,4-triazin-3*(2H)*-on (Coll. Czech. Chem. Comm. 1961, 26, 986-997).

***Pyrimidin-2,4(1H,3H)-(di)(thi)one (XXII-A-III) (mit $V^1$, $V^2$ = O)***

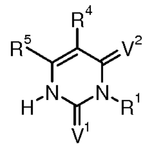

(XXII-A-III)

Uracile der allgemeinen Formel (U-NH) sind kommerziell erhältlich oder können nach bekannten Methoden, z.B. analog J. Biol. Chem. 1925, 65, 469-477, Compt. Rend. 1959, 248, 3444-3446, DE 1248665, WO 9401102, J. Org. Chem. 1972, 11, 11738-11742, J. Org. Chem. 1977, 12, 2185-2187, J. Heterocycl. Chem. 1972, 9(5), 1175-1176, J. Heterocycl. Chem. 1972, 9(6), 1423, Can. J. Chem. 1978, 56(5), 725-729, hergestellt werden.

***Verbindungen der allgemeinen Formel (XXIII) und (XXIII')***

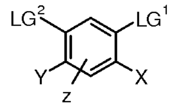

(XXIII)

(XXIII´) $LG^2$ = H

in welcher Z die oben angegebene Bedeutung hat, X steht für Wasserstoff oder eine elektronenziehende Gruppe (insbesondere Nitro, Chlor, Fluor, Cyano), Y stellt elektronenziehende Substituenten (insbesondere Nitro, Chlor, Fluor, Cyano) dar, $LG^1$ steht für typische Abgangsgruppen in nucleophilen Substitutionsreaktionen (insbesondere Fluorid, Chlorid) und $LG^2$ kann für Wasserstoff oder für typische Abgangsgruppen in nucleophilen Substitutionsreaktionen (insbesondere Fluorid, Chlorid) stehen, sind kommerziell erhältlich, literaturbekannt oder lassen sich nach literaturbekannten Methoden herstellen.

Als Beispiele seien folgende Verbindungen genannt, die kommerziell erhältlich sind: 2,4,5-Trifluorbenzonitril (Y=CN, X=$LG^1$=$LG^2$=F, Z=H), 2,4-Difluorobenzonitril (Y=CN, X=Z=H, $LG^1$=$LG^2$=F), 3,4-Difluorbenzonitril (Y=CN, X=$LG^1$=F, $LG^2$=Z=H).

***Verbindungen der allgemeinen Formel (XXIV) und (XXIV')***

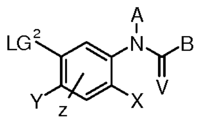

(XXIV)

(XXIV´) $LG^2$ = H

in welcher Z die oben angegebene Bedeutung hat, A und B stehen gemeinsam mit V und den Atomen, an die sie gebunden sind, für eine Substruktur D wie vorstehend angegeben, X steht für Wasserstoff oder eine elektronziehende Gruppe (insbesondere Nitro, Chlorid, Fluorid, Cyano), Y stellt elektronenziehende Substituenten (insbesondere Nitro, Chlorid, Fluorid, Cyano) dar und $LG^2$ kann für Wasserstoff oder für typische Abgangsgruppen in nucleophilen Substitutionsreaktionen (insbesondere Fluorid, Chlorid) stehen, sind kommerziell erhältlich, literaturbekannt oder lassen sich nach literaturbekannten Methoden herstellen, insbesondere wie in den konkreten Synthesebeispielen beschrieben.

Als Beispiele seien folgende Verbindungen genannt: 4-[4-Cyclopropyl-5-oxo-3-(trifluormethyl)-4,5-dihydro-1H-1,2,4-triazol-1-yl]-2,5-difluorbenzonitril (Y=CN, X=$LG^2$=F, Z=H) (s. Synthesebeispiele), Ethyl-4-methyl-5-oxo-1-phenyl-4,5-dihydro-1H-1,2,4-triazol-3-carboxylat (Y=CN, X=F, $LG^2$=Z=H) (kommerziell erhältlich), Methyl-1-(2-fluor-4-methylphenyl)-4-isopropyl-5-oxo-4,5-dihydro-1H-1,2,4-triazol-3-carboxylat (Synthese nach DE 2725148).

*Thiole der allgemeinen Formel (XXV)*

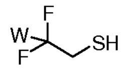

(XXV)

in welcher W die oben angegebenen Bedeutungen haben sind kommerziell erhältlich, literaturbekannt oder anhand literaturbekannter Verfahren synthetisiert werden.

Als Beispiele seien folgende Thiole genannt: 2,2,2-Trifluorethanthiol (W=F) (kommerziell erhältlich), 2,2-Difluorethanthiol (W=H) (Synthese nach J. Amer. Chem. Soc. 1963, 85, 749-754), 2-Chlor-2,2-difluorethanthiol (W=Cl) (Synthese nach Phosp., Sulf., Sil. and rel. Elem. 1996, 119, 161-168).

*Elektrophile der allgemeinen Formel (XXVI)*

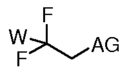

(XXVI)

in welcher W die oben angegebenen Bedeutungen haben und 1 steht für Halogen (insbesondere Chlor, Iod) sind kommerziell erhältlich, literaturbekannt oder anhand literaturbekannter Verfahren synthetisiert werden.

Als Beispiele seien folgende Elektrophile genannt, die kommerziell erhältlich sind: 2-Chlor-1,1,1-trifluorethan (W=F, AG=Cl), 2-Chlor-1,1-difluorethan (W=H, AG=Cl), 2-Brom-1,1,1-trifluorethan (W=F, AG=Br), 2-Brom-1,1-difluorethan (W=H, AG=Br), 2-Iod-1,1,1-trifluorethan (W=F, AG=I), 2-Iod-1,1-difluorethan (W=H, AG=I), 2,2,2-Trifluorethylmethansulfonat (W=F, AG=-$SO_2$Me), 2,2,2-Trifluorethyltriflat (W=F, AG=-$SO_2CF_3$), 2,2,2-Trifluorethyltosylat (W=F, AG=-$SO_2$(4-$CH_3C_6H_4$)).

Die erfindungsgemäßen bzw. erfindungsgemäß zu verwendenden Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm der Arthropoda, insbesondere aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., Epitrimerus pyri, Eutetranychus spp., Eriophyes spp., Glycyphagus domesticus, Halotydeus destructor, Hemitarsonemus spp., Hyalomma spp., Ixodes spp., Latrodectus spp., Loxosceles spp., Metatetranychus spp., Neutrombicula autumnalis, Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., Tetranychus spp., Trombicula alfreddugesi, Vaejovis spp., Vasates lycopersici.;

aus der Klasse der Chilopoda z.B. Geophilus spp., Scutigera spp.;

aus der Ordnung oder der Klasse der Collembola z.B. Onychiurus armatus.;

aus der Klasse der Diplopoda z.B. Blaniulus guttulatus;

aus der Klasse der Insecta, z.B. aus der Ordnung der Blattodea z.B. Blattella asahinai, Blattella germanica, Blatta

orientalis, Leucophaea maderae, Panchlora spp., Parcoblatta spp., Periplaneta spp., Supella longipalpa;

aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptolestes ferrugineus, Cryptorhynchus lapathi, Cylindrocopturus spp., Dermestes spp., Diabrotica spp., Dichocrocis spp., Dicladispa armigera, Diloboderus spp., Epilachna spp., Epitrix spp., Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., Lachnosterna consanguinea, Lasioderma serricorne, Latheticus oryzae, Lathridius spp., Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Necrobia spp., Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllophaga helleri, Phyllotreta spp., Popillia japonica, Premnotrypes spp., Prostephanus truncatus, Psylliodes spp., Ptinus spp., Rhizobius ventralis, Rhizopertha dominica, Sitophilus spp., Sitophilus oryzae, Sphenophorus spp., Stegobium paniceum, Sternechus spp., Symphyletes spp., Tanymecus spp., Tenebrio molitor, Tenebrioides mauretanicus, Tribolium spp., Trogoderma spp., Tychius spp., Xylotrechus spp., Zabrus spp.;

aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Calliphora vicina, Ceratitis capitata, Chironomus spp., Chrysomyia spp., Chrysops spp., Chrysozona pluvialis, Cochliomyia spp., Contarinia spp., Cordylobia anthropophaga, Cricotopus sylvestris, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., Lucilia spp., Lutzomyia spp., Mansonia spp., Musca spp., Oestrus spp., Oscinella frit, Paratanytarsus spp., Paralauterborniella subcincta, Pegomyia spp., Phlebotomus spp., Phorbia spp., Phormia spp., Piophila casei, Prodiplosis spp., Psila rosae, Rhagoletis spp., Sarcophaga spp., Simulium spp., Stomoxys spp., Tabanus spp., Tetanops spp., Tipula spp.;

aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptocorisa varicornis, Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.;

aus der Ordnung der Homoptera z.B. Acizzia acaciaebaileyanae, Acizzia dodonaeae, Acizzia uncatoides, Acrida turrita, Acyrthosipon spp., Acrogonia spp., Aeneolamia spp., Agonoscena spp., Aleyrodes proletella, Aleurolobus barodensis, Aleurothrixus floccosus, Allocaridara malayensis, Amrasca spp., Anuraphis cardui, Aonidiella spp., Aphanostigma piri, Aphis spp., Arboridia apicalis, Arytainilla spp., Aspidiella spp., Aspidiotus spp., Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., Cryptomyzus ribis, Cryptoneossa spp., Ctenarytaina spp., Dalbulus spp., Dialeurodes citri, Diaphorina citri, Diaspis spp., Drosicha spp., Dysaphis spp., Dysmicoccus spp., Empoasca spp., Eriosoma spp., Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Macrosteles facifrons, Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nettigoniclla spectra, Nilaparvata lugens, Oncometopia spp., Orthezia praelonga, Oxya chinensis, Pachypsylla spp., Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Prosopidopsylla flava, Protopulvinaria pyriformis, Pseudaulacaspis pentagona, Pseudococcus spp., Psyllopsis spp., Psylla spp., Pteromalus spp., Pyrilla spp., Quadraspidiotus spp., Quesada gigas, Rastrococcus spp., Rhopalosiphum spp., Saissetia spp., Scaphoideus titanus, Schizaphis graminum, Selenaspidus articulatus, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., Trialeurodes vaporariorum, Trioza spp., Typhlocyba spp., Unaspis spp., Viteus vitifolii, Zygina spp.;

aus der Ordnung der Hymenoptera z.B. Acromyrmex spp., Athalia spp., Atta spp., Diprion spp., Hoplocampa spp., Lasius spp., Monomorium pharaonis, Sirex spp., Solenopsis invicta, Tapinoma spp., Urocerus spp., Vespa spp., Xeris spp.;

aus der Ordnung der Isopoda z.B. Armadillidium vulgare, Oniscus asellus, Porcellio scaber;

aus der Ordnung der Isoptera z.B. Coptotermes spp., Cornitermes cumulans, Cryptotermes spp., Incisitermes spp., Microtermes obesi, Odontotermes spp., Reticulitermes spp.;

aus der Ordnung der Lepidoptera z.B. Achroia grisella, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamstra brassicae, Melanitis leda, Mocis spp., Monopis obviella, Mythimna separata, Nemapogon cloacellus, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudaletia unipuncta, Pseudoplusia includens, Pyrausta nubilalis, Rachiplusia nu, Schoenobius spp., Scirpophaga spp., Scirpophaga innotata, Scotia segetum, Sesamia spp., Sesamia inferens, Sparganothis spp., Spodoptera spp., Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., Tryporyza incertulas, Tuta absoluta, Virachola spp.;

aus der Ordnung der Orthoptera oder Saltatoria z.B. Acheta domesticus, Dichroplus spp., Gryllotalpa spp., Hieroglyphus spp., Locusta spp., Melanoplus spp., Schistocerca gregaria;

aus der Ordnung der Phthiraptera z.B. Damalinia spp., Haematopinus spp., Linognathus spp., Pediculus spp., Phylloera vastatrix, Phtirus pubis, Trichodectes spp.;

aus der Ordnung der Psocoptera z.B. Lepinotus spp., Liposcelis spp.;

aus der Ordnung der Siphonaptera z.B. Ceratophyllus spp., Ctenocephalides spp., Pulex irritans, Tunga penetrans, Xenopsylla cheopsis;

aus der Ordnung der Thysanoptera z.B. Anaphothrips obscurus, Baliothrips biformis, Drepanothrips reuteri, Enneothrips flavens, Frankliniella spp., Heliothrips spp., Hercinothrips femoralis, Rhipiphorothrips cruentatus, Scirtothrips spp., Taeniothrips cardamomi, Thrips spp.;

aus der Ordnung der Zygentoma (= Thysanura), z. B. Ctenolepisma spp., Lepisma saccharina, Lepismodes inquilinus, Thermobia domestica;

aus der Klasse der Symphyla z.B. Scutigerella spp.;

Schädlinge aus dem Stamm der Mollusca, insbesondere aus der Klasse der Bivalvia, z.B. Dreissena spp., sowie

aus der Klasse der Gastropoda z.B. Arion spp., Biomphalaria spp., Bulinus spp., Deroceras spp., Galba spp., Lymnaea spp., Oncomelania spp., Pomacea spp., Succinea spp.;

Tierparasiten aus den Stämmen der Plathelminthes und Nematoda, z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;

Pflanzenschädlinge aus dem Stamm der Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus spp., Trichodorus spp., Tylenchulus spp., Xiphinema spp., Helicotylenchus spp., Tylenchorhynchus spp., Scutellonema spp., Paratrichodorus spp., Meloinema spp., Paraphelenchus spp., Aglenchus spp., Belonolaimus spp., Nacobbus spp., Rotylenchulus spp., Rotylenchus spp., Neotylenchus spp., Paraphelenchus spp., Dolichodorus spp., Hoplolaimus spp., Punctodera spp., Criconemella spp., Quinisulcius spp., Hemicycliophora spp., Anguina spp., Subanguina spp., Hemicriconemoides spp., Psilenchus spp., Pseudohalenchus spp., Criconemoides spp., Cacopaurus spp.

Weiterhin lässt sich aus dem Unterreich der Protozoa die Ordnung der Coccidia z.B. Eimeria spp. bekämpfen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten

Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die die erfindungsgemäßen bzw. erfindungsgemäß zu verwendenden Wirkstoffe können als solche oder in ihren Formulierungen auch in Mischung mit einem oder mehreren geeigneten Fungiziden, Bakteriziden, Akariziden, Molluskiziden, Nematiziden, Insektiziden, Mikrobiologika, Nützlingen, Düngemitteln, Vogelrepellentien, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. Durch Kombination der erfindungsgemäßen bzw. erfindungsgemäß zu verwendenden Wirkstoffe mit Mischpartnern erhält man synergistische Effekte, d.h. die Wirksamkeit der jeweiligen Mischung ist größer als aufgrund der Wirksamkeiten der Einzelkomponenten zu erwarten war. Generell können die Kombinationen sowohl in Vor-, Tank- oder Fertigmischungen als auch in Saatgutanwendungen verwendet werden.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Als Mischpartner geeignete Insektizide / Akarizide / Nematizide sind

(1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder

Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.

(2) GABA-gesteuerte Chlorid-Kanal-Antagonisten, wie beispielsweise

Cyclodien-organochlorine, z.B. Chlordane und Endosulfan; oder

Phenylpyrazole (Fiprole), z.B. Ethiprole und Fipronil.

(3) Natrium-Kanal-Modulatoren / Spannungsabhängige Natrium-Kanal-Blocker, wie beispielsweise

Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder

DDT; oder Methoxychlor.

(4) Nikotinerge Acetylcholin-Rezeptor (nAChR) Agonisten, wie beispielsweise

Neonikotinoide, z.B. Acetamiprid, Clothianidin, Dinotefuran, Imidacloprid, Nitenpyram, Thiacloprid und Thiamethoxam; oder

Nikotin; oder

Sulfoxaflor.

(5) Nikotinerge Acetylcholin-Rezeptor (nAChR) allosterische Aktivatoren, wie beispielsweise
Spinosine, z.B. Spinetoram und Spinosad.

(6) Chlorid-Kanal-Aktivatoren, wie beispielsweise
Avermectine/Milbemycine, z.B. Abamectin, Emamectin-benzoat, Lepimectin und Milbemectin.

(7) Juvenilhormon-Imitatoren, wie beispielsweise

Juvenilhormon-Analoge, z.B. Hydroprene, Kinoprene und Methoprene; oder
Fenoxycarb; oder Pyriproxyfen.

(8) Wirkstoffe mit unbekannten oder nicht spezifischen Wirkmechanismen, wie beispielsweise

Alkylhalide, z.B. Methylbromid und andere Alkylhalide; oder

Chloropicrin; oder Sulfurylfluorid; oder Borax; oder Brechweinstein.

(9) Selektive Fraßhemmer, z.B. Pymetrozine; oder Flonicamid.

(10) Milbenwachstumsinhibitoren, z.B. Clofentezine, Hexythiazox und Diflovidazin; oder
Etoxazole.

(11) Mikrobielle Disruptoren der Insektendarmmembran, z.B. *Bacillus thuringiensis* Subspezies *israelensis, Bacillus thuringiensis* Subspezies *aizawai, Bacillus thuringiensis* Subspezies *kurstaki, Bacillus thuringiensis* Subspezies *tenebrionis* und *B.t.* Pflanzenproteine: Cry1Ab, Cry1Ac, Cry1Fa, Cry1A.105, Cry2Ab, Vip3A, mCry3A, Cry3Ab, Cry3Bb, Cry34 Ab1/35Ab1; oder
*Bacillus sphaericus.*

(12) Inhibitoren der oxidativen Phosphorylierung, ATP-Disruptoren, wie beispielsweise Diafenthiuron; oder

Organozinnverbindungen, z.B. Azocyclotin, Cyhexatin und Fenbutatin-oxid; oder

Propargite; oder Tetradifon.

(13) Entkoppler der oxidativen Phoshorylierung durch Unterbrechung des H-Protongradienten, wie beispielsweise Chlorfenapyr, DNOC und Sulfluramid.

(14) Nikotinerge Acetylcholin-Rezeptor-Antagonisten, wie beispielsweise Bensultap, Cartap-hydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsstörende Wirkstoffe, Dipteran, wie beispielsweise Cyromazine.

(18) Ecdyson-Rezeptor Agonisten, wie beispielsweise Chromafenozide, Halofenozide, Methoxyfenozide und Tebufenozide.

(19) Oktopaminerge Agonisten, wie beispielsweise Amitraz.

(20) Komplex-III-Elektronentransportinhibitoren, wie beispielsweise Hydramethylnon; oder Acequinocyl; oder Fluacrypyrim.

(21) Komplex-I-Elektronentransportinhibitoren, beispielsweise

METI-Akarizide, z.B. Fenazaquin, Fenpyroximate, Pyrimidifen, Pyridaben, Tebufenpyrad und Tolfenpyrad; oder

Rotenone (Derris).

(22) Spannungsabhängige Natriumkanal-Blocker, z.B. Indoxacarb; oder Metaflumizone.

(23) Inhibitoren der Acetyl-CoA-Carboxylase, wie beispielsweise
Tetron- und Tetramsäurederivate, z.B. Spirodiclofen, Spiromesifen und Spirotetramat.

(24) Komplex-IV-Elektronentransportinhibitoren, wie beispielsweise

Phosphine, z.B. Aluminiumphosphid, Calciumphosphid, Phosphin und Zinkphosphid; oder

Cyanid.

(25) Komplex-II-Elektronentransportinhibitoren, wie beispielsweise Cyenopyrafen und Cyflumetofen.

(28) Ryanodinrezeptor-Effektoren, wie beispielsweise

Diamide, z.B. Chlorantraniliprole, Cyantraniliprole und Flubendiamide.

Weitere Wirkstoffe, wie beispielsweise Amidoflumet, Azadirachtin, Benclothiaz, Benzoximate, Bifenazate, Bromopropylate, Chinomethionat, Cryolite, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende Verbindungen: 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-$\lambda^4$-sulfanyliden}cyanamid (bekannt aus WO2007/149134) und seine Diastereomere {[(1R)-1-(6-Chlorpyridin-3-yl)ethyl](methyl)oxido-$\lambda^4$-sulfanyliden}cyanamid (A) und {[(1S)-1-(6-Chlorpyridin-3-yl)ethyl](methyl)oxido-$\lambda^4$-sulfanyliden}cyanamid (B) (ebenfalls bekannt aus WO2007/149134) sowie Diastereomere [(R)-Methyl(oxido){(1R)-1-[6-(trifluormethyl)pyridin-3-yl]ethyl}-$\lambda^4$-sulfanyliden]cyanamid (A1) und [(S)-Methyl(oxido){(1S)-1-[6-(trifluormethyl)pyridin-3-yl]ethyl}-$\lambda^4$-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-$\lambda^4$-sulfanyliden]cyanamid (B1) und [(S)-Methyl(oxido){(1R)-1-[6-(trifluormethyl)pyridin-3-yl]ethyl}-$\lambda^4$-sulfanyliden]cyanamid (B2), bezeichnet als Diastereomerengruppe B (ebenfalls bekannt aus WO

2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), Afidopyropen (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), Pyflubumide (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-fluazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-1[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233), Heptafluthrin, Pyriminostrobin, Flufenoxystrobin und 3-Chlor-$N^2$-(2-cyanpropan-2-yl)-$N^1$-[4-(1,1,1,2,3,3,3-heptafluorpropan-2-yl)-2-methylphenyl]phthalamid (bekannt aus WO2012/034472).

Als Mischpartner geeignete Fungizide sind:

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.

(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des synepimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.

(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2- {2- [(2,5-Dimethylphenoxy)methyl]phenyl}-2-methoxy-N-methylacetamid.

(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.

(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.

(6) Resistenzinduktoren, wie beispielsweise Acibenzolar-S-Methyl, Isotianil, Probenazol und Tiadinil.

(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.

(8) Inhibitoren der ATP Produktion, wie beispielsweise Fentin Acetat, Fentin Chlorid, Fentin Hydroxid und Silthiofam.

(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.

(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos,

Quintozen, Tecnazene und Tolclofos-Methyl.

(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.

(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.

(13) Inhibitoren der Signaltransduktion, wie beispielsweise Chlozolinat, Fenpiclonil, Fludioxonil, Iprodion, Procymidon, Quinoxyfen und Vinclozolin.

(14) Entkoppler, wie beispielsweise Binapacryl, Dinocap, Ferimzon, Fluazinam und Meptyldinocap.

(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl] methyl}-2-phenylacetamid, N-{(Z)-[(Cyclopropylmethoxy)imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat,Phenazin-1-carbonsäure,Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.

(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Die Behandlung der Pflanzen und Pflanzenteile mit den erfindungsgemäßen bzw. erfindungsgemäß zu verwendenden Wirkstoffen, Wirkstoffkombinationen bzw. Mitteln erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-) Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. die Wirkstoffe, Wirkstoffkombinationen bzw. Mittel werden auf das Blattwerk aufgebracht, wobei die Behandlungsfrequenz und die Aufwandmenge auf den Befallsdruck des jeweiligen Pathogens, Schädlings bzw. Unkrauts abgestimmt sein kann.

Bei systemisch wirksamen Verbindungen gelangen die Wirkstoffe, Wirkstoffkombinationen bzw. Mittel über das Wurzelwerk in die Pflanzen. Die Behandlung der Pflanzen erfolgt dann durch Einwirkung der Wirkstoffe, Wirkstoffkombinationen bzw. Mittel auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Wirkstoffe, Wirkstoffkombinationen bzw. Mittel getränkt, oder durch die Bodenapplikation, d.h. die erfindungsgemäßen Wirkstoffe, Wirkstoffkombinationen bzw. Mittel werden in fester Form, (z.B. in Form eines Granulats) in den Standort der Pflanzen eingebracht. Bei Wasserreiskulturen kann das auch durch Zudosieren der Erfindung

in einer festen Anwendungsform (z.B. als Granulat) in ein überflutetes Reisfeld sein.

Die Bekämpfung von Tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen bzw. erfindungsgemäß zu verwendenden Wirkstoff behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang mit einem Wirkstoff der Formel I und Mischungspartner behandelt wird. Es umfasst auch ein Verfahren, in dem das Saatgut zu unterschiedlichen Zeiten mit einem Wirkstoff der Formel I und Mischungspartner behandelt wird.

Die Erfindung bezieht sich ebenfalls auf die Verwendung der erfindungsgemäßen Wirkstoffe zur Behandlung von Saatgut zum Schutz des Saatguts und der daraus entstehenden Pflanze vor Tierischen Schädlingen.

Weiterhin bezieht sich die Erfindung auf Saatgut, welches zum Schutz vor tierischen Schädlingen mit einem erfindungsgemäßen Wirkstoff behandelt wurde. Die Erfindung bezieht sich auch auf Saatgut, welches zur gleichen Zeit mit einem Wirkstoff der Formel I und Mischungspartner behandelt wurde. Die Erfindung bezieht sich weiterhin auf Saatgut, welches zu unterschiedlichen Zeiten mit einem Wirkstoff der Formel I und Mischungspartner behandelt wurde. Bei Saatgut, welches zu unterschiedlichen Zeiten mit einem Wirkstoff der Formel I und Mischungspartner behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel I und Mischungspartner enthalten, gegebenenfalls durch eine Zwischenschicht getrennt sein. Die Erfindung bezieht sich auch auf Saatgut, bei dem ein Wirkstoff der Formel I und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit dem Wirkstoff der Formel (I) bzw. einer ihn enthaltenden Wirkstoffkombination einem Filmcoating - Verfahren unterzogen wurde, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit Wirkstoff der Formel (I) bzw. ihn enthaltenden Wirkstoffkombination Keimung und Auflauf des behandelten Saatguts gefördert werden können.

Ebenso ist es als vorteilhaft anzusehen, dass Wirkstoffe der Formel (I) und die genannten Wirkstoffkombinationen insbesondere auch bei transgenem Saatgut eingesetzt werden können.

Zu nennen ist auch, dass Wirkstoffe der Formel (I) in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit Wirkstoffen der Formel (I) bzw. einer Wirkstoffkombination eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt. Besonders bevorzugt handelt es sich dabei

um ein heterologes Gen, das aus *Bacillus thuringiensis* stammt.

Im Rahmen der vorliegenden Erfindung wird der Wirkstoff der Formel (I) allein (bzw. als Wirkstoffkombination) oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe/Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe/ Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die

Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt des/der Wirkstoffs/Wirkstoffe in den Formulierungen und nach dem Saatgut. Die Aufwandmengen bei Wirkstoffen/Wirkstoffkombinationen liegen im Allgemeinen zwischen 0,001 und 50 g pro Kilogramm Saatgut, vorzugsweise zwischen 0,01 und 15 g pro Kilogramm Saatgut.

Aus dem Stand der Technik ist nicht bekannt, dass die Wirkstoffe der Formel (I) gegen biotische Stressfaktoren und/oder abiotischen Stress von Pflanzen oder im Hinblick auf das Pflanzenwachstum eine Wirkung zeigen.

Es wurde nun gefunden, dass die erfindungsgemäßen Wirkstoffe der Formel (I) zur Steigerung der pflanzeneigenen Abwehrkräfte (Pathogenabwehr in Pflanzen) geeignet sind.

Es ist bekannt, dass Pflanzen auf natürliche Stressbedingungen, wie beispielsweise Kälte, Hitze, Trockenheit, Verwundung, Pathogenbefall (Viren, Bakterien, Pilze), Insekten etc. aber auch auf Herbizide mit spezifischen oder unspezifischen Abwehrmechanismen reagieren (Pflanzenbiochemie, S. 393-462, Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000). Dabei dienen z. B. durch Verwundung entstandene Zellwandbestandteile oder spezifische vom Pathogen stammende Signalsubstanzen als Induktoren pflanzlicher Signaltransduktionsketten, die am Ende zur Bildung von gegen den Stressfaktor gerichteten Abwehrmolekülen führen. Hierbei kann es sich beispielsweise um (a) niedermolekulare Substanzen, wie z.B. Phytoalexine, (b) nicht-enzymatische Proteine, wie z. B. "Pathogenesis-related proteins" (PR-Proteine), (c) enzymatische Proteine, wie beispielsweise Chitinasen, Glucanasen, oder (d) um spezifische Inhibitoren essentieller Proteine, wie beispielsweise um Protease-Inhibitoren, Xylanase-Inhibitoren, handeln, welche das Pathogen direkt angreifen oder seine Proliferation behindern (Dangl and Jones, Nature 411, 826-833, 2001; Kessler and Baldwin, Annual Review of Plant Biology, 53, 299-328, 2003).

Ein zusätzlicher Abwehrmechanismus ist die sogenannte hypersensitive Reaktion (HR), die über oxidativen Stress vermittelt wird und zum Absterben von Pflanzengewebe im Bereich eines Infektionsherdes führt, wodurch eine Ausbreitung von Pflanzenpathogenen, die auf lebende Zellen angewiesen sind, verhindert wird (Pennazio, New Microbiol. 18, 229-240, 1995).

Im weiteren Verlauf einer Infektion werden durch pflanzeneigene Botenstoffe Signale in nicht befallene Gewebe weitergegeben, die auch dort zur Auslösung von Abwehrreaktionen führen und die Entstehung von Sekundärinfektionen behindern (Systemic acquired resistance, SAR) (Ryals et al., The Plant Cell 8, 1809-1819, 1996).

Eine Reihe von pflanzenendogenen Signalstoffen, die in die Stresstoleranz bzw. die Pathogenabwehr involviert sind, sind bereits bekannt. Zu nennen sind hier beispielsweise Salicylsäure, Benzoesäure, Jasmonsäure oder Ethylen (Biochemistry and Molecular Biology of Plants, S. 850-929, American Society of Plant Physiologists, Rockville, Maryland, eds. Buchanan, Gruissem, Jones, 2000). Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben (Sembdner, Parthier, Ann. Rev. Plant Physiol. Plant Mol. Biol. 44, 569-589, 1993). Die Salicylat-vermittelte Abwehr richtet sich besonders gegen phytopathogene Pilze, Bakterien und Viren (Ryals et al., The Plant Cell 8, 1809-1819, 1996).

Ein bekanntes synthetisches Produkt, das eine der Salicylsäure vergleichbare Funktion übernimmt und eine Schutzwirkung gegen phytopathogene Pilze, Bakterien und Viren vermitteln kann, ist Benzothiadiazol (CGA 245704; Common name: Acibenzolar-*S*-methyl; Handelsname: Bion®) (Achuo et al., Plant Pathology 53 (1), 65-72, 2004; Tamblyn et al., Pesticide Science 55 (6), 676-677, 1999; EP-OS 0 313 512).

Andere Verbindungen, die in die Gruppe der Oxylipine gehören, wie z.B. Jasmonsäure, und die durch sie ausgelösten Schutzmechanismen sind besonders gegen Schadinsekten wirksam (Walling, J. Plant Growth Regul. 19, 195-216, 2000) .

Desweiteren ist bekannt, dass die Behandlung von Pflanzen mit Insektiziden aus der Reihe der Neonikotinoide (Chlornikotinyle) zu einer erhöhten Resistenz der Pflanze gegenüber abiotischem Stress führt. Insbesondere gilt dies für das Imidacloprid (Brown et al., Beltwide Cotton Conference Proceedings 2231-2237, 2004). Dieser Schutz erfolgt

durch Beeinflussung physiologischer und biochemischer Eigenschaften der Pflanzenzellen wie z.B. durch Verbesserung der Membranstabilität, Erhöhung der Kohlenhydratkonzentration, Steigerung der Polyolkonzentration und Antioxidantienaktivität (Gonias et al., Beltwide Cotton Conference Proceedings 2225-2229, 2004).

Darüber hinaus ist der Effekt von Chlornikotinylen gegen biotische Stressfaktoren bekannt (Crop Protection 19 (5), 349-354, 2000; Journal of Entomological Science 37(1), 101-112, 2002; Annals of Biology (Hisar, India) 19 (2), 179-181, 2003). Beispielsweise führen Insektizide aus der Reihe der Neonikotinoide (Chlornikotinyle) zu einer erhöhten Expression von Genen aus der Reihe der "Pathogenesis-related Proteins" (PR-Proteine). PR-Proteine unterstützen die Pflanzen primär in der Abwehr von biotischen Stressoren, wie z.B. phytopathogene Pilze, Bakterien und Viren (DE 10 2005 045 174 A; DE 10 2005 022 994 A und WO 2006/122662 A; Thielert Pflanzenschutz-Nachrichten Bayer, 59 (1), 73-86, 2006; Francis et al., European Journal of Plant Pathology, publ. online 23.1.2009).

Des Weiteren ist bekannt, dass die Behandlung von genetisch modifizierten Pflanzen mit Insektiziden aus der Reihe der Neonikotinoide (Chlornikotinyle) zu einer verbesserten Stresstoleranz der Pflanze führt (EP 1 731 037 A), beispielsweise auch gegenüber dem Herbizid Glyphosat (WO 2006/015697 A).

Somit ist bekannt, dass Pflanzen über mehrere endogene Reaktionsmechanismen verfügen, die eine wirksame Abwehr gegenüber verschiedensten Schadorganismen (biotischer Stress) und/oder abiotischem Stress bewirken können.

Die Anzucht von gesunden und gleichmäßig gewachsenen Jungpflanzen bildet eine wesentliche Voraussetzung für den großflächigen Anbau und die ökonomische Bestandesführung landwirtschaftlicher, gartenbaulicher und forstwirtschaftlicher Kulturpflanzen.

Zahlreiche Jungpflanzen-Anzuchtverfahren sind in der Land- und Forstwirtschaft sowie im Gartenbau etabliert. Hierbei werden als Anzuchtsubstrate neben gedämpfter Erde auch spezielle Substrate u.a. auf Basis von Torfmoosen, Kokosfasern, Steinwolle, wie z.B. Grodan®, Bims, Blähton, wie z.B. Lecaton® oder Lecadan®, Tongranulate, wie z.B. Seramis®, Schaumstoffe, wie z.B. Baystrat®, Vermiculite, Perlite, künstliche Erden, wie z.B. Hygromull®, oder Kombinationen dieser Substrate eingesetzt, in das entweder mit Fungiziden und/oder Insektiziden gebeiztes oder ungebeiztes Saatgut ausgesät wird.

In speziellen Kulturen, wie z.B. Tabak, werden Jungpflanzen zunehmend im sogenannten "Float-Verfahren" oder "Floating-Verfahren" angezogen (Leal, R. S., The use of Confidor S in the float , a new tobacco seedlings production system in the South of Brazil. Pflanzenschutz-Nachrichten Bayer (Deutsche Ausgabe) (2001), 54(3), Seiten 337 bis 352; Rudolph, R. D.; Rogers, W. D.; The efficacy of imidacloprid treatment for reduction in the severity of insect vectored virus diseases of tobacco. Pflanzenschutz-Nachrichten Bayer (Deutsche Ausgabe) (2001), 54(3), Seiten 311 bis 336). Bei diesem Verfahren wird das Saatgut in speziellen Behältern, z.B. Styropor-Lochtabletts, in spezieller Anzuchterde auf Basis Torf-Kultur-Substrat ausgesät und anschließend in Containern mit geeigneter Nährlösung bis zum Erreichen der gewünschten Verpflanzungsgröße kultiviert (Abbildung 1). Dabei gestattet man den Behältern auf der Nährlösung zu treiben, wovon sich der Name der Anzuchtmethode ableitet (Leal, 2001, s.o.). In Floating-Verfahren werden seit einigen Jahren zur Bekämpfung von saugenden Schädlingen Insektizide aus der Klasse der Neonicotiniode (Chlornikotinyle) eingesetzt. Üblicherweise werden die Pflanzen im Float-Verfahren kurz vor dem Verpflanzen mit Neonikotinoid (Chlornikotinyle) Insektiziden besprüht oder unmittelbar vor oder beim Verpflanzen ins Feld mit Neonikotinoid (Chlornikotinyle) Insektiziden angegossen, was als "Drenching" bezeichnet wird (Leal, 2001, s.o.; Rudolph and Rogers, 2001, s.o.). Beide Applikationsverfahren sind technisch relativ aufwendig.

Zum Schutz des auflaufenden Saat- oder Pflanzgutes vor pilzlichen Krankheitserregern und Schädlingen werden hierbei bis zur Verpflanzung Fungizide und Insektizide verwendet. Die Wahl der Pflanzenschutzmittel, der Ort und Zeitpunkt der Anwendung sowie die Aufwandmenge der Mittel richten sich hierbei vor allem nach der Art der auftretenden Pilzkrankheiten und Schädlinge, der spezifischen Wirkungsweise und Wirkungsdauer der Mittel sowie deren Pflanzenverträglichkeit, und kann somit unmittelbar an die spezifischen Erfordernisse unterschiedlicher Kulturen und Regionen angepasst werden.

Die Wirkstoffeder Formel (I) führen dabei unabhängig von einer Insektenbekämpfung zu einem guten Schutz der Pflanze vor Schäden durch pilzliche, bakterielle oder virale Pathogene.

Ohne an eine Theorie gebunden sein zu wollen wird zurzeit davon ausgegangen, dass die Abwehr der Pathogene durch die Induktion von PR Proteinen als Folge einer Behandlung mit mindestens einem Wirkstoff der Formel (I) erfolgt.

Insbesondere zeigt die erfindungsgemäße Verwendung in der Saatgutbehandlung, in der Bodenbehandlung, in speziellen Anzucht- und Kultivierungsverfahren (z.B. Floating Box, Rockwool, Hydroponic), aber auch Stamm- und Blattbehandlung die beschriebenen Vorteile. Kombinationen eines Wirkstoffs der Formel (I) unter anderem mit Insektiziden, Fungiziden und Bakteriziden zeigen synergistische Wirkung bei der Bekämpfung von Pflanzenkrankheiten. Die kombinierte Verwendung der Wirkstoffe der Formel (I) mit gentechnisch veränderten Sorten in Bezug auf erhöhte abiotische Stresstoleranz führt darüberhinaus zu einer synergistischen Verbesserung des Wachstums.

Schließlich wurde erfindungsgemäß auch gefunden, dass die Wirkstoffe der Formel (I) nicht nur zur Steigerung der Pathogenabwehr in Pflanzen, sondern auch zur Verbesserung des Pflanzenwachstums und/oder zur Steigerung

der Widerstandfähigkeit von Pflanzen gegenüber Pflanzenkrankheiten, welche durch Pilze, Bakterien, Viren, MLO (Mycoplasma-like organisms) und/oder RLO (Rickettsia-like organisms) verursacht werden, insbesondere gegenüber bodenbürtigen Pilzkrankheiten, und/oder zur Erhöhung der Widerstandsfähigkeit von Pflanzen gegenüber abiotischen Stressfaktoren geeignet sind.

Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Gegenstand der vorliegenden Erfindung ist somit zunächst die Verwendung von mindestens einem Wirkstoff der Formel (I) zur Steigerung von pflanzeneigenen Abwehrkräften und/oder zur Verbesserung des Pflanzenwachstums und/oder zur Steigerung der Widerstandfähigkeit von Pflanzen gegenüber Pflanzenkrankheiten, welche durch Pilze, Bakterien, Viren, MLO (Mycoplasma-like organisms) und/oder RLO (Rickettsia-like organisms) verursacht werden, insbesondere gegenüber bodenbürtige Pilzkrankheiten, und/oder zur Steigerung der Widerstandsfähigkeit von Pflanzen gegenüber abiotischen Stressfaktoren.

Unter der Bezeichnung Pflanzenwachstum werden im Rahmen der vorliegenden Erfindung verschiedenartige Vorteile für Pflanzen verstanden, die nicht unmittelbar mit der bekannten pestiziden Wirksamkeit, bevorzugt der insektiziden Wirksamkeit der Wirkstoffe der Formel (I) verbunden sind. Solche vorteilhaften Eigenschaften sind beispielsweise die nachfolgend genannten verbesserten Pflanzencharakteristika: beschleunigte Keimung und Auflaufen des Saat- und Pflanzgutes, verbessertes Wurzelwachstum hinsichtlich Oberfläche und Tiefe, vermehrte Ausläuferbildung oder Bestockung, stärkere und produktivere Ausläufer und Bestockungstriebe, Verbesserung des Sprosswachstums, erhöhte Standfestigkeit, vergrößerter Sprossbasisdurchmesser, vergrößerte Blattfläche, grünere Blattfarbe, höhere Erträge an Nähr- und Inhaltsstoffen, wie z.B. Kohlenhydrate, Fette, Öle, Proteine, Vitamine, Mineralstoffe, ätherische Öle, Farbstoffe, Fasern, bessere Faserqualität, früheres Blühen, gesteigerte Blütenanzahl, reduzierter Gehalt an toxischen Produkten wie Mycotoxine, reduzierter Gehalt an Rückständen oder unvorteilhaften Bestandteilen jeglicher Art oder bessere Verdaulichkeit, verbesserte Lagerstabilität des Erntegutes, verbesserte Toleranz gegenüber unvorteilhaften Temperaturen, verbesserte Toleranz gegenüber Dürre und Trockenheit wie auch Sauerstoffmangel durch Wasserüberschuss, verbesserte Toleranz gegenüber erhöhten Salzgehalten in Böden und Wasser, gesteigerte Toleranz gegenüber UV-Strahlung, gesteigerte Toleranz gegenüber Ozonstress, verbesserte Verträglichkeit gegenüber Herbiziden und anderen Pflanzenbehandlungsmitteln, verbesserte Wasseraufnahme und Photosyntheseleistung, vorteilhafte Pflanzeneigenschaften, wie beispielsweise Beschleunigung der Reifung, gleichmäßigere Abreife, größere Anziehungskraft für Nützlinge, verbesserte Bestäubung oder andere Vorteile, die einem Fachmann durchaus bekannt sind.

Die weiter oben genannten verschiedenartigen Vorteile für Pflanzen lassen sich bekannterweise partiell zusammenfassen und mit allgemein gültigen Begriffen belegen. Solche Begriffe sind beispielsweise die nachfolgend aufgeführten Bezeichnungen: phytotonischer Effekt, Widerstandsfähigkeit gegenüber Stressfaktoren, weniger Pflanzenstress, Pflanzengesundheit, gesunde Pflanzen, Pflanzenfitness ("Plant Fitness"), "Plant Wellness", "Plant Concept", "Vigor Effect", "Stress Shield", Schutzschild, "Crop Health", "Crop Health Properties", "Crop Health Products", "Crop Health Management", "Crop Health Therapy", "Plant Health", "Plant Health Properties", "Plant Health Products", "Plant Health Management", "Plant Health Therapy", Grünungseffekt ("Greening Effect" oder "Re-greening Effect"), "Freshness" oder andere Begriffe, die einem Fachmann durchaus bekannt sind.

Es wurde ferner gefunden, dass Wirkstoffe der Formel (I) zu einer erhöhten Expression von Genen aus der Reihe der "Pathogenesis-related proteins" (PR-Proteine) führen. PR-Proteine unterstützen die Pflanzen primär in der Abwehr von biotischen Stressoren, wie z.B. phytopathogene Pilze, Bakterien und Viren. Dies hat zur Folge, dass Pflanzen nach Anwendung von Wirkstoffen der Formel (I) besser geschützt sind vor Infektionen phytopathogener Pilze, Bakterien und Viren. Bei notwendigem Einsatz von Insektiziden, Fungiziden und Bakteriziden in Mischung wie auch bei sequentieller Anwendung mit Wirkstoffen der Formel (I) wird deren Wirkung unterstützt.

Erfindungsgemäß wurde darüber hinaus gefunden, dass die Anwendung der Wirkstoffe der Formel (I) in Kombination mit einem Düngemittel wie weiter unten stehend definiert auf Pflanzen oder in deren Umgebung einen synergistischen wachstumssteigernden Effekt bewirkt.

Düngemittel die erfindungsgemäß zusammen mit den oben näher erläuterten Wirkstoffen oder Mitteln verwendet werden können sind im Allgemeinen organische und anorganische Stickstoff-haltige Verbindungen wie beispielsweise Harnstoffe, Harnstoff-Formaldehyd-Kondensationsprodukte, Aminosäuren, Ammoniumsalze und -nitrate, Kaliumsalze (bevorzugt Chloride, Sulfate, Nitrate), Phosphorsäuresalze und/oder Salze von Phosphoriger Säure (bevorzugt Kaliumsalze und Ammoniumsalze). Insbesondere zu nennen sind in diesem Zusammenhang die NPK-Dünger, d.h. Düngemittel, die Stickstoff, Phosphor und Kalium enthalten, Kalkammonsalpeter, d.h. Düngemittel, die noch Calcium enthalten, Ammonsulfatsalpeter (Allgemeine Formel $(NH_4)_2SO_4$ $NH_4NO_3$), Ammonphosphat und Ammonsulfat. Diese Düngemittel sind dem Fachmann allgemein bekannt, siehe auch beispielsweise Ullmann's Encyclopedia of Industrial Chemistry, 5. Edition, Vol. A 10, Seiten 323 bis 431, Verlagsgesellschaft, Weinheim, 1987.

Die Düngemittel können auch Salze aus Mikronährstoffen (bevorzugt Calcium, Schwefel, Bor, Mangan, Magnesium, Eisen, Bor, Kupfer, Zink, Molybdän und Kobalt) und Phytohormonen (z. B. Vitamin B1 und Indol-3-ylessigsäure (IAA)) oder Gemische davon enthalten. Erfindungsgemäß eingesetzte Düngemittel können auch weitere Salze wie Monoammoniumphosphat (MAP), Diammoniumphosphat (DAP), Kaliumsulfat, Kaliumchlorid oder Magnesiumsulfat enthalten. Geeignete Mengen für die sekundären Nährstoffe oder Spurenelemente sind Mengen von 0,5 bis 5 Gew.-%, bezogen auf das gesamte Düngemittel. Weitere mögliche Inhaltsstoffe sind Pflanzenschutzmittel, Insektizide oder Fungizide, Wachstumsregulatoren oder Gemische davon. Hierzu folgen weiter unten weitergehende Ausführungen.

Die Düngemittel können beispielsweise in Form von Pulvern, Granulaten, Prills oder Kompaktaten eingesetzt werden. Die Düngemittel können jedoch auch in flüssiger Form, gelöst in einem wässrigen Medium, eingesetzt werden. In diesem Fall kann auch verdünnter wässriger Ammoniak als Stickstoffdüngemittel eingesetzt werden. Weitere mögliche Inhaltsstoffe für Düngemittel sind beispielsweise in Ullmann's Encyclopedia of Industrial Chemistry, 5. Auflage, 1987, Band A 10, Seiten 363 bis 401, DE-A 41 28 828, DE-A 19 05 834 und DE-A 196 31 764 beschrieben.

Die allgemeine Zusammensetzung der Düngemittel, bei welchen es sich im Rahmen der vorliegenden Erfindung um Einzelnährstoff- und/oder Mehrnährstoffdünger handeln kann, beispielsweise aus Stickstoff, Kalium oder Phosphor, kann innerhalb eines breiten Bereichs variieren. Im Allgemeinen ist ein Gehalt von 1 bis 30 Gew.-% Stickstoff (bevorzugt 5 bis 20 Gew.-%), von 1 bis 20 Gew.-% Kalium (bevorzugt 3 bis 15 Gew.-%) und ein Gehalt von 1 bis 20 Gew.-% Phosphor (bevorzugt 3 bis 10 Gew.-%) vorteilhaft. Der Gehalt von Mikroelementen ist üblicherweise im ppm Bereich, bevorzugt im Bereich von 1 bis 1000 ppm.

Im Rahmen der vorliegenden Erfindung kann das Düngemittel sowie der Wirkstoff der Formel (I) zeitgleich, d.h. synchron, verabreicht werden. Es ist jedoch auch möglich, zunächst das Düngemittel und dann den Wirkstoff der Formel (I) oder zunächst den Wirkstoff der Formel (I) und dann das Düngemittel anzuwenden. Bei nicht zeitgleicher Anwendung des Wirkstoffs der Formel (I) und des Düngemittels erfolgt im Rahmen der vorliegenden Erfindung jedoch die Anwendung in funktionellem Zusammenhang, insbesondere innerhalb eines Zeitraums von im Allgemeinen 24 Stunden, bevorzugt 18 Stunden, besonders bevorzugt 12 Stunden, speziell 6 Stunden, noch spezieller 4 Stunden, noch weiter spezieller innerhalb 2 Stunden. In ganz besonderen Ausführungsformen der vorliegenden Erfindung erfolgt die Anwendung der erfindungsgemäßen Wirkstoffe der allgemeinen Formel (I) und des Düngemittels in einem zeitlichen Rahmen von weniger als 1 Stunde, vorzugsweise weniger als 30 Minuten, besonders bevorzugt weniger als 15 Minuten.

Darüber hinaus ist es möglich, formstabile Mischungen, beispielsweise in der Form von Stäbchen, Granulaten, Tabletten etc., ausgehend von mindestens einem erfindungsgemäß zu verwendenden Wirkstoff und mindestens einem Düngemittel herzustellen. Um eine entsprechende formstabile Mischung herzustellen, können die entsprechenden Bestandteile miteinander gemischt und gegebenenfalls extrudiert werden bzw. kann der mindestens eine erfindungsgemäß zu verwendende Wirkstoff der Formel (I) auf das Düngemittel aufgezogen werden. Gegebenenfalls können auch Formulierungshilfsmittel in den formstabilen Mischungen, wie beispielsweise Streckmittel oder Haftkleber, verwendet werden, um eine Formstabilität der resultierenden Mischung zu erreichen. Durch die entsprechende Formstabilität eignen sich entsprechende Mischungen insbesondere für die Anwendung im Bereich "Home & Garden", d.h. bei einem Privatanwender oder Hobbygärtner, welche die formstabile Mischung bzw. die darin enthaltenden Bestandteile mit einer vorgegebenen, klar definierten Menge und ohne besondere Hilfsmittel verwenden können.

Unabhängig hiervon können die Mischungen aus mindestens einem der erfindungsgemäß zu verwendenden Wirkstoffe und dem mindestens einen Düngemittel auch flüssig vorliegen, so dass - beispielsweise bei einem professionellen Anwender im Bereich der Landwirtschaft - die resultierende Mischung als so genannte Tanklösung ausgebracht werden kann.

Durch die Verwendung mindestens eines der erfindungsgemäß zu verwendenen Wirkstoffe und mindestens einem Düngemittel wird ein vergrössertes Wurzelwachstum ermöglicht, welches wiederum eine höhere Nährstoffaufnahme ermöglicht und damit das Planzenwachstum fördert.

Die erfindungsgemäß zu verwendenden Wirkstoffe können, gegebenenfalls in Kombination mit Düngemitteln, bevorzugt an folgenden Pflanzen angewendet werden, wobei die folgende Aufzählung nicht beschränkend ist.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen zählen z.B. folgende Pflanzenarten: Turf, Reben, Getreide, beispielsweise Weizen, Gerste, Roggen, Hafer, Triticale, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen sind folgende Pflanzen anzusehen: Bamwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Als Bäume seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume können genannt werden: Aus der Baumart Pinus: P. radiate, P. ponderosa, P. contorta, P. sylvestre, P. strobes; aus der Baumart Eucalyptus: E. grandis, E. globulus, E. camadentis.

Als ganz besonders bevorzugte Bäume können genannt werden: Rosskastanie, Platanengewächse, Linde, Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; *Poa spp.*), wie "Kentucky bluegrass" (*Poa pratensis* L.), "rough bluegrass" (*Poa trivialis* L.), "Canada bluegrass" (*Poa compressa* L.), "annual bluegrass" (*Poa annua* L.), "upland bluegrass" (Poa *glaucantha Gaudin*), "wood bluegrass" (*Poa nemoralis* L.) und "bulbous bluegrass" (*Poa bulbosa* L.); Straussgräser ("Bentgrass", *Agrostis spp.*), wie "creeping bentgrass" (*Agrostis palustris* Huds.), "colonial bentgrass" (*Agrostis tenuis* Sibth.), "velvet bentgrass" (*Agrostis canina* L.), "South German Mixed Bentgrass" (*Agrostis spp.* einschließlich *Agrostis tenius* Sibth., *Agrostis canina* L., und *Agrostis palustris* Huds.), und "redtop" (*Agrostis alba* L.);

Schwingel ("Fescues", *Festucu spp.*), wie "red fescue" (*Festuca rubra* L. spp. *rubra*), "creeping fescue" (*Festuca rubra* L.), "chewings fescue" (*Festuca rubra commutata* Gaud.), "sheep fescue" (*Festuca ovina* L.), "hard fescue" (*Festuca longifolia* Thuill.), "hair fescue" (*Festucu capillata* Lam.), "tall fescue" (*Festuca arundinacea* Schreb.) und "meadow fescue" (*Festuca elanor* L.);

Lolch ("ryegrasses", *Lolium* spp.), wie "annual ryegrass" (*Lolium multiflorum* Lam.), "perennial ryegrass" (*Lolium perenne* L.) und "italian ryegrass" (*Lolium multiflorum* Lam.);

und Weizengräser ("wheatgrasses", *Agropyron* spp..), wie "fairway wheatgrass" (*Agropyron cristatum* (L.) Gaertn.), "crested wheatgrass" (*Agropyron desertorum* (Fisch.) Schult.) und "western wheatgrass" (*Agropyron smithii* Rydb.).

Beispiele für weitere "cool season turfgrasses" sind "beachgrass" (*Ammophila breviligulata* Fern.), "smooth bromegrass" (*Bromus inermis* Leyss.), Schilf ("cattails") wie "Timothy" (*Phleum pratense* L.), "sand cattail" (*Phleum subulatum* L.), "orchardgrass" (*Dactylis glomerata* L.), "weeping alkaligrass" (*Puccinellia distans* (L.) Pari.) und "crested dog's-tail" (*Cynosurus cristatus* L.).

Beispiele für "warm season turfgrasses" sind "Bermudagrass" (*Cynodon spp.* L. C. Rich), "zoysiagrass" (*Zoysia spp.* Willd.), "St. Augustine grass" (*Stenotaphrum secundatum* Walt Kuntze), "centipedegrass" (*Eremochloa ophiuroides* Munro Hack.), "carpetgrass" (*Axonopus affinis* Chase), "Bahia grass" (*Paspalum notatum* Flugge), "Kikuyugrass" (*Pennisetum clandestinum* Hochst. ex Chiov.), "buffalo grass" (*Buchloe dactyloids* (Nutt.) Engelm.), "Blue gramma" (*Bouteloua gracilis* (H.B.K.) Lag. ex Griffiths), "seashore paspalum" (*Paspalum vaginatum* Swartz) und "sideoats grama" (*Bouteloua curtipendula* (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Die Wirkstoffe der Formel (I) und ihre Zusammensetzungen eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Wirkstoffe oder Zusammensetzungen allein oder in Kombination mit anderen Wirk- und/oder Hilfsstoffen verwendet. Bevorzugt werden sie in Haushaltsinsektizid-Produkten verwendet. Die erfindungsgemäßen Wirkstoffe sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera,

Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Darüber hinaus können die Wirkstoffe der Formel (I) zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Auf dem Gebiet der Tiergesundheit, d.h. dem Gebiet der Tiermedizin, sind die erfindungsgemäßen Wirkstoffe gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die erfindungsgemäßen Verbindungen, die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z.B. in der Aquakultur; oder gegebenenfalls auch Insekten wie Bienen.

Zu den Haustieren zählen zum Beispiel Säugetiere wie Hamster, Meerschweinchen, Ratten, Mäuse, Chinchillas, Frettchen oder insbesondere Hunde, Katzen; Stubenvögel; Reptilien; Amphibien oder Aquariumfische.

Gemäß einer bevorzugten Ausführungsform werden die erfindungsgemäßen Verbindungen an Säugetiere verabreicht.

Gemäß einer weiteren bevorzugten Ausführungsform werden die erfindungsgemäßen Verbindungen an Vögel, nämlich Stubenvögel oder insbesondere Geflügel, verabreicht.

Durch Verwendung der erfindungsgemäßen Wirkstoffe für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Wirkstoffe wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass der Wirkstoff den jeweiligen Parasiten abtöten, sein Wachstum verhindern oder seine Vermehrung verhindern kann.

Zu Beispielen für Arthropoden zählen, jedoch ohne Einschränkung:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.; aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin sind unter den Arthropoden die folgenden Akari beispielhaft, jedoch ohne Einschränkung, zu nennen: aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida

(Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Zu Beispielen für parasitäre Protozoen zählen, jedoch ohne Einschränkung:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis.

Sarcomastigophora (Rhizopoda), wie Entamoebidae, zum Beispiel Entamoeba histolytica, Hartmanellidae, zum Beispiel Acanthamoeba sp., Harmanella sp.

Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec.

Zu Beispielen für pathogene Endoparasiten, bei denen es sich um Helminthen handelt, zählen Plattwürmer (z.B. Monogenea, Cestodes und Trematodes), Rundwürmer, Acanthocephala und Pentastoma. Zu weiteren Helminthen zählen, jedoch ohne Einschränkung:

Monogenea: z.B.: Gyrodactylus spp., Dactylogyrus spp., Polystoma spp.

Cestodes: aus der Ordnung Pseudophyllidea zum Beispiel: Diphyllobothrium spp., Spirometra spp., Schistocephalus spp., Ligula spp., Bothridium spp., Diplogonoporus spp.

Aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Ründwürmer: Trichinellida zum Beispiel: Trichuris spp., Capillaria spp., Trichomosoides spp., Trichinella spp.

Aus der Ordnung Tylenchida zum Beispiel: Micronema spp., Strongyloides spp.

Aus der Ordnung Rhabditina zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp.,

Marshallagia spp., Cooperia spp., Nematodirus spp., Hyostrongylus spp., Obeliscoides spp., Amidostomum spp., Ollulanus spp.

Aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.

Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.,

Aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der erfindungsgemäßen Wirkstoffe nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf erfindungsgemäße Verbindungen für die Verwendung als Arzneimittel.

Ein weiterer Aspekt bezieht sich auf erfindungsgemäße Verbindungen für die Verwendung als Antiendoparasitikum, insbesondere ein Helminthizid oder ein Mittel gegen Protozoen. Zum Beispiel eigenen sich die erfindungsgemäßen Verbindungen für die Verwendung als Antiendoparasitikum, insbesondere ein Helminthizid oder Mittel gegen Protozoen, z.B. in der Tierzucht, in der Tierhaltung, in Ställen und auf dem Hygienesektor.

Ein weiterer Aspekt wiederum betrifft erfindungsgemäße Verbindungen für die Verwendung als Antiektoparasitikum, insbesondere ein Arthropodizid wie ein Insektizid oder ein Akarizid. Zum Beispiel eignen sich die erfindungsgemäßen Verbindungen für die Verwendung als Antiektoparasitikum, insbesondere ein Arthropodizid wie ein Insektizid oder Akarizid, zum Beispiel in der Tierhaltung, in der Tierzucht, in Ställen, auf dem Hygienesektor.

Die Wirkstoffe der Formel (I) und sie enthaltende Zusammensetzungen eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer erfindungsgemäßen Ausführungsform enthalten die erfindungsgemäßen Zusammensetzungen oder Mittel noch mindestens ein weiteres Insektizid und/oder mindestens ein Fungizid.

In einer weiteren Ausführungsform ist diese erfindungsgemäße Zusammensetzung eine anwendungsfertige (ready-to-use) Zusammensetzung, d.h., sie kann ohne weitere Änderungen auf das entsprechende Material aufgebracht werden. Als weitere Insektizide oder als Fungizide kommen die oben genannten in Frage.

Überraschenderweise wurde auch gefunden, dass die erfindungsgemäßen Wirkstoffe und Zusammensetzungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die erfindungsgemäßen Wirkstoffe und Zusammensetzungen allein oder in Kombinationen mit anderen Wirkstoffen als Antifouling-Mittel eingesetzt werden.

## Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC-MS (Liquid Chromatography Mass Spectrometry) und/oder GC-MS (Gas Chromatography-Mass Spectrometry) charakterisiert.

Die Bestimmung der logP Werte erfolgte analog OECD Guideline 117 (EC Directive 92/69/EEC) durch HPLC (High Performance Liquid Chromatography) an reversed-phase Säulen (C 18), mit nachfolgenden Methoden:

[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten mit einem linearen Gradient von 10% Acetonitril bis 95%

Acetonitril; oder mit 0,025% wässriger Trifluoressigsäure und Acetonitril (enthält 0,025% Trifluoressigsäure) als Eluenten mit einem linearen Gradient von 50% Acetonitril bis 80% Acetonitril.

[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit Bruker II Avance 400, zum Teil ausgestattet mit einem 1,7 mm TCI-Probenkopf, gemessen. In Einzelfällen wurden die NMR Spektren mit einem Bruker Avance II 600 bestimmt.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), m (Multiplett), breit (für breite Signale). Als Lösungsmittel wurden $CD_3CN$, $CDCl_3$ oder D6-DMSO verwendet, wobei als Referenz Tetramethylsilan (0.00 ppm) eingesetzt wurde.

**Herstellbeispiele:**

**Herstellbeispiel 1:** 2-{2-Fluor-4-methyl-5-[(2,2,2-trifluorethyl)sulfinyl]phenyl}-1,2,4-triazin-3,5(2H,4H)-dion (Bsp.Nr.1)

Stufe 1: *Diethyl-[2-(2-{2-fluor-4-methyl-5-[(2,2,2-trifluorethyl)sulfanyl]phenyl}hydrazinyliden)-1,3-dioxopropan-1,3-diyl]biscarbamat*

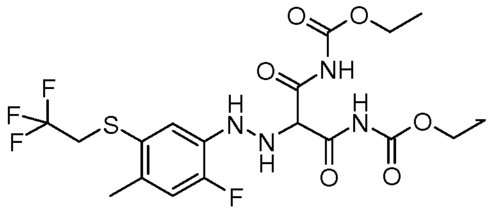

10,0 g (41,7 mmol) 2-Fluor-4-methyl-5-[(2,2,2-trifluorethyl)sulfanyl]anilin wurden bei 0 °C in 100 ml Essigsäure vorgelegt unt mit 12 ml konzentrierter Salzsäure versetzt. Nach der Zugabe von 2,90 g (41,7 mmol) Natriumnitrit und 10,8 g (41,7 mmol) Diethyl-(1,3-dioxopropan-1,3-diyl)biscarbamat wurde das Reaktionsgemisch für 1 Stunde gerührt. Anschließend wurden bei 0 °C 10,3 g (125,1 mmol) Natriumacetat hinzugegeben und über Nacht bei Raumtemperatur nachgerührt. Das Reaktionsgemisch wurde auf Eiswasser gegossen und der entstandene Niederschlag wurde abgesaugt, mit Diethylether gewaschen und unter Vakuum getrocknet. Es wurden 7,00 g (94% Reinheit nach LC/MS, 34% der Theorie) Produkt als brauner Feststoff erhalten.
logP[a]: 4,07; 1H-NMR (D6-DMSO, 400MHz) δ ppm: 13,03(bs,1H), 11,31(bs,1H), 10,49(bs,1H), 8,07(d,1H), 7,32(d,1H), 4,21-4,04(m,6H), 2,35(s,3H), 1,27-1,22(m,6H)

Stufe 2: *Ethyl-[(2-{2-fluor-4-methyl-5-[(2,2,2-trifluorethyl)sulfanyl]phenyl}-3,5-dioxo-2,3,4,5-tetrahydro-1,2,4-triazin-6-yl)carbonyl]carbamat*

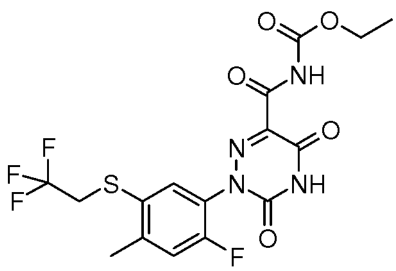

Zu einer Lösung von 7,00 g (13,7 mmol) Diethyl-[2-(2-{2-fluor-4-methyl-5-[(2,2,2-trifluorethyl)sulfanyl]phenyl}hydrazinyliden)-1,3-dioxopropan-1,3-diyl]biscarbamat in 70 ml Essigsäure wurden bei Raumtemperatur 1,12 g (13,7 mmol) Natriumacetat dazu gegeben. Das Reaktionsgemisch wurde 6 Stunden refluxiert und nach Abkühlen auf Eiswasser gegossen. Der Niederschlag wurde abgesaugt, mit Diethylether gewaschen und unter vermindertem Druck getrocknet. So erhielt man 5,00 g (93% Reinheit nach LC/MS, 79% der Theorie) Produkt als leicht bräunlichen Feststoff.
logP[a]: 2,50; 1H-NMR (D6-DMSO, 400MHz) δ ppm: 12,90(bs,1H), 11,52(bs,1H), 7,78(d,1H), 7,44(d,1H), 4,15(q,2H), 3,94(q,2H), 2,45(s,3H), 1,21(t,3H)

Stufe 3: *2-{2-Fluor-4-methyl-5-[(2,2,2-trifluorethyl)sulfanyl]phenyl}-3,5-dioxo-2,3,4,5-tetrahydro-1,2,4-triazin-6-carbonsäure*

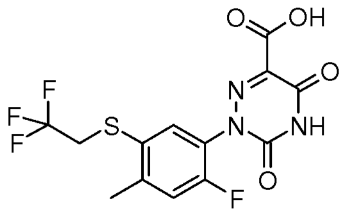

Eine Lösung von 5,00 g (11,1 mmol) Ethyl-[(2-{2-fluor-4-methyl-5-[(2,2,2-trifluorethyl)sulfanyl]phenyl}-3,5-dioxo-2,3,4,5-tetrahydro-1,2,4-triazin-6-yl)carbonyl]carbamat in 50 ml konzentrierter Salzsäure wurde 4 Stunden bei Rückfluss und dann über Nacht bei Raumtemperatur gerührt. Nach Abkühlen wurde das Reaktionsgemisch auf Eiswasser gegossen. Der Niederschlag wurde abgesaugt, mit Diethylether gewaschen und unter vermindertem Druck getrocknet. Isoliert wurden 2,30 g (98% Reinheit nach LC/MS, 55% der Theorie) Produkt als Feststoff.
logP[a]: 1,79; 1H-NMR (D6-DMSO, 400MHz) δ ppm: 12,75(bs,1H), 7,79(d,1H), 7,43(d,1H), 3,91(q,2H), 2,45(s,3H)

Stufe 4: *2-{2-Fluor-4-methyl-5-[(2,2,2-trifluorethyl)sulfanyl]phenyl}-1,2,4-triazin-3,5(2H,4H)-dion*

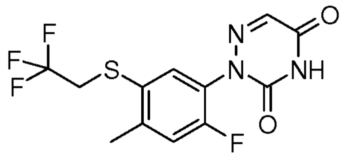

2,30 g (6,06 mmol) 2-{2-Fluor-4-methyl-5-[(2,2,2-trifluorethyl)sulfanyl]phenyl}-3,5-dioxo-2,3,4,5-tetrahydro-1,2,4-triazin-6-carbonsäure wurden in 20 ml Diphenylether vorgelegt und 6 Stunden bei 220 °C gerührt. Nach Abkühlung wurde das Rohgemisch mittels MPLC über Kieselgel (100-200 mesh) mit 20% Essigester in Petrolether aufgereinigt. Isoliert wurden 1,20 g (99% Reinheit nach LC/MS, 59% der Theorie) der Titelverbindung als gelblicher Feststoff.
logP[a]: 2,45; 1H-NMR (D6-DMSO, 400MHz) δ ppm: 12,50(bs,1H), 7,76(d,1H), 7,70(s,1H), 7,41(d,1H), 3,93(q,2H), 2,44(s,3H)

Stufe 5: *2-{2-Fluor-4-methyl-5-[(2,2,2-trifluorethyl)sulfinyl]phenyl}-1,2,4-triazin-3,5(2H,4H)-dion* (Bsp.Nr.1)

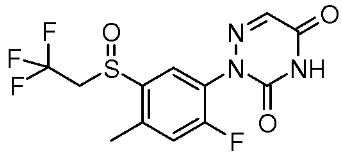

Zu einer Lösung von 200 mg (0,60 mmol) 2-{2-Fluor-4-methyl-5-[(2,2,2-trifluorethyl)sulfanyl]phenyl}-1,2,4-triazin-3,5(2H,4H)-dion in Aceton/Wasser (1:1) wurden 91 mg (0,60 mmol) Oxone(R) gegeben.

Das Reaktionsgemisch wurde 2 Stunden bei Raumtemperatur gerührt, dann mit Wasser verdünnt und mehrmals

mit Essigester extrahiert. Die vereinten organischen Phasen wurden mit Wasser und gesättigter Natriumchloridlösung gewaschen, über Natriumsulfat getrocknet und filtriert. Das Lösungsmittel wurde unter vermindertem Druck entfert. Isoliert wurden 150 mg (98% Reinheit nach LC/MS, 72% der Theorie) der Titelverbindung als gelblicher Feststoff.
logP[a]: 1,58; 1H-NMR (D6-DMSO, 400MHz) δ ppm: 12,45(bs,1H), 8,02(d,1H), 7,71(s,1H), 7,52(d,1H), 4,34-4,22(m,1H),4,04-3,93(m,1H),2,43(s,3H)

**Herstellbeispiel 2:** 2-{2-Fluor-4-methyl-5-[(2,2,2-trifluorethyl)sulfanyl]phenyl}-4-methyl-l,2,4-triazin-3,5(2H,4H)-dion (Bsp.Nr.3)

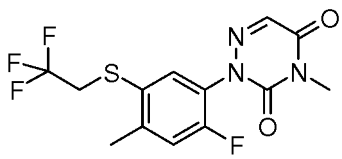

Zu einer gerührten Lösung von 500 mg (1,41 mmol) 2-{2-Fluor-4-methyl-5-[(2,2,2-trifluorethyl)sulfanyl]phenyl}-1,2,4-triazin-3,5(2H,4H)-dionin 5 ml *N*,*N*-Dimethylformamid wurden bei 0 °C 176 μl (400 mg, 2,82 mmol) Methyliodid und 390 mg (2,82 mmol) Kaliumcarbonat gegeben. Das Reaktionsgemisch wurde 3 Stunden bei Raumtemperatur gerührt, dann mit Wasser verdünnt und mehrmals mit Essigester extrahiert. Die vereinten organischen Phasen wurden mit Wasser und gesättigter Natriumchloridlösung gewaschen, über Natriumsulfat getrocknet und filtriert. Das Lösungsmittel wurde unter vermindertem Druck entfert und das Rohprodukt wurde mittels MPLC über Kieselgel (100-200 mesh) mit 26% Essigester in Petrolether aufgereinigt. Isoliert wurden 350 mg (100% Reinheit nach LC/MS, 67% der Theorie) der Titelverbindung als gelblicher Feststoff.
logP[a]: 2,94; 1H-NMR ($CDCl_3$,400MHz) δ ppm: 7,58-7,56(m,2H), 7,14(d,1H), 3,41(s,3H), 3,35(q,2H), 2,54(s,3H)

**Herstellbeispiel 3:** 2-{2-Fluor-4-methyl-5-[(2,2,2-trifluorethyl)sulfinyl]phenyl}-4-methyl-1,2,4-triazin-3,5(2H,4H)-dion (Bsp.Nr.2)

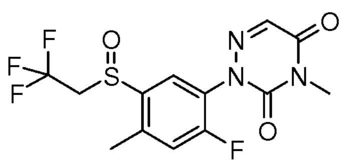

Zu einer Lösung von 200 mg (0,57 mmol) 2-{2-Fluor-4-methyl-5-[(2,2,2-trifluorethyl)sulfanyl]phenyl}-4-methyl-1,2,4-triazin-3,5(2H,4H)-dion in Aceton/Wasser (1:1) wurden 87 mg (0,56 mmol) Oxone(R) gegeben. Das Reaktionsgemisch wurde 2 Stunden bei Raumtemperatur gerührt, dann mit Wasser verdünnt und mehrmals mit Essigester extrahiert. Die vereinten organischen Phasen wurden mit Wasser und gesättigter Natriumchloridlösung gewaschen, über Natriumsulfat getrocknet und filtriert. Das Lösungsmittel wurde unter vermindertem Druck entfert und das Rohprodukt wurde mit n-Pentan gewaschen. Isoliert wurden 170 mg (98% Reinheit nach LC/MS, 81% der Theorie) der Titelverbindung als gelblicher Feststoff.
logP[a]: 1,94; 1H-NMR ($CDCl_3$, 400MHz) δ ppm: 8,06(d,1H), 7,58(s,1H), 7,18(d,1H), 3,49-3,42(m,5H), 2,46(3H)

**Herstellbeispiel 4:** 1-{2-Fluor-4-methyl-5-[(2,2,2-trifluorethyl)sulfanyl]phenyl}-6-methyl-2,4-dioxo-1,2,3,4-tetrahydropyrimidin-5-carbonitril (Bsp.Nr.17)

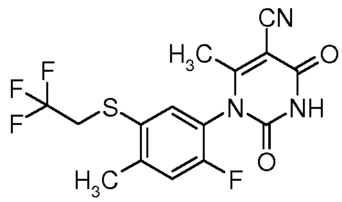

200 mg (0,84 mmol) 2-Fluor-4-methyl-5-[(2,2,2-trifluorethyl)sulfanyl]anilin wurden in 3 ml Dimethylformamid mit 190 mg (0,84 mmol) Ethyl-[(2Z)-2-cyan-3-ethoxybut-2-enoyl]carbamat 18 h unter Rückfluss erhitzt. Nach Entfernen des Lösungsmittels unter vermindertem Druck wurde der verbleibende Rückstand säulenchromatographisch mittels MPLC über RP(C-18) mit Wasser/Acetonitril aufgereinigt. Man erhielt 35 mg (92% Reinheit nach LC/MS, 11% der Theorie) Produkt.
logP[a]: 2,43; 1H-NMR (D6-DMSO, 400MHz) $\delta$ ppm: 12,33(s,1H), 7,83-7,82(m,1H), 7,49-7,47(m,1H), 4,06-3,93(m,2H), 2,43(s,3H), 2,13(s,3H)

**Herstellbeispiel 5:** 1-{2-Fluor-4-methyl-5-[(2,2,2-trifluorethyl)sulfinyl]phenyl} -6-methyl-2,4-dioxo-1,2,3,4-tetrahydropyrimidin-5-carbonitril (Bsp.Nr.16)

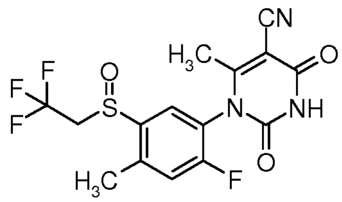

100 mg (0,392 mmol) 2-Fluor-4-methyl-5-[(2,2,2-trifluorethyl)sulfinyl]anilin werden in 10 ml Ethanol und einer katalytischen Menge Essigsäure mit 80 mg (0,354 mmol) Ethyl-[(2Z)-2-cyan-3-ethoxybut-2-enoyl]carbamat [CAS-RN 925982-34-9] 18 h unter Rückfluss erhitzt. Nach dem Abkühlen wird unter vermindertem Druck eingeengt. Der verbleibende Rückstand wird in 5 ml Dimethylformamid aufgenommen und weitere 18 h bei 140°C gerührt. Nach Entfernen des Lösungsmittels unter vermindertem Druck wird der verbleibende Rückstand säulenchromatographisch mittels MPLC über RP(C-18) mit Wasser/Acetonitril aufgereinigt. Man erhält 18,8 mg (83% Reinheit nach LC/MS, 10% der Theorie) Produkt als Diastereomeren-Gemisch.
1H-NMR (D6-DMSO, 400MHz) $\delta$ ppm: 12,31(s,1H), 8,14-8,12(m,1H), 7,61-7,58(m,1H), 4,36-3,88(m,2H), 2,45(s,3H), 2,20(s,3H)

**Herstellbeispiel 6:** 1-{2,4-Diethyl-5-[(2,2,2-trifluorethyl)sulfanyl]phenyl}-6-methyl-2,4-dioxo-1,2,3,4-tetrahydropyrimidin-5-carbonitril (Bsp.Nr. 18)

Stufe 1: *1-{2,4-Diethyl-5-[(2,2,2-trifluorethyl)sulfinyl]phenyl}-6-methyl-2,4-dioxo-1,2,3,4-tetrahydropyrimidin-5-carbonitril*

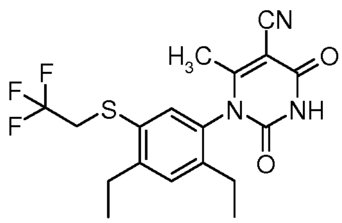

250 mg (0,95 mmol) 2,4-Diethyl-5-[(2,2,2-trifluorethyl)sulfanyl]anilin wurden in 10 ml Ethanol und einer katalytischen Menge Essigsäure mit 195 mg (0,862 mmol) Ethyl-[(2Z)-2-cyan-3-ethoxybut-2-enoyl]carbamat 18 h unter Rückfluss erhitzt. Nach dem Abkühlen wurde das Reaktionsgemisch unter vermindertem Druck eingeengt. Der verbleibende

Rückstand wurde in 5 ml Dimethylformamid aufgenommen und weitere 18 h unter Rückfluß erhitzt. Nach Entfernen des Lösungsmittels unter vermindertem Druck wurde der verbleibende Rückstand säulenchromatographisch mittels MPLC über RP(C-18) mit Wasser/Acetonitril aufgereinigt. Man erhielt 80 mg (94% Reinheit nach LC/MS, 20% der Theorie) Produkt.
logP[a]: 3,16; 1H-NMR (D6-DMSO, 400MHz) δ ppm: 12,19(s,1H), 7,62(s,1H), 7,33(s,1H), 4,05-3,91(m,2H), 2,79-2,73(m,2H), 2,53-2,33(m,2H), 2,01(s,3H), 1,21(t,3H), 1,10(t,3H).

Stufe 2: *1-{2,4-Diethyl-5-[(2,2,2-trifluorethyl)sulfinyl]phenyl}-6-methyl-2,4-dioxo-1,2,3,4-tetrahydropyrimidin-5-carbonitril* (Bsp.Nr. 18)

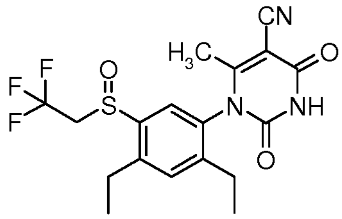

50 mg (0,13 mmol) 1-{2,4-Diethyl-5-[(2,2,2-trifluorethyl)sulfanyl]phenyl}-6-methyl-2,4-dioxo-1,2,3,4-tetrahydropyrimidin-5-carbonitril wurden bei 0 °C in 3 ml Essigsäure vorgelegt. Nach der Zugabe katalytischer Mengen von Natriumwolframat wurden bei 0 °C portionsweise 155 mg (0,137 mmol) 3 % ige wässrige Wasserstoffperoxidlösung dazugegeben und das Reaktionsgemisch wurde 24 h bei Raumtemperatur gerührt. Nach der Zugabe einer 33%igen wäßrigen Bisulfit-Lösung wurde das Gemisch zweimal mit Dichlormethan extrahiert. Die vereinigten organischen Phasen wurden mit Wasser gewaschen, über Natriumsulfat getrocknet und filtriert. Nach Entfernen des Lösungsmittels unter vermindertem Druck wurde der Rückstand säulenchromatographisch mittels MPLC über RP(C-18) aufgereinigt. Mit Wasser/Acetonitril erhielt man 36 mg Produkt als hellgelben Feststoff (89% Reinheit nach LC/MS, 62% der Theorie).
logP[a]: 2,15; logP[b]: 1,55; 1H-NMR (D6-DMSO, 400MHz) δ ppm: 12,18(s,1H), 7,96(s,1H), 7,51(s,1H), 4,29-3,84(m,2H), 2,76(m,2H), 2,51(m,2H), 2,00(s,3H), 1,27(t,3H), 1,15(t,3H).

**Herstellbeispiel 7:** Chirale Oxidation zu (-)- und (+)- 2-[2-Fluor-4-methyl-5-(2,2,2-trifluorethylsulfinyl)phenyl]-4-methyl-1,2,4-triazin-3,5-dione (Bsp.Nr. 28 und 29)

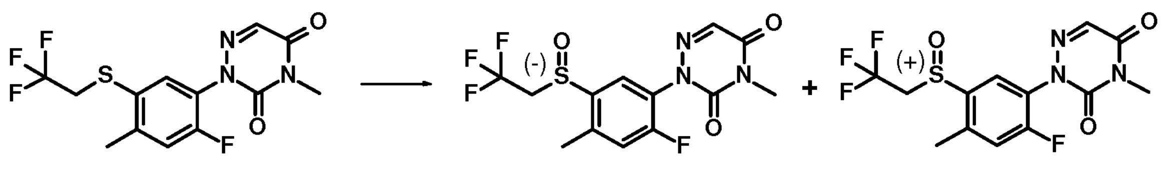

16,0 mg (0,06 mmol) Vanadiumacetylacetonat und 28,6 mg (0,09 mmol) *(S)*-(2,4-Di-*tert*-butyl-6-{(E)-[(1-hydroxy-3,3-dimethylbutan-2-yl)imino]methyl}phenol wurden in 1 mL Chloroform vorgelegt und 1 h bei Raumtemperatur gerührt. 200,0 mg (0,57 mmol) 2-[2-Fluor-4-methyl-5-(2,2,2-trifluorethylsulfanyl)phenyl]-4-methyl-1,2,4-triazin-3,5-dion (Bsp.Nr. 3) wurden in 1 mL Chloroform gelöst und dazu gegeben. Die Lösung wurde weitere 5 Minuten bei Raumtemperatur nachgerührt. Eine Lösung aus 66,8 mg (59 ml, 0,68 mmol) 35%iger $H_2O_2$ und 101 mg (100 ml) Puffer-Lösung pH 7 ($KH_2PO_4$/$Na_2HPO_4$) wurde über 2 Stunden zudosiert. Anschließend weitere 2 Stunden bei Raumtemperatur nachgerührt und über Nacht bei Raumtemperatur rühren lassen. Der Verlauf der Umsetzung wurde mittels DC verfolgt: noch Edukt vorhanden, ca. 50% Umsatz. Erneute Zugabe von 159 ml Lösung $H_2O_2$ / Puffer pH7 über einen Zeitraum von 2 Stunden. Die DC-Kontrolle zeigte keine Veränderung, deshalb wurde die Reaktion abgebrochen, mit 2 ml Chloroform verdünnt und mit ein Paar ml 1M Natriumthiosulfat-Lösung versetzt. Die Phasen wurden getrennt und das Lösungsmittel unter Vakuum entfernt. Man erhielt 199 mg eines Gemisches, das laut LC/MS aus 34% 2-[2-Fluor-4-methyl-5-(2,2,2-trifluorethylsulfanyl)phenyl]-4-methyl-1,2,4-triazin-3,5-dion (Bsp.Nr. 3) und 56% 2-[2-Fluor-4-methyl-5-(2,2,2-trifluorethylsulfinyl)phenyl]-4-methyl-1,2,4-triazin-3,5-dion (Bsp.Nr. 2) bestand.
Der Enantiomerenüberschuss wurde mittels HPLC an chiraler Phase (Chiracel OD-RH 150) mit einem Verhältnis (-)- zu (+)-Enantiomer von 26,1 : 73,9 bestimmt.

(-)-2-[2-Fluor-4-methyl-5-(2,2,2-trifluorethylsulfinyl)phenyl]-4-methyl-1,2,4-triazin-3,5-dion (Bsp.Nr. 28), spezifischer Drehwert: -34,7 in Acetonitril (c=0,009)

(+)-2-[2-Fluor-4-methyl-5-(2,2,2-trifluorethylsulfinyl)phenyl]-4-methyl-1,2,4-triazin-3,5-dion (Bsp.Nr. 29), spezifischer Drehwert: 33,7 in Acetonitril (c=0,009)

**Herstellbeispiel 8:** 1-{2-Fluor-4-methyl-5-[(2,2,2-trifluorethyl)sulfanyl]phenyl}pyrimidin-2,4(1H,3H)-dion (Bsp. Nr. 55)

Stufe 1: *(2E)-3-Ethoxyacryloylchlorid*

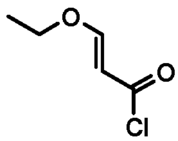

Zu 228 g (1,8 mol) Oxalylchlorid wurden bei 0 °C 86,5 g (1,2 mol) Ethylvinylether so langsam zugetropft, dass die Innentmperatur nicht über 5 °C stieg. Die Reaktionsmischung wurde 2 h bei 0 °C und über Nacht bei Raumtemperatur gerührt, dann unter vermindertem Druck von überschüssigem Oxalylchlorid befreit. Der erhaltene, schwarze, flüssige Rückstand enthielt 179 g 4-Ethoxy-2-oxobut-3-enoylchlorid (ca. 83% Ausbeute bei ca. 90%iger Reinheit).
$^1$H-NMR ($CDCl_3$, 400MHz) $\delta$ ppm: 7,89(d,1H), 6,05(d,1H), 4,12(q,2H), 1,40(t,3H). $^{13}$C-NMR ($CDCl_3$, 400MHz) $\delta$ ppm: 176,9; 169,0; 168,9; 98,9; 69,0; 14,3.

Das 4-Ethoxy-2-oxobut-3-enoylchlorid wurde unter Rühren am Rückflusskühler für 30 min auf 120 °C erhitzt. Das schwarze, flüssige Reaktionsgemisch wurde mittels Destillation gereinigt (0,9-1,3 mbar, Hauptfraktion bei 40-44 °C), man erhielt 88,6 g (90% Reinheit, 49% der Theorie) der Titelverbindung als hellgelbe Flüssigkeit.
$^1$H-NMR ($CDCl_3$, 400MHz) $\delta$ ppm: 7,78(d,1H), 5,51(d,1H), 4,05(q,2H), 1,40(t,3H). $^{13}$C-NMR ($CDCl_3$, 400MHz) $\delta$ ppm: 168,1; 164,7; 102,9; 68,7; 14,4.

Stufe 2: *(2E)-3-Ethoxyacryloylisocyanat*

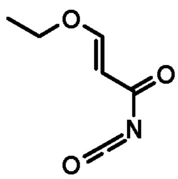

Unter Argonatmosphäre wurden 26,49 g (149,9 mmol) Silbercyanat in trockenem Toluol (120 mL) 30 min zum Rückfluss erhitzt, dann bei ca. 100 °C mit einer Lösung von 11,65 g (86,8 mmol) (2E)-3-Ethoxyacryloylchlorid in trockenem Toluol (30 mL) versetzt. Die Reaktionsmischung wurde 30 min zum Rückfluss erhitzt, dann zunächst auf Raumtemperatur und anschließend auf 0 °C abgekühlt. Die überstehende Lösung der Titelverbindung wurde abdekantiert bzw. mit Hilfe einer Spritze vom Silberchlorid-Niederschlag abgenommen und ohne weitere Aufreinigung weiter umgesetzt, eine Probe wurde nach Konzentration NMR-spektroskopisch analysiert.
$^1$H-NMR ($CDCl_3$, 400MHz) $\delta$ ppm: 7,70(d,1H), 5,29(d,1H), 4,00(q,2H), 1,38(t,3H)$^{13}$C-NMR ($CDCl_3$, 400MHz) $\delta$ ppm: 166,8; 164,3; 139,4; 100,7; 68,0; 14,3.

Stufe 3: *(2E)-3-Ethoxy-N-({2-fluor-4-methyl-5-[(2,2,2-trifluorethyl)sulfanyl]phenyl}carbamoyl) acrylamid*

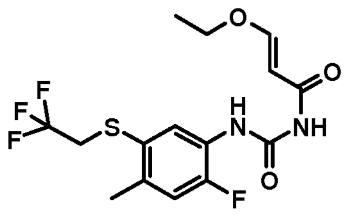

Unter Argonatmosphäre wurden 12,22 g (86,6 mmol) 2-Fluor-4-methyl-5-[(2,2,2-trifluorethyl)-sulfanyl]-anilin in trockenem *N,N*-Dimethylformamid (50 mL) mit 11,8 g aktiviertem 4Å-Molsieb vorgelegt, auf -20 °C abgekühlt und mit der in Stufe 2 erhaltenen toluolischen Lösung von (2E)-3-Ethoxyacryloylisocyanat tropfenweise versetzt, so dass die Innentemperatur nicht über -15 °C stieg. Die Reaktionsmischung wurde über Nacht bei Raumtemperatur gerührt, dann filtriert und unter vermindertem Druck konzentriert. Der erhaltene gelbe Feststoff wurde mit Cyclohexan verrieben, filtriert und mit wenig Cyclohexan gewaschen. Man erhielt 10,6 g (95% Reinheit, 46% der Theorie) der Titelverbindung als gelblichen Feststoff.
logP[a]: 3,78; logP[b]: 3,70; $^1$H-NMR (D$^6$-DMSO, 400MHz) $\delta$ ppm: 11,11(s,1H), 10,60(s,1H), 8,37(d,1H), 7,72(d,1H), 7,29(d,1H), 5,59(d,1H), 4,01(q,2H), 3,79(q,2H), 2,38(s,3H), 1,27(t,3H)

Stufe 4: *1-{2-Fluor-4-methyl-5-[(2,2,2-trifluorethyl)sulfanyl]phenyl}pyrimidin-2,4(1H,3H)-dion (Bsp. Nr. 55)*

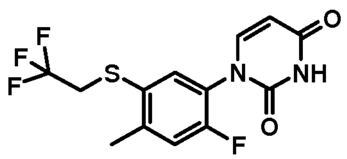

Zu einer Lösung von 10,0 g (26,3 mmol) (2*E*)-3-Ethoxy-*N*-({2-fluor-4-methyl-5-[(2,2,2-trifluorethyl)sulfanyl]phenyl}carbamoyl)acrylamid in Ethanol (37 mL) wurden 117 mL (233 mmol) 2M Schwefelsäure gegeben und die Reaktionsmischung wurde über Nacht unter Rühren zum Rückfluss erhitzt. Nach Abkühlen auf Raumtemperatur wurde Ethanol am Rotationsverdampfer unter Vakuum entfernt und die Schwefelsäurephase mit Dichlormethan versetzt. Der dabei unlösliche farblose Feststoff wurde abgesaugt und getrocknet. Man erhielt 7,50 g (100% Reinheit, 85% der Theorie) der Titelverbindung als hellbraunen Feststoff. Die getrocknete und konzentrierte Dichlormethanphase enthielt nur wenig Produkt und wurde verworfen.
logP[a]: 2,13; logP[b]: 2,06; $^1$H-NMR (D$^6$-DMSO, 400MHz) $\delta$ ppm: 11,58(s,1H), 7,76(d,1H), 7,68(d,1H), 7,39(d,1H), 5,74-5,71(m,1H), 3,99(q,2H), 2,41(s,3H)

**Herstellbeispiel 9:** 1-{2-Fluor-4-methyl-5- [(2,2,2-trifluorethyl)sulfinyl]phenyl}pyrimidin-2,4(1H,3H)-dion (Bsp. Nr. 56)

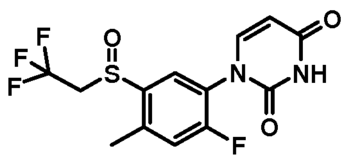

Zu einer Lösung von 1,76 g (5,27 mmol) 1-{2-Fluor-4-methyl-5-[(2,2,2-trifluorethyl)sulfanyl]-phenyl}pyrimidin-2,4(1H,3H)-dion in Acetonitril (35 mL) wurden bei 0 °C 43,4 mg (0,13 mmol) Natriumwolframat(VI)-dihydrat sowie tropfenweise 6,27 g (5,53 mmol) einer 3%igen wässrigen Wasserstoffperoxidlösung gegeben. Die Reaktionsmischung wurde über Nacht bei Raumtemperatur gerührt, dann erneut mit 2,98 g (2,63 mmol) einer 3%igen wässrigen Wasserstoffperoxidlösung versetzt und eine weitere Nacht bei Raumtemperatur gerührt. Es wurde mit Natriumbisulfitlösung (40%ig in Wasser) sowie Dichlormethan versetzt und mit Dichlormethan extrahiert. Die vereinten organischen Phasen wurden mit gesättigter wässriger Ammoniumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhielt man 1,43 g (97% Reinheit, 75% der Theorie) der Titelverbindung als gelblichen Feststoff.

logP[a]: 1,30; logP[b]: 1,24; $^1$H-NMR ($D^6$-DMSO, 400MHz) δ ppm: 11,62(s,1H), 7,98(d,1H), 7,74(d,1H), 7,52(d,1H), 5,79-5,76(m,1H), 4,29-4,16(m,1H), 4,13-4,01(m,1H), 2,44(s,3H)

**Herstellbeispiel 10:** 1-{2-Fluor-4-methyl-5-[(2,2,2-trifluorethyl)sulfinyl]phenyl}-3-methylpyrimidin-2,4(1H,3H)-dion (Bsp. Nr. 54)

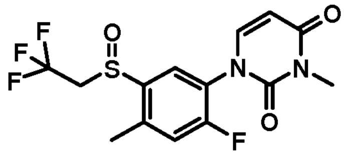

Zu einer Lösung von 200 mg (0,57 mmol) 1-{2-Fluor-4-methyl-5-[(2,2,2-trifluorethyl)sulfinyl]-phenyl}pyrimidin-2,4(1H,3H)-dion in trockenem *N,N*-Dimethylformamid (3 mL) wurden unter Argonatmosphäre bei 0 °C zunächst 243 mg (1,71 mmol) Methyliodid, dann 28,5 mg (0,71 mmol) Natriumhydrid (60%ige Suspension in Mineralöl) gegeben. Die Reaktionsmischung wurde 2 h bei 0 °C gerührt, dann auf Eiswasser gegossen und mit Essigester extrahiert. Die vereinten organischen Phasen wurden mit Wasser, gesättigter wässriger Natriumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhielt man 124 mg (100% Reinheit, 60% der Theorie) der Titelverbindung als farblosen Feststoff.
logP[a]: 1,66; logP[b]: 1,61; $^1$H-NMR ($D^6$-DMSO, 400MHz) δ ppm: 7,99(d,1H), 7,78(d,1H), 7,54(d,1H), 5,92(d,1H), 4,30-4,16(m,1H), 4,12-3,98(m,1H), 3,21(s,3H), 2,45(s,3H)

**Herstellbeispiel 11:** 1-{2-Fluor-4-methyl-5-[(2,2,2-trifluorethyl)sulfanyl]phenyl}-3-methylpyrimidin-2,4(1H,3H)-dion (Bsp. Nr. 53)

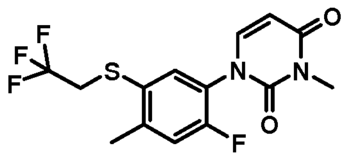

Zu einer Lösung von 1,67 g (5,0 mmol) 1-{2-Fluor-4-methyl-5-[(2,2,2-trifluorethyl)sulfanyl]-phenyl}pyrimidin-2,4(1H,3H)-dion in trockenem *N,N*-Dimethylformamid (15 mL) wurden unter Argonatmosphäre bei 0 °C zunächst 2,13 g (15,0 mmol) Methyliodid, dann 300 mg (7,5 mmol) Natriumhydrid (60%ige Suspension in Mineralöl) gegeben. Die Reaktionsmischung wurde 2 h bei 0 °C bis Raumtemperatur gerührt, dann auf Eiswasser gegossen und mit Essigester extrahiert. Die vereinten organischen Phasen wurden mit Wasser, gesättigter wässriger Natriumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhielt man 1,58 g (100% Reinheit, 91% der Theorie) der Titelverbindung als gelben Feststoff.
logP[a]: 2,59; logP[b]: 2,53; $^1$H-NMR ($D^6$-DMSO, 400MHz) δ ppm: 7,77(d,1H), 7,74(d,1H), 7,41(d,1H), 5,88(d,1H), 3,98(q,2H), 3,21(s,3H), 2,43(s,3H)

**Herstellbeispiel 12:** 3-Ethyl-1-{2-fluor-4-methyl-5-[(2,2,2-trifluorethyl)sulfanyl]phenyl}pyrimidin-2,4(1H,3H)-dion (Bsp. Nr. 71)

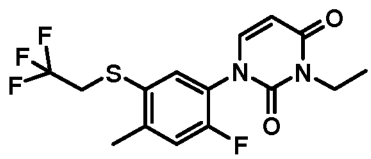

Zu einer Lösung von 186 mg (0,56 mmol) 1-{2-Fluor-4-methyl-5-[(2,2,2-trifluorethyl)sulfanyl]-phenyl}pyrimidin-2,4(1H,3H)-dion in trockenem *N*,*N*-Dimethylformamid (3 mL) wurden 33,4 mg (0,84 mmol) Natriumhydrid (60%ige Suspension in Mineralöl) gegeben, 15 min bei Raumtemperatur gerührt, dann 174 mg (1,11 mmol) Ethyliodid zugesetzt und bei Raumtemperatur gerührt. Nach 1h wurden erneut 22,3 mg (0,56 mmol) Natriumhydrid (60%ige Suspension in Mineralöl) sowie 86,8 mg (0,56 mmol) Ethyliodid zugegeben und über Nacht bei Raumtemperatur gerührt, dann auf Eiswasser gegossen und mit Essigester extrahiert. Die vereinten organischen Phasen wurden mit Wasser, gesättigter wässriger Natriumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhielt man 40,6 mg (97% Reinheit, 20% der Theorie) der Titelverbindung als farblosen Feststoff.
logP[a]: 2,93; logP[b]: 2,86; $^1$H-NMR ($D^6$-DMSO, 400MHz) $\delta$ ppm: 7,78(d,1H), 7,72(d,1H), 7,41(d,1H), 5,86(d,1H), 3,99(q,2H), 3,86(q,2H), 2,42(s,3H), 1,12(t,3H)

**Herstellbeispiel 13:** 3-Ethyl-1-{2-fluor-4-methyl-5-[(2,2,2-trifluorethyl)sulfinyl]phenyl}pyrimidin-2,4(1H,3H)-dion (Bsp. Nr. 59)

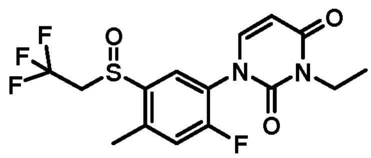

Zu einer Lösung von 208 mg (0,57 mmol) 3-Ethyl-1-{2-fluor-4-methyl-5-[(2,2,2-trifluorethyl)sulfanyl]-phenyl}pyrimidin-2,4(1H,3H)-dion in 5 mL Dichlormethan wurden bei 0 °C 135 mg (0,60 mmol) *meta*-Chlorperbenzoesäure (ca. 77%ig) gegeben. Die Reaktionsmischung wurde 2 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhielt man 145 mg (100% Reinheit, 67% der Theorie) der Titelverbindung als farblosen Feststoff.
logP[a]: 1,94; logP[b]: 1,88; $^1$H-NMR ($D^6$-DMSO, 400MHz) $\delta$ ppm: 8,00(d,1H), 7,77(d,1H), 7,53(d,1H), 5,90(d,1H), 4,30-4,12(m,1H), 4,12-3,99(m,1H), 3,87(q,2H), 2,45(s,3H), 1,13(t,3H)

**Herstellbeispiel 14:** 3-Methyl-1-{4-methyl-3-[(2,2,2-trifluorethyl)sulfanyl]phenyl}pyrimidin-2,4(1H,3H)-dion (Bsp. Nr. 58)

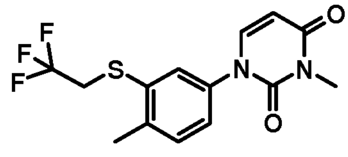

375 mg (1,5 mmol) {4-Methyl-3-[(2,2,2-trifluorethyl)sulfanyl]phenyl}borsäure, 189 mg (1,5 mmol) 3-Methyluracil, 409 mg (2,25 mmol) Kupfer(II)-acetat, 237 mg (3,0 mmol) Pyridin sowie 1,0 g aktiviertes 3Å-Molsieb wurden in 20 mL trockenem Dichlormethan 4 d bei Raumtemperatur gerührt. Die Reaktionsmischung wurde auf Kieselgur adsorbiert und säulenchromatographisch an Kieselgel mittels MPLC mit Cyclohexan/Essigester als Laufmittel gereinigt. Man erhielt 176 mg (93% Reinheit, 33% der Theorie) der Titelverbindung als farbloses Öl.
logP[a]: 2,48; logP[b]: 2,39; $^1$H-NMR ($D^6$-DMSO, 400MHz) $\delta$ ppm: 7,71(d,1H), 7,61(d,1H), 7,37(d,1H), 7,27-7,24(m,1H), 5,83(d,1H), 4,04(q,2H), 3,20(s,3H), 2,38(s,3H)

**Herstellbeispiel 15:** 3-Methyl-1-{4-methyl-3-[(2,2,2-trifluorethyl)sulfinyl]phenyl}pyrimidin-2,4(1H,3H)-dion (Bsp. Nr. 57)

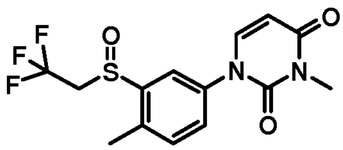

Zu einer Lösung von 88,0 mg (0,27 mmol) 3-Methyl-1-{4-methyl-3-[(2,2,2-trifluorethyl)sulfanyl]phenyl}pyrimidin-2,4(1H,3H)-dion in 10 mL Dichlormethan wurden bei 0 °C 62,7 mg (0,28 mmol) *meta*-Chlorperbenzoesäure (ca. 77%ig) gegeben. Die Reaktionsmischung wurde 2 h bei 0 °C gerührt, mit 1M Natronlauge (5 mL) gewaschen und unter vermindertem Druck vom Lösungsmittel befreit. Man erhielt 82 mg (100% Reinheit, 89% der Theorie) der Titelverbindung als farblosen Feststoff.
logP[a]: 1,50; logP[b]: 1,49; $^1$H-NMR (D$^6$-DMSO, 400MHz) δ ppm: 7,87(d,1H), 7,80(d,1H), 7,61-7,58(m,1H), 7,49(d,1H), 5,88(d,1H), 4,30-4,12(m,1H), 4,09-3,95(m,1H), 3,21(s,3H), 2,42(s,3H)

**Synthese von Anilinen der Formel (IVa)**

**2,4-Diethyl-5-[(2,2,2-trifluorethyl)sulfanyl]anilin**

Stufe 1: *5-N-(2,4-Diethylphenyl)acetamid*

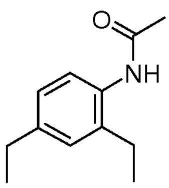

Zu einer Lösung von 8,40 g (56,3 mmol) 2,4-Diethylanilin in 150 ml Toluol wurden langsam 6,00 g (58,8 mmol) Essigsäureanhydrid zugetropft, anschließend wurde noch 18 h bei Raumtemperatur nachgerührt. Es wurde im Vakuum vom Lösungsmittel befreit und der verbleibende Feststoffbrei wurde mit Wasser verrührt und abgesaugt. Man erhielt 9,50 g Produkt (100% nach 1H-NMR, 88% der Theorie).
1H-NMR (D6-DMSO, 400MHz) δ ppm: 9,20(breit,1H), 7,20-7,18(m,1H), 7,04(s,1H), 6,99-6,97(m,1H), 2,55(m,4H), 2,02(s,3H), 1,16(t,3H), 1,10(t,3H)

Stufe 2: *5-Acetamido-2,4-diethylbenzolsulfonylchlorid*

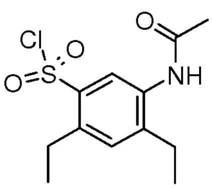

9,50 g (49,7 mmol) 5-*N*-(2,4-Diethylphenyl)acetamid wurden portionsweise zu 30,0 g (257,5 mmol) Chlorsulfonsäure gegeben und 4 h bei 80°C nachgerührt. Nach dem Abkühlen wurde auf Eiswasser gegeben und der erhaltene Feststoff abgesaugt, es verblieben 10,3 g Produkt (100% Reinheit nach 1H-NMR, 72% der Theorie).
1H-NMR (D6-DMSO, 400MHz) δ ppm: 9,29(breit,1H), 7,58(s,1H), 7,00(s,1H), 2,95(q,2H), 2,51(q,2H), 2,02(s,3H), 1,16(t,3H), 1,08(t,3H)

Stufe 3: *NN'-[Disulfanediylbis(4,6-diethylbenzol-3,1-diyl)]diacetamid*

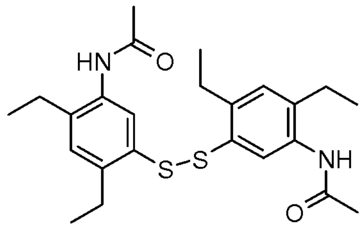

13,4 g (46,2 mmol) 5-Acetamido-2,4-diethylbenzolsulfonylchlorid wurden mit 7,40 g (132,5 mmol) Eisenpulver in 150 ml Ethanol und 18,7 g konzentrierter Salzsäure 12 h unter Rückfluss erhitzt. Nach dem Entfernen des Lösungsmittels unter vermindertem Druck wurde der Rückstand mit Wasser verrührt und abgesaugt und man erhielt 10,3 g Rohprodukt (73% Reinheit nach LC/MS, 50% der Theorie) als hellbraunen Feststoff.
logP(HCOOH): 4,02

Stufe 4: N-{*2,4-Diethyl-5-[(2,2,2-trifluorethyl)sulfanyl]phenyl}acetamid*

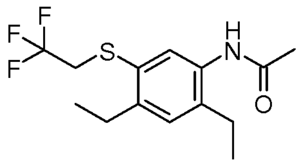

10,3 g (23,16 mmol) N,N'-[Disulfanediylbis(4,6-diethylbenzol-3,1-diyl)]diacetamid werden in 60 ml Dimethylformamid vorgelegt und mit 6,3 g Natrium-Dithionit, 15,5 g Kaliumcarbonat und 5,3 g Natriumhydrogenphosphat und 40 ml Wasser versetzt und anschliessend 3 h bei 60°C gerührt. Nach dem Abkühlen werden 12 g (57,16 mmol) 1,1,1-Trifluor-2-iodethan dazugegeben und weitere 12 h bei 80°C gerührt. Nach Entfernen des Lösungsmittels unter vermindertem Druck wird der verbleibende Rückstand mit konzentrierter Salzsäure angesäuert und der entstandene graue Niederschlag abgesaugt. Es verbleiben 11,9 g Rohrodukt (70% Reinheit nach LC/MS, 84% der Theorie,).
logP(HCOOH): 3,07

Stufe 5: 2,4-Diethyl-5-[(2,2,2-trifluorethyl)sulfanyl]anilin

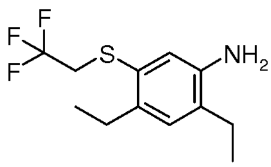

11,9 g (39,0 mmol) N-{2,4-Diethyl-5-[(2,2,2-trifluorethyl)sulfanyl]phenyl}acetamid werden in 125 ml 5 molarer Salzsäure 18 h unter Rückfluß gerührt. Das Reaktionsgemisch wird mit Natronlauge basisch gestellt und mit Dichlormethan extrahiert. Die organische Phase wird abgetrennt, über Natriumsulfat getrocknet und unter Vakuum vom Lösungsmittel befreit. Es verbleiben 5,6 g Produkt als braunes Öl (100 % Reinheit nach 1H-NMR, 55% der Theorie).
1H-NMR (D6-DMSO, 400MHz) $\delta$ ppm: 6,81-6,79(m,2H), 4,77(breit,2H), 3,75-3,67(q,2H), 2,63-2,58(q,2H), 2,43-2,37(q,2H), 1,10(t,3H), 1,09(t,3H)

**Synthese von Estern der Formel (I-A-IIIa-Ester)**

Ethyl-3-cyclopropyl-1-{2-fluor-4-methyl-5-[(2,2,2-trifluorethyl)sulfanyl]phenyl}-2,4-dioxo-1,2,3,4-tetrahydropyrimidin-5-carboxylat

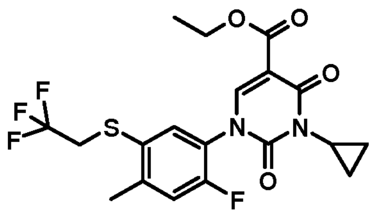

Zu einer Lösung von 1,20 g (5,0 mmol) 2-Fluor-4-methyl-5-[(2,2,2-trifluorethyl)-sulfanyl]-anilin in trockenem Tetrahydrofuran (20 mL) wurden bei 0 °C unter Argonatmosphäre 457 mg (5,0 mmol) Cyclopropylisocyanat gegeben, dann 30 min bei 0 °C, über Nacht bei Raumtemperatur sowie 2 h bei 45 °C und 2 h bei 55 °C gerührt. Nach erneuter Zugabe von 457 mg (5,0 mmol) Cyclopropylisocyanat sowie wenigen Tropfen Hünigbase wurde die Reaktionsmischung über Nacht bei Raumtemperatur gerührt, dann unter vermindertem Druck konzentriert. Reaktionskontrolle mittels LC-MS zeigte Bildung des Intermediats 1-Cyclopropyl-3-{2-fluor-4-methyl-5-[(2,2,2-trifluorethyl)sulfanyl]phenyl}harnstoff an. Der Rückstand wurde in Ethanol (20 mL) aufgenommen, mit 1,14 g (5,25 mmol) Ethoxymethylenmalonsäurediethylester und 1,70 g (5,35 mmol) Natriumethylat-Lösung (21%ig in Ethanol) versetzt, über Nacht bei Raumtemperatur und 2 h bei 55 °C gerührt. Nach Abkühlen auf Raumtemperatur wurde die Reaktionsmischung mit Salzsäure auf pH=6 eingestellt, Ethanol unter vermindertem Druck entfernt und mit Essigester extrahiert. Die vereinten organischen Phasen wurden mit gesättigter wässriger Natriumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhielt man 862 mg (92% Reinheit, 36% der Theorie) der Titelverbindung als gelben harzigen Feststoff.
logP[a]: 3,07; logP[b]: 3,02; $^1$H-NMR (D$^6$-DMSO, 400MHz) $\delta$ ppm: 8,34(s,1H), 7,61(d,1H), 7,38(d,1H), 4,21(q,2H), 3,87(q,2H), 3,29-3,20(m,1H), 2,45(s,3H), 1,25(t,3H), 1,03-0,90(m,4H)

Gemäß den zuvor beschriebenen Verfahren wurden die folgenden Verbindungen der allgemeinen Formel (I) hergestellt.

**Tabelle 1** (Verbindungen der Formel (I) mit Substruktur (I-A-1))

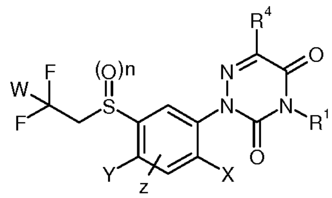

Z=H, W=F und $R^4$=H

| Bsp.Nr. | n | Y | X | $R^1$ |
|---|---|---|---|---|
| 1 | 1 | $CH_3$ | F | H |
| 2 | 1 | CH3 | F | CH3 |
| 3 | 0 | CH3 | F | CH3 |
| 4 | 1 | CH3 | CH3 | CH3 |
| 5 | 0 | CH3 | CH3 | CH3 |
| 6 | 1 | CH3 | CH3 | CH2CF3 |
| 7 | 0 | CH3 | CH3 | CH2CF3 |
| 8 | 0 | Cl | Cl | H |
| 9 | 1 | CH3 | F | CH2CF3 |
| 10 | 0 | CH3 | F | CH2CF3 |
| 11 | 1 | Cl | Cl | CH2CF3 |
| 12 | 0 | Cl | Cl | $CH_2CF_3$ |

(fortgesetzt)

| Bsp.Nr. | n | Y | X | $R^1$ |
|---|---|---|---|---|
| 13 | 1 | Cl | Cl | $CH_3$ |
| 14 | 0 | Cl | Cl | $CH_3$ |
| 15 | 1 | Cl | Cl | H |
| 19 | 0 | $CH_3$ | F | $CH_2CH_3$ |
| 20 | 0 | $CH_3$ | F | $CH_2CH(CH_3)_2$ |
| 21 | 1 | $CH_3$ | F | $CH_2CH_3$ |
| 22 | 1 | $CH_3$ | F | $CH_2CH(CH_3)_2$ |
| 23 | 0 | $CH_3$ | H | $CH_3$ |
| 24 | 1 | $CH_3$ | H | $CH_3$ |
| 25 | 0 | $CH_3$ | F | Cyclopropylmethyl |
| 26 | 0 | $CH_3$ | F | $CH(CH_3)_2$ |
| 27 | 1 | $CH_3$ | F | Cyclopropylmethyl |
| 28 | 1 | $CH_3$ | F | $CH_3$ |
| 29 | 1 | $CH_3$ | F | $CH_3$ |
| 30 | 1 | $CH_3$ | F | $CH(CH_3)_2$ |
| 31 | 0 | Cl | H | $CH_3$ |
| 32 | 0 | Cl | H | $CH_3$ |
| 33 | 0 | $CH_3$ | $CH_3$ | $CH_2CH_3$ |
| 34 | 0 | $CH_3$ | $CH_3$ | Cyclopropylmethyl |
| 35 | 1 | $CH_3$ | $CH_3$ | $CH_2CH_3$ |
| 36 | 0 | $CH_3$ | $CH_3$ | $CH(CH_3)_2$ |
| 37 | 1 | $CH_3$ | $CH_3$ | Cyclopropylmethyl |
| 38 | 1 | $CH_3$ | $CH_3$ | $CH(CH_3)_2$ |
| 39 | 0 | $CH_3$ | F | $CH_2CN$ |
| 40 | 0 | $CH_3$ | $CH_3$ | $CH_2CN$ |
| 41 | 0 | $CH_3$ | $CH_3$ | $CH_2CH(CH_3)_2$ |
| 42 | 0 | $CH_3$ | F | Benzyl |
| 43 | 0 | $CH_3$ | F | 3-Pyridylmethyl |
| 44 | 1 | $CH_3$ | F | $CH_2CN$ |
| 45 | 1 | $CH_3$ | F | Benzyl |
| 46 | 1 | $CH_3$ | $CH_3$ | $CH_2CF_3$ |
| 47 | 1 | $CH_3$ | $CH_3$ | $CH_2CF_3$ |
| 48 | 1 | $CH_3$ | F | N-Oxid-3-Pyridylmethyl |
| 49 | 1 | $CH_3$ | F | 3-Pyridylmethyl |
| 50 | 1 | $CH_3$ | F | $CH_2CF_3$ |
| 51 | 1 | $CH_3$ | F | $CH_2CF_3$ |
| 52 | 1 | $CH_3$ | $CH_3$ | Benzyl |

**Tabelle 2** (Verbindungen der Formel (I) mit Substruktur (I-A-9)

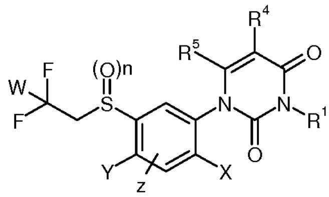

Z=H und W=F

| Bsp.Nr. | n | Y | X | $R^1$ | $R^4$ | $R^5$ |
|---|---|---|---|---|---|---|
| 16 | 1 | $CH_3$ | F | H | CN | $CH_3$ |
| 17 | 0 | $CH_3$ | F | H | CN | $CH_3$ |
| 18 | 1 | $CH_2CH_3$ | $CH_2CH_3$ | H | CN | $CH_3$ |
| 53 | 0 | $CH_3$ | F | $CH_3$ | H | H |
| 54 | 1 | $CH_3$ | F | $CH_3$ | H | H |
| 55 | 0 | $CH_3$ | F | H | H | H |
| 56 | 1 | $CH_3$ | F | H | H | H |
| 57 | 1 | $CH_3$ | H | $CH_3$ | H | H |
| 58 | 0 | $CH_3$ | H | $CH_3$ | H | H |
| 59 | 1 | $CH_3$ | F | $CH_2CH_3$ | H | H |
| 60 | 1 | $CH_3$ | F | $CH_2CF_3$ | H | H |
| 61 | 1 | $CH_3$ | F | $CH_2CN$ | H | H |
| 62 | 1 | $CH_3$ | F | $CH_2$-Cyclopropyl | H | H |
| 63 | 1 | $CH_3$ | F | $CH(CH_3)_2$ | H | H |
| 64 | 1 | $CH_3$ | F | $CH_2$-4-Fluorophenyl | H | H |
| 65 | 1 | $CH_3$ | F | $CH_2CH{=}CH_2$ | H | H |
| 66 | 1 | $CH_3$ | F | $CH_2CH_2CH_3$ | H | H |
| 67 | 1 | $CH_3$ | F | $CH_2CCH$ | H | H |
| 68 | 1 | $CH_3$ | F | $CH_2OCH_3$ | H | H |
| 69 | 1 | $CH_3$ | F | $CH_2CH(CH_3)_2$ | H | H |
| 70 | 1 | $CH_3$ | F | $CH_2CHF_2$ | H | H |
| 71 | 0 | $CH_3$ | F | $CH_2CH_3$ | H | H |
| 72 | 0 | $CH_3$ | F | $CH_2CF_3$ | H | H |
| 73 | 0 | $CH_3$ | F | $CH_2CN$ | H | H |
| 74 | 0 | $CH_3$ | F | $CH_2$-Cyclopropyl | H | H |
| 75 | 0 | $CH_3$ | F | $CH(CH_3)_2$ | H | H |
| 76 | 0 | $CH_3$ | F | $CH_2$-4-Fluorophenyl | H | H |
| 77 | 0 | $CH_3$ | F | $CH_2CH{=}CH_2$ | H | H |
| 78 | 0 | $CH_3$ | F | $CH_2CH_2CH_3$ | H | H |
| 79 | 0 | $CH_3$ | F | $CH_2CCH$ | H | H |
| 80 | 0 | $CH_3$ | F | $CH_2OCH_3$ | H | H |

(fortgesetzt)

| Bsp.Nr. | n | Y | X | $R^1$ | $R^4$ | $R^5$ |
|---|---|---|---|---|---|---|
| 81 | 0 | $CH_3$ | F | $CH_2CH(CH_3)_2$ | H | H |
| 82 | 0 | $CH_3$ | F | $CH_2CHF_2$ | H | H |
| 83 | 0 | $CH_3$ | H | $CH_2CH_3$ | H | H |
| 84 | 0 | $CH_3$ | H | $CH_2CF_3$ | H | H |
| 85 | 0 | $CH_3$ | H | $CH_2CN$ | H | H |
| 86 | 0 | $CH_3$ | H | $CH_2$-Cyclopropyl | H | H |
| 87 | 0 | $CH_3$ | H | $CH(CH_3)_2$ | H | H |
| 88 | 0 | $CH_3$ | H | H | H | H |
| 89 | 0 | $CH_3$ | $CH_3$ | H | H | H |
| 90 | 1 | $CH_3$ | H | $CH_2CF_3$ | H | H |
| 91 | 1 | $CH_3$ | H | H | H | H |
| 92 | 0 | $CH_3$ | $CH_3$ | $CH_3$ | H | H |
| 93 | 1 | $CH_3$ | $CH_3$ | $CH_3$ | H | H |
| 94 | 0 | $CH_3$ | $CH_3$ | $CH_2CF_3$ | H | H |
| 95 | 1 | $CH_3$ | $CH_3$ | $CH_2CF_3$ | H | H |
| 96 | 0 | $CH_3$ | $CH_3$ | $CH_2CH_3$ | H | H |
| 97 | 1 | $CH_3$ | $CH_3$ | $CH_2CH_3$ | H | H |
| 98 | 1 | $CH_3$ | H | $CH_2CH_3$ | H | H |
| 99 | 1 | $CH_3$ | $CH_3$ | H | H | H |
| 100 | 1 | $CH_3$ | H | $CH_2CN$ | H | H |

NMR-Daten der Verbindungen gemäß Tabellen 1 und 2:

| Bsp.Nr. | LogP[b] | LogP[a] | 1H-NMR $\delta$ ppm |
|---|---|---|---|
| 1 | | 1,58 | (D6-DMSO, 400MHz) 12,45(bs,1H), 8,02(d,1H), 7,71(s,1H), 7,52(d,1H), 4,34-4,22(m,1H), 4,04-3,93(m,1H), 2,43(s,3H) |
| 2 | | 1,94 | ($CDCl_3$, 400MHz) 8,06(d,1H), 7,58(s,1H), 7,18(d,1H), 3,49-3,42(m,5H), 2,46 (s,3H) |
| 3 | | 2,94 | ($CDCl_3$, 400MHz) 7,58-7,56(m,2H), 7,14(d,1H), 3,41(s,3H), 3,35(q,2H), 2,54 (s,3H) |
| 4 | | 2,01 | ($CDCl_3$, 400MHz) 7,88(bs,1H), 7,56(bs,1H), 7,25(bs,1H), 3,48-3,40(m,5H), 2,41(s,3H), 2,26(s,3H) |
| 5 | | 3,08 | ($CDCl_3$, 400MHz) 7,55(s,1H), 7,34(s,1H), 7,21(s,1H), 3,42-3,33(m,5H), 2,49 (s,3H), 2,17(s,3H) |
| 6 | | 2,73 | (D6-DMSO, 400MHz) 7,89(s,2H), 7,40(s,1H), 4,71-4,64(m,2H), 4,27-4,21(m, 1H), 3,94-3,87(m,1H), 2,39(s,3H), 2,21(s,3H) |
| 7 | | 3,74 | ($CDCl_3$, 400MHz) 7,61(s,1H), 7,40(s,1H), 7,22(s,1H), 4,70(q,2H), 3,37(q,2H), 2,49(s,3H), 2,16(s,3H) |
| 8 | 1,4 | 2,72 | ($CDCl_3$, 400MHz) 8,58(bs,1H), 7,66(s,1H), 7,60(s,1H), 7,57(s,1H), 3,50(q, 2H) |
| 9 | | 2,65 | ($CDCl_3$, 400MHz) 8,08(d,1H), 7,65(s,1H), 7,19(d,1H), 4,68(q,2H) |

(fortgesetzt)

| Bsp.Nr. | LogP[b] | LogP[a] | 1H-NMR δ ppm |
|---|---|---|---|
| 10 | | 3,65 | ($CDCl_3$, 400MHz) 7,63-7,59(m,2H), 7,15(d,1H), 4,68(q,2H), 3,36(q,2H), 2,54 (s,3H) |
| 11 | | 3,21 | ($CDCl_3$, 400MHz) 8,06(s,1H), 7,70(s,1H), 7,66(s,1H), 4,69(q,2H), 3,84-3,78 (m,1H), 3,48-3,43(m,1H) |
| 12 | | 3,93 | ($CDCl_3$, 400MHz) 7,66-7,61(m,3H), 4,68(q,2H), 3,50(q,2H) |
| 13 | | 2,48 | ($CDCl_3$, 400MHz) 8,04(m,1H), 7,69(m,1H), 7,59(m,1H), 3,86-3,75(m,1H), 3,50-3,39(m,4H) |
| 14 | | 3,31 | ($CDCl_3$, 400MHz) 7,65(s,1H), 7,59-7,57(m,2H), 3,49(q,2H), 3,42(s,3H) |
| 15 | 1,1 | 2,02 | ($CDCl_3$, 400MHz) 8,66(bs,1H), 8,06(s,1H), 7,69(s,1H), 7,58(s,1H), 3,86-3,75 (m,1H), 3,52-3,41(m,1H) |
| 16 | 0,88 | 1,58 | (D6-DMSO, 400MHz) 12,31(s,1H), 8,14-8,12(m,1H), 7,61-7,58(m,1H), 4,36-3,88(m,2H), 2,45(s,3H), 2,20(s,3H) |
| 17 | | 2,43 | (D6-DMSO, 400MHz) 12,33(s,1H), 7,83-7,82(m,1H), 7,49-7,47(m,1H), 4,06-3,93(m,2H), 2,43(s,3H), 2,13(s,3H) |
| 18 | 1,55 | 2,15 | (D6-DMSO, 400MHz) 12,18(s,1H), 7,96(s,1H), 7,51(s,1H), 4,29-3,84(m,2H), 2,76(m,2H), 2,51(m,2H), 2,00(s,3H), 1,27(t,3H), 1,15(t,3H) |
| 19 | 3,27 | 3,31 | (D6-DMSO, 400MHz) 7,79-7,76(m,2H), 7,42(d,1H), 3,96-3,83(m,4H), 2,45 (s,3H), 1,16(t,3H) |
| 20 | 4 | 4,03 | (D6-DMSO, 400MHz) 7,80-7,76(m,2H), 7,42(d,1H), 3,92(q,2H), 3,67(d,2H), 2,45(s,3H), 2,07-2,00(m,1H), 0,89(d,6H) |
| 21 | 2,22 | 2,24 | (D6-DMSO, 400MHz) 8,04(d,1H), 7,80(s,1H), 7,53(d,1H), 4,32-4,23(m,1H), 4,03-3,83(m,3H), 2,44(s,3H), 1,67(t,3H) |
| 22 | 2,9 | 2,92 | (D6-DMSO, 400MHz) 8,05(d,1H), 7,81(s,1H), 7,53(d,1H), 4,32-4,26(m,1H), 4,01-3,95(m,1H), 3,66(d,2H), 2,43(s,3H), 2,07-2,01(m,1H), 0,89(d,6H) |
| 23 | 2,83 | 2,9 | (D6-DMSO, 400MHz) 7,75(s,1H), 7,67(d,1H), 7,40-7,32(m,2H), 3,95(q,2H), 3,21(s,3H), 2,41(s,3H) |
| 24 | 1,83 | 1,84 | (D6-DMSO, 400MHz) 8,00(d,1H), 7,79(s,1H), 7,66(dd,1H), 7,49(d,1H), 4,27-4,21(m,1H), 3,97-3,88(m,1H), 3,22(s,3H), 2,41(s,3H) |
| 25 | 3,76 | 3,79 | (D6-DMSO, 400MHz) 7,82-7,79(m,2H), 7,43(d,1H), 3,93(q,2H), 3,72(d,2H), 2,45(s,3H), 1,19-1,12(m,1H), 0,50-0,46(m,2H), 0,36-0,33(m,2H) |
| 26 | 3,67 | 3,73 | (D6-DMSO, 400MHz) 7,77(d,1H), 7,71(s,1H), 7,41(d,1H), 4,98-4,91(m,1H), 3,92(q,2H), 2,45(s,3H), 1,41(d,6H) |
| 27 | 2,65 | 2,72 | (D6-DMSO, 400MHz) 8,06(d,1H), 7,83(s,1H), 7,54(d,1H), 4,32-4,26(m,1H), 4,05-3,96(m,1H), 3,72(d,2H), 2,44(s,3H), 1,19-1,13(m,1H), 0,51-0,46(m,2H), 0,37-0,33(m,2H) |
| 28 | 1,9 | 1,95 | (D6-DMSO, 400MHz) 8,02(d,1H), 7,81(s,1H), 7,54(d,1H), 4,32-3,93(m,m, 2H), 3,21(s,3H), 2,43(s,3H) |
| 29 | 1,9 | 1,95 | (D6-DMSO, 400MHz) 8,02(d,1H), 7,81(s,1H), 7,54(d,1H), 4,32-3,93(m,m, 2H), 3,21(s,3H), 2,43(s,3H) |
| 30 | 2,58 | 2,64 | (D6-DMSO, 400MHz) 8,04(d,1H), 7,72(s,1H), 7,52(d,1H), 4,98-4,91(m,1H), 4,31-4,25(m,1H), 4,03-3,94(m,1H), 2,43(s,3H), 1,41(d,6H) |
| 31 | 2,91 | 3 | (D6-DMSO, 400MHz) 7,82-7,79(m,2H), 7,67(d,1H), 7,43(dd,1H), 4,10(q,2H), 3,22(s,3H) |

(fortgesetzt)

| Bsp.Nr. | LogP[b] | LogP[a] | 1H-NMR δ ppm |
|---|---|---|---|
| 32 | 2,16 | 2,18 | (D6-DMSO, 400MHz) 8,05(d,1H), 7,83-7,81(m,3H), 4,38-4,31(m,1H), 4,11-4,04(m,1H), 3,22(s,3H) |
| 33 | 3,38 | 3,45 | (D6-DMSO, 400MHz) 7,73(s,1H), 7,57(s,1H), 7,28(s,1H), 3,93-3,83(m,4H), 2,39(s,3H), 2,09(s,3H), 1,17(t,3H) |
| 34 | 3,87 | 3,94 | (D6-DMSO, 400MHz) 7,76(s,1H), 7,59(s,1H), 7,28(s,1H), 3,91(q,2H), 3,72 (d,2H), 2,39(s,3H), 2,10(s,3H), 1,19-1,14(m,1H), 0,50-0,45(m,2H), 0,37 -0,33 (m,2H) |
| 35 | 2,27 | 2,31 | (D6-DMSO, 400MHz) 7,86(s,1H), 7,73(s,1H), 7,38(s,1H), 4,26-4,10(m,1H), 3,93-3,83(m,3H), 2,39(s,3H), 2,20(s,3H), 1,24-1,15(m,3H) |
| 36 | 3,81 | 3,88 | (D6-DMSO, 400MHz) 7,66(s,1H), 7,57(s,1H), 7,27(s,1H), 5,00-4,93(m,1H), 3,89(q,2H), 2,39(s,3H), 2,09(s,3H), 1,42(d,6H) |
| 37 | 2,73 | 2,77 | (D6-DMSO, 400MHz) 7,87(s,1H), 7,77(s,1H), 7,39(s,1H), 4,25-4,16(m,1H), 3,95-3,89(m,1H), 3,72(d,2H), 2,39(s,3H), 2,21(s,3H), 1,20-1,14(m,1H), 0,50-0,46(m,2H), 0,37-0,33(m,2H) |
| 38 | 2,67 | 2,69 | (D6-DMSO, 400MHz) 7,86(s,1H), 7,66(s,1H), 7,37(s,1H), 4,99-4,92(m,1H), 4,25-4,18(m,1H), 3,93-3,87(m,1H), 2,38(s,3H), 2,20(s,3H), 1,42(d,6H) |
| 39 | 2,9 | 2,96 | (D6-DMSO, 400MHz) 7,92(s,1H), 7,77(d,1H), 7,45(d,1H); 4,90(s,2H), 3,90 (q,2H), 3H unter dem DMSO-Peak |
| 40 | 3,05 | 3,1 | (D6-DMSO, 400MHz) 7,86(s,1H), 7,59(s,1H), 7,30(s,1H), 4,88(s,2H), 3,87 (q,2H), 2,33(s,3H), 2,13(s,3H) |
| 41 | 4,1 | 4,18 | (D6-DMSO, 400MHz) 7,75(s,1H), 7,57(d,1H); 7,28(d,1H); 3,93(m,2H); 3,66 (d,2H); 2,39(s,3H); 2,09(s,3H); 2,09(m,1H); 0,9(d,6H) |
| 42 | 3,93 | 4,04 | (D6-DMSO, 400MHz) 7,87(s,1H), 7,80(d,1H), 7,42(d,1H), 7,39-7,27(m,5H), 5,01(s,2H), 3,92(q,2H), 2,45(s,3H) |
| 43 | 2,87 | 2,36 | (D6-DMSO, 400MHz) 8,59(s,1H), 8,51(dd,1H), 7,87(s,1H), 7,80-7,76(m,2H), 7,44-7,36(m,2H), 5,03(s,2H), 3,90(q,2H), 2,45(s,3H) |
| 44 | 1,96 | 2,02 | (D6-DMSO, 400MHz) 8,06(d,1H), 7,93(s,1H), 7,56(d,1H), 4,89(s,2H), 4,34-4,25(m.1H), 3,99-3,90(m,1H), 2,44(s,3H) |
| 45 | 2,9 | 3,00 | (D6-DMSO, 400MHz) 8,07(d,1H), 7,88(s,1H), 7,53(d,1H), 7,38-7,26(m,5H), 5,01(s,2H), 4,31-4,22(m,1H); 4,06-3,92(m,1H), 2,43(s,3H) |
| 46 | 2,66 | 2,72 | (D6-DMSO, 400MHz) 7,88(d,2H), 7,40(s,1H), 4,67(q,2H), 4,27-4,20(m,1H), 3,93-3,87(m,1H), 2,39(s,3H), 2,21(s,3H) |
| 47 | 2,66 | 2,72 | (D6-DMSO, 400MHz) 7,88(d,2H), 7,40(s,1H), 4,67(q,2H), 4,27-4,20(m,1H), 3,93-3,87(m,1H), 2,39(s,3H), 2,21(s,3H) |
| 48 | 1,45 | 1,51 | (D6-DMSO, 400MHz) 8,30(s,1H), 8,14(dd,1H), 8,07(d,1H), 7,89(s,1H), 7,53 (d,1H), 7,41-7,33(m,2H), 4,97(s,2H), 4,32-4,23(m,1H), 4,04-3,89(m,1H), 2,43(s,3H) |
| 49 | 2,00 | 1,47 | (D6-DMSO, 400MHz) 8,61(d,1H), 8,49(dd,1H), 8,07(d,1H), 7,88(s,1H); 7,80-7,77(m,1H), 7,53(d,1H), 7,37(dd,1H), 5,03(s,2H), 4,32-4,22(m,1H), 4,01-3,95(m,1H), 2,43(s,3H) |
| 50 | 2,57 | 2,62 | (D6-DMSO, 400MHz) 8,06(d,1H), 7,94(s,1H), 7,55(d,1H), 4,68(q,2H), 4,33-4,24(m,2H), 4,01-3,95(m,1H), 2,44(s,3H) |
| 51 | 2,57 | 2,62 | (D6-DMSO, 400MHz) 8,06(d,1H), 7,94(s,1H), 7,55(d,1H), 4,68(q,2H), 4,33-4,24(m,2H), 4,01-3,95(m,1H), 2,44(s,3H) |

(fortgesetzt)

| Bsp.Nr. | LogP[b] | LogP[a] | 1H-NMR δ ppm |
|---|---|---|---|
| 52 | 3,00 | 3,06 | (D6-DMSO, 400MHz) 7,89(s,1H), 7,82(s,1H), 7,38-7,26(m,6H), 5,02(s,2H), 4,25-4,16(m,1H), 3,93-3,87(m,1H), 2,38(s,3H), 2,19(s,3H) |
| 53 | 2,59 | 2,53 | (D6-DMSO, 400MHz) 7,77(d,1H), 7,74(d,1H), 7,41(d,1H), 5,88(d,1H), 3,98 (q,2H), 3,21(s,3H), 2,43(s,3H) |
| 54 | 1,66 | 1,61 | (D6-DMSO, 400MHz) 7,99(d,1H), 7,78(d,1H), 7,54(d,1H), 5,92(d,1H), 4,30-4,16(m,1H), 4,12-3,98(m,1H), 3,21(s,3H), 2,45(s,3H) |
| 55 | 2,13 | 2,06 | (D6-DMSO, 400MHz) 11,58(s,1H), 7,76(d,1H), 7,68(d,1H), 7,39(d,1H), 5,74-5,71(m,1H), 3,99(q,2H), 2,41(s,3H) |
| 56 | 1,3 | 1,24 | (D6-DMSO,400MHz) 11,62(s,1H), 7,98(d,1H), 7,74(d,1H), 7,52(d,1H), 5,79-5,76(m,1H), 4,29-4,16(m,1H), 4,13-4,01(m,1H), 2,44(s,3H) |
| 57 | 1,5 | 1,49 | (D6-DMSO, 400MHz) 7,87(d,1H), 7,80(d,1H), 7,61-7,58(m,1H), 7,49(d,1H), 5,88(d,1H), 4,30-4,12(m,1H), 4,09-3,95(m,1H), 3,21(s,3H), 2,42(s,3H) |
| 58 | 2,48 | 2,39 | (D6-DMSO, 400MHz) 7,71(d,1H), 7,61(d,1H), 7,37(d,1H), 7,27-7,24(m,1H), 5,83(d,1H), 4,04(q,2H), 3,20(s,3H), 2,38(s,3H) |
| 59 | 1,94 | 1,88 | (D6-DMSO, 400MHz) 8,00(d,1H), 7,77(d,1H), 7,53(d,1H), 5,90(d,1H), 4,30-4,12(m,1H), 4,12-3,99(m,1H), 3,87(q,2H), 2,45(s,3H), 1,13(t,3H) |
| 60 | 2,34 | 2,31 | (D6-DMSO, 400MHz) 8,04(d,1H), 7,88(d,1H), 7,55(d,1H), 6,02(d,1H), 4,68 (q,2H), 4,30-4,15(m,1H), 4,15-4,00(m,1H), 2,45(s,3H) |
| 61 | 1,73 | 1,72 | (D6-DMSO, 400MHz) 8,06(d,1H), 7,88(d,1H), 7,56(d,1H), 6,03(d,1H), 4,87 (s,2H), 4,32-4,18(m,1H), 4,10-3,95(m,1H), 2,45(s,3H) |
| 62 | 2,37 | 2,29 | (D6-DMSO, 400MHz) 8,01(d,1H), 7,79(d,1H), 7,53(d,1H), 5,93(d,1H), 4,30-4,15(m,1H), 4,15-4,00(m,1H), 3,73(d,2H), 2,45(s,3H), 1,21-1,10(m,1H), 0,49-0,40(m,2H), 0,35-0,30(m,2H) |
| 63 | 2,31 | 2,22 | (D6-DMSO, 400MHz) 7,99(d,1H), 7,73(d,1H), 7,52(d,1H), 5,85(d,1H), 5,10-5,00(m,1H), 4,30-4,15(m,1H), 4,15-4,00(m,1H), 2,45(s,3H), 1,39(d,6H) |
| 64 | 2,77 | 2,68 | (D6-DMSO, 400MHz) 8,03(d,1H), 7,83(d,1H), 7,53(d,1H), 7,38-7,35(m,2H), 7,18-7,13(m,2H), 5,97(d,1H), 5,00(s,2H), 4,30-4,15(m,1H), 4,15-3,99(m,1H), 2,44(s,3H) |
| 65 | 2,08 | 2,01 | (D6-DMSO, 400MHz) 8,02(d,1H), 7,81(d,1H), 7,53(d,1H), 5,94(d,1H), 5,90-5,80(m,1H), 5,18-5,08(m,2H), 4,44(d,2H), 4,29-4,15(m,1H), 4,15-4,00 (m,1H), 2,45(s,3H) |
| 66 | 2,25 | 2,17 | (D6-DMSO, 400MHz) 8,00(d,1H), 7,78(d,1H), 7,53(d,1H), 5,91(d,1H), 4,30-4,15(m,1H), 4,15-4,00(m,1H), 3,79(t,2H), 2,45(s,3H), 1,62-1,50(m,2H), 0,87(t,3H) |
| 67 | 1,92 | 1,86 | (D6-DMSO, 400MHz) 8,03(d,1H), 7,84(d,1H), 7,55(d,1H), 5,97(d,1H), 4,57 (d,2H), 4,30-4,15(m,1H), 4,15-4,00(m,1H), 3,16(t,1H), 2,45(s,3H) |
| 68 | 1,74 | 1,67 | (D6-DMSO, 400MHz) 8,03(d,1H), 7,82(d,1H), 7,54(d,1H), 5,94(d,1H), 5,22 (s,2H), 4,31-4,19(m,1H), 4,15-3,99(m,1H), 3,31(s,3H), 2,45(s,3H) |
| 69 | 2,55 | 2,48 | (D6-DMSO, 400MHz) 8,01(d,1H), 7,79(d,1H), 7,52(d,1H), 5,91(d,1H), 4,29-4,15(m,1H), 4,15-4,00(m,1H), 3,68(d,2H), 2,45(s,3H), 2,10-1,98(m,1H), 0,86(d,6H) |
| 70 | 2,11 | 2,06 | (D6-DMSO, 400MHz) 8,03(d,1H), 7,85(d,1H), 7,55(d,1H), 6,40-6,07(m,1H), 5,87(d,1H), 4,30-4,15(m,3H), 4,11-3,98(m,1H), 2,45(s,3H) |
| 71 | 2,93 | 2,86 | (D6-DMSO, 400MHz) 7,78(d,1H), 7,72(d,1H), 7,41(d,1H), 5,86(d,1H), 3,99 (q,2H), 3,86(q,2H), 2,42(s,3H), 1,12(t,3H) |

(fortgesetzt)

| Bsp.Nr. | LogP[b] | LogP[a] | 1H-NMR δ ppm |
|---|---|---|---|
| 72 | 3,29 | 3,16 | (D6-DMSO, 400MHz) 7,84(d,1H), 7,80(d,1H), 7,43(d,1H), 5,98(d,1H), 4,68 (q,2H), 4,00(q,2H), 2,43(s,3H) |
| 73 | 2,65 | 2,62 | (D6-DMSO, 400MHz) 7,85(d,1H), 7,82(d,1H), 7,45(d,1H), 6,01(d,1H), 4,88 (s,2H), 3,98(q,2H), 2,44(s,3H) |
| 74 | 3,38 | 3,24 | (D6-DMSO, 400MHz) 7,80(d,1H), 7,72(d,1H), 7,41(d,1H), 5,88(d,1H), 4,01 (q,2H), 3,73(d,2H), 2,42(s,3H), 1,19-1,10(m,1H), 0,49-0,40(m,2H), 0,36-0,28 (m,2H) |
| 75 | 3,35 | 3,21 | (D6-DMSO, 400MHz) 7,77(d,1H), 7,68(d,1H), 7,40(d,1H), 5,81(d,1H), 5,11-5,01(m,1H), 4,00(q,2H), 2,42(s,3H), 1,38(d,6H) |
| 76 | 3,74 | 3,56 | (D6-DMSO, 400MHz) 7,81(d,1H), 7,78(d,1H), 7,43-7,34(m,3H), 7,19-7,13 (m,2H), 5,93(d,1H), 5,00(s,2H), 3,99(q,2H), 2,42(s,3H) |
| 77 | 3,06 | 3,08 | (D6-DMSO, 400MHz) 7,79(d,1H), 7,76(d,1H), 7,41(d,1H), 5,89(d,1H), 5,88-5,78(m,1H), 5,15-5,08(m,2H), 4,43(d,2H), 4,00(q,2H), 2,42(s,3H) |
| 78 | 3,27 | 3,13 | (D6-DMSO, 400MHz) 7,78(d,1H), 7,72(d,1H), 7,41(d,1H), 5,87(d,1H), 3,99 (q,2H), 3,81-3,76(m,2H), 2,42(s,3H), 1,61-1,50(m,2H), 0,87(t,3H) |
| 79 | 2,81 | 2,78 | (D6-DMSO, 400MHz) 7,82-7,78(m,2H), 7,43(d,1H), 5,93(d,1H), 4,57(d,2H), 4,00(q,2H), 3,17(t,1H), 2,43(s,3H) |
| 80 | 2,66 | 2,62 | (D6-DMSO, 400MHz) 7,80(d,1H), 7,77(d,1H), 7,42(d,1H), 5,89(d,1H), 5,22 (s,2H), 4,00(q,2H), 3,31(s,3H), 2,43(s,3H) |
| 81 | 3,62 | 3,56 | (D6-DMSO, 400MHz) 7,78(d,1H), 7,73(d,1H), 7,40(d,1H), 5,87(d,1H), 4,00 (q,2H), 3,68(d,2H), 2,42(s,3H), 2,07-1,97(m,1H), 0,86(d,6H) |
| 82 | 3,06 | 2,99 | (D6-DMSO, 400MHz) 7,83-7,78(m,2H), 7,43(d,1H), 6,38-6,07(m,1H), 5,95 (d,1H), 4,32-4,23(m,2H), 3,99(q,2H), 2,43(s,3H) |
| 83 | 2,81 | 2,74 | (D6-DMSO, 400MHz) 7,70(d,1H), 7,61(d,1H), 7,37(d,1H), 7,29-7,25(m,1H), 5,81(d,1H), 4,05(q,2H), 3,87(q,2H), 1,18(t,3H), 1,13(t,3H) |
| 84 | 3,15 | 3,16 | (D6-DMSO, 400MHz) 7,81(d,1H), 7,63(d,1H), 7,39(d,1H), 7,30-7,26(m,1H), 5,92(d,1H), 4,67(q,2H), 4,05(q,2H), 2,39(s,3H) |
| 85 | 2,62 | 2,55 | (D6-DMSO, 400MHz) 7,82(d,1H), 7,65(d,1H), 7,40(d,1H), 7,31-7,28(m,1H), 5,94(d,1H), 4,86(s,2H), 4,04(q,2H), 2,39(s,3H) |
| 86 | 3,31 | 3,21 | (D6-DMSO, 400MHz) 7,72(d,1H), 7,62(d,1H), 7,37(d,1H), 7,30-7,26(m,1H), 5,83(d,1H), 4,05(q,2H), 3,72(d,2H), 2,38(s,3H), 1,19-1,12(m,1H), 0,50-0,42 (m,2H), 0,40-0,32(m,2H) |
| 87 | 3,25 | 3,18 | (D6-DMSO, 400MHz) 7,66(d,1H), 7,60(d,1H), 7,36(d,1H), 7,27-7,23(m,1H), 5,75(d,1H), 5,11-5,04(m,1H), 4,05(q,2H), 2,38(s,3H), 1,39(d,6H) |
| 88 | 2,05 | 1,99 | (D6-DMSO, 400MHz) 11,45(s,1H), 7,67(d,1H), 7,59(d,1H), 7,36(d,1H), 7,26-7,23(m,1H), 5,70-5,66(m,1H), 4,05(q,2H), 2,37(s,3H) |
| 89 | 2,25 | 2,18 | (D6-DMSO, 400MHz) 11,46(s,1H), 7,54(d,1H), 7,52(s,1H), 7,27(s,1H), 5,70-5,66(m,1H), 3,98(q,2H), 2,36(s,3H), 2,07(s,3H) |
| 90 | 2,21 | 2,18 | (D6-DMSO, 400MHz) 7,90(d,1H), 7,88(s,1H), 7,62-7,59(m,1H), 7,51(d,1H), 5,97(d,1H), 4,67(q,2H), 4,26-4,14(m,1H), 4,09-3,97(m,1H), 2,43(s,3H) |
| 91 | 1,19 | 1,20 | (D6-DMSO, 400MHz) 11,50(s,1H), 7,85(d,1H), 7,77(d,1H), 7,62-7,57(m,1H), 7,48(d,1H), 5,74(d,1H), 4,23-3,98(m,2H), 2,42(s,3H) |
| 92 | 2,72 | 2,66 | (D6-DMSO, 400MHz) 7,59(d,1H), 7,53(s,1H), 7,28(s,1H), 5,83(d,1H), 3,96 (q,2H), 3,21(s,3H), 2,37(s,3H), 2,07(s,3H) |

(fortgesetzt)

| Bsp.Nr. | LogP[b] | LogP[a] | 1H-NMR δ ppm |
|---|---|---|---|
| 93 | 1,7 | 1,68 | (D6-DMSO, 400MHz) 7,77(d,1H), 7,64(t,1H), 7,40,(d,1H), 5,90-5,84(m,1H), 4,28-3,89(m,2H), 3,21(d,3H), 2,39(s,3H), 2,17(s,3H) |
| 94 | 3,49 | 3,39 | (D6-DMSO, 400MHz) 7,70(d,1H), 7,56(s,1H), 7,29(s,1H), 5,93(d,1H), 4,76-4,60(m,2H), 3,97(q,2H), 2,37(s,3H), 2,06(s,3H) |
| 95 | 2,44 | 2,40 | (D6-DMSO, 400MHz) 7,81(d,1H), 7,75(t,1H), 7,42(d,1H), 6,00-5,93(m,1H), 4,74-4,61(m,2H), 4,29-3,90(m,2H), 2,40(s,3H), 2,17(s,3H) |
| 96 | 3,07 | 2,98 | (D6-DMSO, 400MHz) 7,58(d,1H), 7,54(s,1H), 7,28(s,1H), 5,81(d,1H), 4,02-3,91(m,2H), 3,90-3,81(m,2H), 2,37(s,3H), 2,06(s,3H), 1,13(t,3H) |
| 97 | 1,99 | 1,96 | (D6-DMSO, 400MHz) 7,77(d,1H), 7,65-7,61(m,1H), 7,40(d,1H), 5,89-5,82 (m,1H), 4,29-3,82(m,4H), 2,40(s,3H), 2,16(s,3H), 1,18-1,10(m,3H) |
| 98 | 1,77 | 1,77 | (D6-DMSO, 400MHz) 7,87(d,1H), 7,79(d,1H), 7,62-7,58(m,1H), 7,49(d,1H), 5,86(d,1H), 4,26-4,12(m,1H), 4,10-3,97(m,1H), 3,88(q,2H), 2,42(s,3H), 1,34 (t,3H) |
| 99 | 1,38 | 1,35 | (D6-DMSO, 400MHz) 11,52(s,1H), 7,75(d,1H), 7,62-7,57(m,1H), 7,39(d,1H), 5,76-5,69(m,1H), 4,28-3,93(m,2H), 2,39(s,3H), 2,17(s,3H) |
| 100 | 1,62 | 1,63 | (D6-DMSO, 400MHz) 7,93(d,1H), 7,89(d,1H), 7,64-7,60(m,1H), 7,51(d,1H), 5,98(d,1H), 4,86(s,2H), 4,27-4,15(m,1H), 4,07-3,95(m,1H), 2,43(s,3H) |

Die Bestimmung der Drehwerte erfolgte an einem Perkin Elmer 341, Seriennummer 9123, bei einer Wellenlänge von 589 nm und einer Temperatur von 20 °C, nach der nachfolgender Formel:

$$(spezifischer Drehwert \alpha)_{D}^{°C} = \frac{Drehwinkel \alpha * Lösungsvolumen(ml)}{Küvettenlänge(dm) * Einwaage(g)}$$

Die unten stehenden spezifischen Drehwerte sind als Durchschnitt aus 5 unterschiedlichen Messungen zu verstehen:

| | |
|---|---|
| 28 | -34,7 in $CHCl_3$ (c=0,009) |
| 29 | 33,7 in $CHCl_3$ (c=0,009) |
| 46 | -31,9 in $CHCl_3$ (c=0,009) |
| 47 | 31,9 in $CHCl_3$ (c=0,009) |
| 50 | -28,8 in $CHCl_3$ (c=0,009) |
| 51 | 28,2 in $CHCl_3$ (c=0,009) |

## Anwendungsbeispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe die unbeweglich auf dem Boden verbleiben oder sich unkoordiniert bewegen, gelten als tot bzw. angeschlagen.

Eine Substanz zeigt gute Wirkung gegen *Ctenocephalides felis*, wenn in diesem Test bei einer Aufwandmenge von 5 $\mu g/cm^2$ mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigten z.B. die folgenden Verbindungen der Herstellungsbeispiele eine Wirkung von 100% bei einer Aufwandmenge von 5 $\mu g/cm^3$ (= 500g/ha): 2

## Boophilus microplus -Injektionstest

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigten z.B. die folgenden Verbindungen der Herstellungsbeispiele eine Wirkung von 100% bei einer Aufwandmenge von 20µg/Tier: 2, 3, 4, 6, 9, 10, 11, 19, 20, 21, 22, 54, 29

## Meloidogyne incognita- Test

Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeitgen z. B. die folgenden Verbindungen der Herstellungsbeispiele Wirkung von 100% bei einer Aufwandmenge von 20ppm: 9, 10, 21, 25, 27, 30, 53, 54, 58, 59, 60, 61, 62, 63, 64, 65, 67, 68, 69, 70, 71, 72, 73, 74, 75, 76, 77, 78, 79, 80, 82, 84, 90, 95, 98, 100

Bei diesem Test zeigten z. B. die folgenden Verbindungen der Herstellungsbeispiele Wirkung von 90% bei einer Aufwandmenge von 20ppm: 2, 3, 8, 11, 12, 14, 15, 19, 20, 22, 39, 56, 57, 85, 86, 87, 92, 97

## Myzus persicae - Sprühtest

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 90% bei einer Aufwandmenge von 500g/ha: 32, 43, 44, 48, 49

**Phaedon cochleariae - Sprühtest**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100% bei einer Aufwandmenge von 500g/ha: 25, 27, 60, 79

Bei diesem Test zeigten z. B. die folgenden Verbindungen der Herstellungsbeispiele Wirkung von 83% bei einer Aufwandmenge von 500g/ha: 10

**Tetranychus urticae - Sprühtest, OP-resistent**

| | | |
|---|---|---|
| Lösungsmittel: | 78,0 | GewichtsteileAceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 100% bei einer Aufwandmenge von 500g/ha: 3, 4, 5, 9, 11, 14, 19, 20, 21, 22, 23, 24, 25, 26, 27, 30, 32, 33, 34, 35, 36, 37, 38, 39, 40, 42, 54, 55, 56, 84, 85, 86, 87, 89, 90, 91, 93, 94, 95, 96, 97, 98, 99

Bei diesem Test zeigten z. B. die folgenden Verbindungen der Herstellungsbeispiele eine Wirkung von 90% bei einer Aufwandmenge von 500g/ha: 2, 6, 7, 10, 12, 13, 16, 17, 18, 41, 43, 53, 58, 59, 61, 62, 63, 66, 68, 70, 71, 72, 73, 74, 76, 77, 78, 79, 81, 88, 92, 100

Bei diesem Test zeigten z. B. die folgenden Verbindungen der Herstellungsbeispiele eine Wirkung von 100% bei einer Aufwandmenge von 100g/ha: 31

Bei diesem Test zeigten z. B. die folgenden Verbindungen der Herstellungsbeispiele eine Wirkung von 90% bei einer Aufwandmenge von 100g/ha: 52, 64, 65, 75

Bei diesem Test zeigten z. B. die folgenden Verbindungen der Herstellungsbeispiele eine Wirkung von 90%

bei einer Aufwandmenge von 20g/ha: 67

**Meloidogyne incognita -Test**

| | | |
|---|---|---|
| Lösungsmittel: | 7 | Gewichtsteile Dimethylformamid |
| Emulgator: | 2,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration, wobei das Erdvolumen in das gedrencht wird, mitberücksichtigt werden muss. Es ist darauf zu achten, dass in der Erde eine Konzentration von 20 ppm Emulgator nicht überschritten wird. Zur Herstellung weiterer Testkonzentrationen wird mit Wasser verdünnt.

Mit Erde (lehmiger Sand) gefüllte Töpfe werden mit der Wirkstofflösung angegossen. Eine Ei-Larven-Suspension des südlichen Wurzelgallenälchens (*Meloidogyne incognita*) wird hinzugegeben, die Erdoberfläche mit Salatsamen bestreut und mit Quarzsand abgedeckt. Die Salatsamen keimen und die Pflänzchen entwickeln sich. An den Wurzeln entwickeln sich die Gallen.

Nach 21 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigten z. B. die folgenden Verbindungen der Herstellungsbeispiele Wirkung von 100% bei einer Aufwandmenge von 8ppm: 29

Bei diesem Test zeigten z. B. die folgenden Verbindungen der Herstellungsbeispiele Wirkung von 99% bei einer Aufwandmenge von 8ppm: 50

**Mvzus persicae - Sprühtest**

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigten z. B. die folgenden Verbindungen der Herstellungsbeispiele Wirkung von 97% bei einer Aufwandmenge von 20ppm: 31

**Tetranychus urticae- Sprühtest; OP-resistent**

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden

diese jeweils in einer Konzentration von 1000 ppm der Präparatelösung zugefügt.

Bohnenpflanzen (*Phaseolus vulgaris*), die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele Wirkung von 100% bei einer Aufwandmenge von 20ppm: 29, 47, 50, 60, 80, 82

Bei diesem Test zeigten z. B. die folgenden Verbindungen der Herstellungsbeispiele Wirkung von 95% bei einer Aufwandmenge von 20ppm: 44

Bei diesem Test zeigten z. B. die folgenden Verbindungen der Herstellungsbeispiele Wirkung von 90% bei einer Aufwandmenge von 20ppm: 45

## Patentansprüche

1. Verbindung der Formel (I)

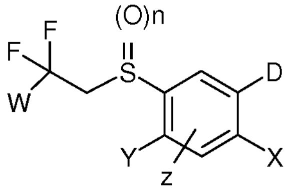

(I),

worin gilt:

D steht für eine Substruktur der Formel (I-A-1) oder (I-A-9)

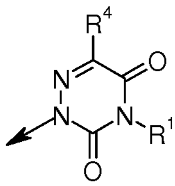

(I-A-1)

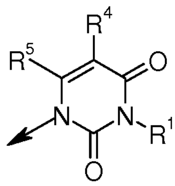

(I-A-9)

,

worin der Stickstoff mit dem Sechsring gemäß Formel (I) verbunden ist und der Pfeil die Bindung zu diesem Sechsring bedeutet,

$R^1$ steht für Wasserstoff;

oder für $(C_1-C_6)$Alkyl, $(C_3-C_8)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl, Phenyl$(C_1-C_3)$alkyl oder Pyridyl$(C_1-C_3)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl$(C_1-C_3)$alkyl vorhandene N-Atom auch als N-Oxid vorliegen kann;

oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridizanyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl vorhandene N-Atom auch als N-Oxid vorliegen kann;

$R^4$ und $R^5$ stehen unabhängig voneinander

für Wasserstoff, Halogen, $(C_1-C_4)$Alkyl, $(C_1-C_4)$Halogenalkyl, Cyano$(C_1-C_6)$alkyl, $(C_3-C_6)$Cycloalkyl, Cyano

oder Carboxyl;
$R^{11}$ steht für Wasserstoff, $(C_1-C_4)$Alkyl, $(C_1-C_4)$Halogenalkyl, Cyano$(C_1-C_6)$alkyl, oder $(C_3-C_6)$Cycloalkyl;
wobei $R^{11}$ insbesondere für Wasserstoff, Methyl, Ethyl, *tert*-Butyl, Trifluoromethyl, Methoxy, Trifluoromethoxy oder Cyclopropyl steht;
W steht für Wasserstoff oder Fluor;
n steht für die Zahl 0 oder 1;
X und Y stehen unabhängig voneinander für Wasserstoff, Fluor, Chlor, Brom, Methyl, Ethyl, Trifluoromethyl, Difluoromethyl, Difluoromethoxy, Trifluoromethoxy, Cyclopropyl, Cyano, Amino, Hydroxy oder Nitro;
Z steht für Wasserstoff.

**2.** Verbindung nach Anspruch 1, worin gilt:

D steht für eine Substruktur der Formel (I-A-1) oder (I-A-9);
$R^1$ steht für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, $CH_2CCH$, Cyclopropylmethyl, Trifluoroethyl, (2,2)-Difluoroethyl, $CH_2CN$, $CH_2OCH_3$, $CH_2CH_2OCH_3$, Benzyl, Cyclopropyl, Cyclobutyl, Phenyl, 2-Fluorophenylmethyl, 3-Fluorophenylmethyl, 4-Fluorophenylmethyl, 2-Pyridylmethyl, N-Oxid-2-pyridylmethyl, 3-Pyridylmethyl, N-Oxid-3-pyridylmethyl, 4-Pyridylmethyl oder N-Oxid-4-pyridylmethyl;
$R^4$ und $R^5$ stehen unabhängig voneinander
für Wasserstoff, Methyl, Ethyl, *tert*-Butyl, Trifluoromethyl, Methoxy, Trifluoromethoxy, Cyclopropyl, Cyano oder Carboxyl;
W steht für Wasserstoff oder Fluor;
n steht für die Zahl 0 oder 1;
X steht für Wasserstoff, Chlor, Fluor, Methyl oder Ethyl;
Y steht für Chlor, Brom, Cyano, Methyl, Ethyl, Trifluoromethyl, Fluor oder Methoxy;
Z steht für Wasserstoff.

**3.** Verbindung nach Anspruch 1, in welcher D für eine Substruktur der Formel (I-A-1) steht

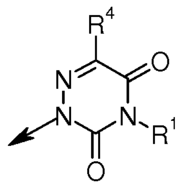

(I-A-1) ,

worin

$R^1$ für Wasserstoff steht;
oder für $(C_1-C_6)$Alkyl, $(C_3-C_8)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl, Phenyl$(C_1-C_3)$alkyl oder Pyridyl$(C_1-C_3)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl$(C_1-C_3)$alkyl vorhandene N-Atom auch als N-Oxid vorliegen kann;
oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridizanyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl vorhandene N-Atom auch als N-Oxid vorliegen kann;
$R^4$ für Wasserstoff, Halogen, $(C_1-C_4)$Alkyl, $(C_1-C_4)$Halogenalkyl, Cyano$(C_1-C_6)$alkyl, $(C_3-C_6)$Cycloalkyl, Cyano oder Carboxyl steht;

W für Wasserstoff oder Fluor steht;
n für die Zahl 0 oder 1 steht;
X und Y unabhängig voneinander
für Wasserstoff, Fluor, Chlor, Brom, Methyl, Ethyl, Trifluoromethyl, Difluoromethyl, Difluoromethoxy, Trifluoromethoxy, Cyclopropyl, Cyano, Amino, Hydroxy oder Nitro stehen;
Z für Wasserstoff steht.

**4.** Verbindung nach Anspruch 1, in welcher D für eine Substruktur der Formel (I-A-9) steht

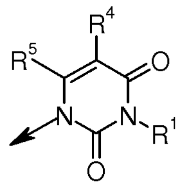

(I-A-9) ,

worin

$R^1$ für Wasserstoff steht;
oder für $(C_1-C_6)$Alkyl, $(C_3-C_8)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl, Phenyl$(C_1-C_3)$alkyl oder Pyridyl$(C_1-C_3)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl$(C_1-C_3)$alkyl vorhandene N-Atom auch als N-Oxid vorliegen kann;
oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridizanyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können und wobei das in Pyridyl vorhandene N-Atom auch als N-Oxid vorliegen kann;
$R^4$ und $R^5$ unabhängig voneinander
für Wasserstoff, Halogen, $(C_1-C_4)$Alkyl, $(C_1-C_4)$Halogenalkyl, Cyano$(C_1-C_6)$alkyl, $(C_3-C_6)$Cycloalkyl, Cyano oder Carboxyl stehen;
W für Wasserstoff oder Fluor steht;
n für die Zahl 0 oder 1 steht;
X und Y unabhängig voneinander
für Wasserstoff, Fluor, Chlor, Brom, Methyl, Ethyl, Trifluoromethyl, Difluoromethyl, Difluoromethoxy, Trifluoromethoxy, Cyclopropyl, Cyano, Amino, Hydroxy oder Nitro stehen;
Z für Wasserstoff steht.

**5.** Formulierung, insbesondere agrochemische Formulierung, umfassend mindestens eine Verbindung der Formel (I) gemäß einem der Ansprüche 1 bis 4.

**6.** Formulierung nach Anspruch 5 ferner umfassend mindestens ein Streckmittel und/oder mindestens eine oberflächenaktive Substanz.

**7.** Formulierung nach Anspruch 5 oder 6, **dadurch gekennzeichnet, dass** die Verbindung der Formel (I) in Mischung mit mindestens einem weiteren Wirkstoff vorliegt.

**8.** Verfahren zur Bekämpfung von Schädlingen, insbesondere tierischen Schädlingen, **dadurch gekennzeichnet, dass** man eine Verbindung der Formel (I) gemäß einem der Ansprüche 1 bis 4 oder eine Formulierung gemäß einem der Ansprüche 5 bis 7 auf die Schädlinge und/oder ihren Lebensraum einwirken lässt.

**9.** Verfahren nach Anspruch 8, **dadurch gekennzeichnet, dass** der Schädling ein tierischer Schädling ist und ein Insekt, einen Akariden oder einen Nematoden umfasst, oder dass der Schädling ein Insekt, ein Akaride oder ein Nematode ist.

**10.** Verwendung einer Verbindung der Formel (I) gemäß einem der Ansprüche 1 bis 4 oder einer Formulierung gemäß einem der Ansprüche 5 bis 7 zur Bekämpfung von tierischen Schädlingen.

**11.** Verwendung nach Anspruch 10, **dadurch gekennzeichnet, dass** der tierische Schädling ein Insekt, einen Akariden oder einen Nematoden umfasst, oder dass der tierische Schädling ein Insekt, ein Akaride oder ein Nematode ist.

**12.** Verwendung nach Anspruch 10 oder 11 im Pflanzenschutz.

**13.** Verwendung nach Anspruch 10 oder 11 auf dem Gebiet der Tiergesundheit.

**14.** Verfahren zum Schutz eines Saatgutes oder einer keimenden Pflanze vor Schädlingen, insbesondere tierischen Schädlingen, umfassend einen Verfahrensschritt, in welchem das Saatgut in Kontakt mit einer Verbindung der Formel (I) gemäß einem der Ansprüche 1 bis 4 oder mit einer Formulierung gemäß einem der Ansprüche 5 bis 7 gebracht wird.

**15.** Saatgut, erhalten durch ein Verfahren gemäß Anspruch 14.

**Claims**

**1.** Compound of the formula (I)

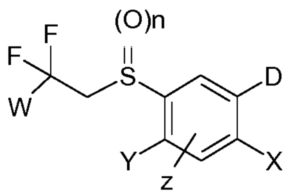

(I)

in which:

D is a substructure of the formula (I-A-1) or (I-A-9)

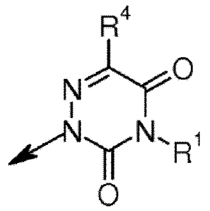

(I-A-1)

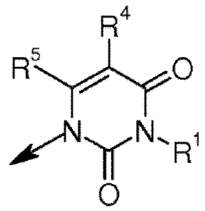

(I-A-9)

,

in which the nitrogen is bonded to the six-membered ring in formula (I) and the arrow represents the bond to this six-membered ring,
$R^1$ is hydrogen;
or $(C_1-C_6)$alkyl, $(C_3-C_8)$cycloalkyl $(C_1-C_3)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy $(C_1-C_6)$alkyl, phenyl$(C_1-C_3)$alkyl or pyridyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo$(C_1-C_6)$alkylsulphanyl, halo$(C_1-C_6)$alkylsulphonyl or optionally methyl-, fluorine-, chlorine-, cyano-substituted cyclopropyl, and where the nitrogen atom present in pyridyl $(C_1-C_3)$alkyl may also be in the form of the N-oxide; or $(C_3-C_6)$cycloalkyl, $(C_3-C_6)$cycloalkenyl, azetidinyl,

azolidinyl, azinanyl, oxetanyl, oxolanyl, oxanyl, thiethanyl, thiolanyl, thianyl, phenyl, pyridyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo $(C_1-C_6)$alkylsulphinyl, halo$(C_1-C_6)$alkylsulphanyl, halo $(C_1-C_6)$alkylsulphonyl or optionally methyl-, fluorine-, chlorine-, cyano-substituted cyclopropyl, and where the nitrogen atom present in pyridyl may also be in the form of the N-oxide;
$R^4$ and $R^5$ are each independently
hydrogen, halogen, $(C_1-C_4)$alkyl, $(C_1-C_4)$haloalkyl, cyano $(C_1-C_6)$alkyl, $(C_3-C_6)$cycloalkyl, cyano or carboxyl;
$R^{11}$ is hydrogen, $(C_1-C_4)$alkyl, $(C_1-C_4)$haloalkyl, cyano $(C_1-C_6)$alkyl or $(C_3-C_6)$cycloalkyl;
where $R^{11}$ is especially hydrogen, methyl, ethyl, *tert*-butyl, trifluoromethyl, methoxy, trifluoromethoxy or cyclopropyl;
W is hydrogen or fluorine;
n is the number 0 or 1;
X and Y are each independently hydrogen, fluorine, chlorine, bromine, methyl, ethyl, trifluoromethyl, difluoromethyl, difluoromethoxy, trifluoromethoxy, cyclopropyl, cyano, amino, hydroxyl or nitro;
Z is hydrogen.

**2.** Compound according to Claim 1, in which:

D is a substructure of the formula (I-A-1) or (I-A-9)
$R^1$ is hydrogen, methyl, ethyl, n-propyl, $CH(CH_3)_2$, n-butyl, sec-butyl, $C(CH_3)_3$, $CH_2C(CH_3)_3$, $CH_2CH(CH_3)_2$, $CH_2CH=CH_2$, $CH_2CCH$, cyclopropylmethyl, trifluoroethyl, 2,2-difluoroethyl, $CH_2CN$, $CH_2OCH_3$, $CH_2CH_2OCH_3$, benzyl, cyclopropyl, cyclobutyl, phenyl, 2-fluorophenylmethyl, 3-fluorophenylmethyl, 4-fluorophenylmethyl, 2-pyridylmethyl, N-oxide-2-pyridylmethyl, 3-pyridylmethyl, N-oxide-3-pyridylmethyl, 4-pyridylmethyl or N-oxide-4-pyridylmethyl;
$R^4$ and $R^5$ are each independently
hydrogen, methyl, ethyl, tert-butyl, trifluoromethyl, methoxy, trifluoromethoxy, cyclopropyl, cyano or carboxyl;
W is hydrogen or fluorine;
n is the number 0 or 1;
X is hydrogen, chlorine, fluorine, methyl or ethyl;
Y is chlorine, bromine, cyano, methyl, ethyl, trifluoromethyl, fluorine or methoxy;
Z is hydrogen.

**3.** Compound according to Claim 1, in which D is a substructure of the formula (I-A-1)

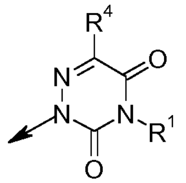

(I-A-1) ,

in which

$R^1$ is hydrogen;
or $(C_1-C_6)$alkyl, $(C_3-C_8)$cycloalkyl $(C_1-C_3)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy$(C_1-C_6)$alkyl, phenyl$(C_1-C_3)$alkyl or pyridyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo$(C_1-C_6)$alkylsulphanyl, halo$(C_1-C_6)$alkylsulphonyl or optionally methyl-, fluorine-, chlorine-, cyano-substituted cyclopropyl, and where the nitrogen atom present in pyridyl$(C_1-C_3)$alkyl may also be in the form of the N-oxide;
or $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyridyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by

halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl, and where the nitrogen atom present in pyridyl may also be in the form of the N-oxide;
$R^4$ is hydrogen, halogen, $(C_1-C_4)$alkyl, $(C_1-C_4)$ haloalkyl, cyano $(C_1-C_6)$alkyl, $(C_3-C_6)$cycloalkyl, cyano or carboxyl;
W is hydrogen or fluorine;
n is the number 0 or 1;
X and Y are each independently
hydrogen, fluorine, chlorine, bromine, methyl, ethyl, trifluoromethyl, difluoromethyl, difluoromethoxy, trifluoromethoxy, cyclopropyl, cyano, amino, hydroxyl or nitro;
Z is hydrogen.

**4.** Compound according to Claim 1, in which D is a substructure of the formula (I-A-9)

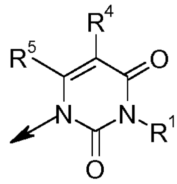

(I-A-9) ,

in which

$R^1$ is hydrogen;
or $(C_1-C_6)$alkyl, $(C_3-C_8)$cycloalkyl $(C_1-C_3)$alkyl, $(C_2-C_6)$alkenyl, $(C_2-C_6)$alkynyl, halo$(C_1-C_6)$alkyl, cyano $(C_1-C_6)$alkyl, $(C_1-C_6)$alkoxy $(C_1-C_6)$alkyl, phenyl $(C_1-C_3)$alkyl or pyridyl $(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl, and where the nitrogen atom present in pyridyl $(C_1-C_3)$alkyl may also be in the form of the N-oxide;
or $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyridyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl, and where the nitrogen atom present in pyridyl may also be in the form of the N-oxide;
$R^4$ and $R^5$ are each independently
hydrogen, halogen, $(C_1-C_4)$alkyl, $(C_1-C_4)$haloalkyl, cyano $(C_1-C_6)$alkyl, $(C_3-C_6)$cycloalkyl, cyano or carboxyl;
W is hydrogen or fluorine;
n is the number 0 or 1;
X and Y are each independently
hydrogen, fluorine, chlorine, bromine, methyl, ethyl, trifluoromethyl, difluoromethyl, difluoromethoxy, trifluoromethoxy, cyclopropyl, cyano, amino, hydroxyl or nitro;
Z is hydrogen.

**5.** Formulation, especially agrochemical formulation, comprising at least one compound of the formula (I) according to any of Claims 1 to 4.

**6.** Formulation according to Claim 5, further comprising at least one extender and/or at least one surface-active substance.

**7.** Formulation according to Claim 5 or 6, **characterized in that** the compound of the formula (I) is in a mixture with at least one further active ingredient.

**8.** Method for controlling pests, especially animal pests, **characterized in that** a compound of the formula (I) according to any of Claims 1 to 4 or a formulation according to any of Claims 5 to 7 is allowed to act on the pests and/or their habitat.

**9.** Method according to Claim 8, **characterized in that** the pest is an animal pest and comprises an insect, an acarid or a nematode, or **in that** the pest is an insect, an acarid or a nematode.

**10.** Use of a compound of the formula (I) according to any of Claims 1 to 4 or of a formulation according to any of Claims 5 to 7 for controlling animal pests.

**11.** Method according to Claim 10, **characterized in that** the animal pest comprises an insect, an acarid or a nematode, or **in that** the animal pest is an insect, an acarid or a nematode.

**12.** Use according to Claim 10 or 11 in crop protection.

**13.** Use according to Claim 10 or 11 in the field of animal health.

**14.** Method for protecting seed or a germinating plant from pests, especially animal pests, comprising a method step in which the seed is contacted with a compound of the formula (I) according to any of Claims 1 to 4 or with a formulation according to any of Claims 5 to 7.

**15.** Seed obtained by a method according to Claim 14.

## Revendications

**1.** Composé de formule (I)

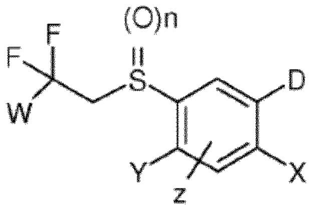

(I),

dans laquelle

D représente une sous-structure de formule (I-A-1) ou (I-A-9)

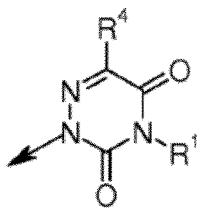

(I-A-1)

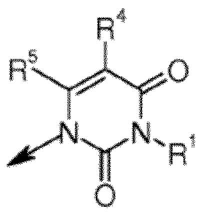

(I-A-9)

dans lesquelles l'azote est relié avec le cycle à six chaînons selon la formule (I) et la flèche signifie la liaison à ce cycle à six chaînons,
$R^1$ représente hydrogène ;
ou alkyle en ($C_1$-$C_6$), cycloalkyle en ($C_3$-$C_8$)-alkyle en ($C_1$-$C_3$), alcényle en ($C_2$-$C_6$), alcynyle en ($C_2$-$C_6$), halogéno-alkyle en ($C_1$-$C_6$), cyano-alkyle en ($C_1$-$C_6$), alcoxy en ($C_1$-$C_6$)-alkyle en ($C_1$-$C_6$), phényl-alkyle en ($C_1$-$C_3$) ou pyridyl-alkyle en ($C_1$-$C_3$), les radicaux susmentionnés pouvant chacun éventuellement être substitués une fois à trois fois par halogène, cyano, nitro, hydroxy, amino, alkyle en ($C_1$-$C_6$), halogéno-alkyle en ($C_1$-$C_6$), alcoxy

en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyl-alkyle en ($C_1$-$C_3$) pouvant également se présenter sous la forme d'un N-oxyde ;

ou cycloalkyle en ($C_3$-$C_6$), cycloalcényle en ($C_3$-$C_6$), azétidinyle, azolidinyle, azinanyle, oxétanyle, oxolanyle, oxanyle, thiéthanyle, thiolanyle, thianyle, phényle, pyridyle, pyrimidyle, pyridizanyle, pyrazinyle, triazinyle, thiazolyle, thiazadiazolyle, oxazolyle, oxadiazolyle, pyrazolyle, triazolyle, les radicaux susmentionnés pouvant chacun être substitués une fois à trois fois par halogène, cyano, nitro, hydroxy, amino, alkyle en ($C_1$-$C_6$), halogéno-alkyle en ($C_1$-$C_6$), alcoxy en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyle pouvant également se présenter sous la forme d'un N-oxyde ;

$R^4$ et $R^5$ représentent indépendamment l'un de l'autre

hydrogène, halogène, alkyle en ($C_1$-$C_4$), halogénoalkyle en ($C_1$-$C_4$), cyano-alkyle en ($C_1$-$C_6$), cycloalkyle en ($C_3$-$C_6$), cyano ou carboxyle ;

$R^{11}$ représente hydrogène, alkyle en ($C_1$-$C_4$), halogénoalkyle en ($C_1$-$C_4$), cyano-alkyle en ($C_1$-$C_6$)ou cycloalkyle en ($C_3$-$C_6$);

$R^{11}$ représentant notamment hydrogène, méthyle, éthyle, tert-butyle, trifluorométhyle, méthoxy, trifluorométhoxy ou cyclopropyle ;

W représente hydrogène ou fluor ;

n représente le nombre 0 ou 1 ;

X et Y représentent indépendamment l'un de l'autre hydrogène, fluor, chlore, brome, méthyle, éthyle, trifluorométhyle, difluorométhyle, difluorométhoxy, trifluorométhoxy, cyclopropyle, cyano, amino, hydroxy ou nitro ;

Z représente hydrogène.

**2.** Composé selon la revendication 1, dans lequel :

D représente une sous-structure de formule (I-A-1) ou (I-A-9) ;

$R^1$ représente hydrogène, méthyle, éthyle, n-propyle, $CH(CH_3)_2$, n-butyle, sec-butyle, $C(CH_3)_3$, $CH_2C(CH_3)_3$, $CH_2CH(CH_3)_2$, $CH_2CH=CH_2$, $CH_2CCH$, cyclopropylméthyle, trifluoroéthyle, (2,2)-difluoroéthyle, $CH_2CN$, $CH_2OCH_3$, $CH_2CH_2OCH_3$, benzyle, cyclopropyle, cyclobutyle, phényle, 2-fluorophénylméthyle, 3-fluorophénylméthyle, 4-fluorophénylméthyle, 2-pyridylméthyle, N-oxyde-2-pyridylméthyle, 3-pyridylméthyle, N-oxyde-3-pyridylméthyle, 4-pyridylméthyle ou N-oxyde-4-pyridylméthyle;

$R^4$ et $R^5$ représentent indépendamment l'un de l'autre

hydrogène, méthyle, éthyle, tert-butyle, trifluorométhyle, méthoxy, trifluorométhoxy, cyclopropyle, cyano ou carboxyle ;

W représente hydrogène ou fluor ;

n représente le nombre 0 ou 1 ;

X représente hydrogène, chlore, fluor, méthyle ou éthyle ;

Y représente chlore, brome, cyano, méthyle, éthyle, trifluorométhyle, fluor ou méthoxy ;

Z représente hydrogène.

**3.** Composé selon la revendication 1, dans lequel D représente une sous-structure de formule (I-A-1)

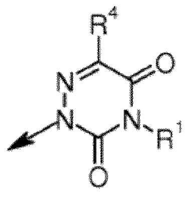

(I-A-1)

dans laquelle

$R^1$ représente hydrogène ;

ou alkyle en ($C_1$-$C_6$), cycloalkyle en ($C_3$-$C_8$)-alkyle en ($C_1$-$C_3$), alcényle en ($C_2$-$C_6$), alcynyle en ($C_2$-$C_6$), halogéno-alkyle en ($C_1$-$C_6$), cyano-alkyle en ($C_1$-$C_6$), alcoxy en ($C_1$-$C_6$)-alkyle en ($C_1$-$C_6$), phényl-alkyle en ($C_1$-$C_3$)

ou pyridyl-alkyle en ($C_1$-$C_3$), les radicaux susmentionnés pouvant chacun éventuellement être substitués une fois à trois fois par halogène, cyano, nitro, hydroxy, amino, alkyle en ($C_1$-$C_6$), halogéno-alkyle en ($C_1$-$C_6$), alcoxy en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyl-alkyle en ($C_1$-$C_3$) pouvant également se présenter sous la forme d'un N-oxyde ;

ou cycloalkyle en ($C_3$-$C_6$), cycloalcényle en ($C_3$-$C_6$), azétidinyle, azolidinyle, azinanyle, oxétanyle, oxolanyle, oxanyle, thiéthanyle, thiolanyle, thianyle, phényle, pyridyle, pyrimidyle, pyridizanyle, pyrazinyle, triazinyle, thiazolyle, thiazadiazolyle, oxazolyle, oxadiazolyle, pyrazolyle, triazolyle, les radicaux susmentionnés pouvant chacun être substitués une fois à trois fois par halogène, cyano, nitro, hydroxy, amino, alkyle en ($C_1$-$C_6$), halogéno-alkyle en ($C_1$-$C_6$), alcoxy en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyle pouvant également se présenter sous la forme d'un N-oxyde ;

$R^4$ représente hydrogène, halogène, alkyle en ($C_1$-$C_4$), halogénoalkyle en ($C_1$-$C_4$), cyano-alkyle en ($C_1$-$C_6$), cycloalkyle en ($C_3$-$C_6$), cyano ou carboxyle ;

W représente hydrogène ou fluor ;

n représente le nombre 0 ou 1 ;

X et Y représentent indépendamment l'un de l'autre

hydrogène, fluor, chlore, brome, méthyle, éthyle, trifluorométhyle, difluorométhyle, difluorométhoxy, trifluorométhoxy, cyclopropyle, cyano, amino, hydroxy ou nitro ;

Z représente hydrogène.

**4.** Composé selon la revendication 1, dans lequel D représente une sous-structure de formule (I-A-9)

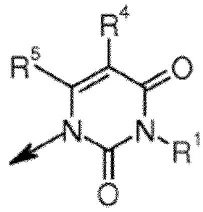

(I-A-9)

dans laquelle

$R^1$ représente hydrogène ;

ou alkyle en ($C_1$-$C_6$), cycloalkyle en ($C_3$-$C_8$)-alkyle en ($C_1$-$C_3$), alcényle en ($C_2$-$C_6$), alcynyle en ($C_2$-$C_6$), halogéno-alkyle en ($C_1$-$C_6$), cyano-alkyle en ($C_1$-$C_6$), alcoxy en ($C_1$-$C_6$)-alkyle en ($C_1$-$C_6$), phényl-alkyle en ($C_1$-$C_3$) ou pyridyl-alkyle en ($C_1$-$C_3$), les radicaux susmentionnés pouvant chacun éventuellement être substitués une fois à trois fois par halogène, cyano, nitro, hydroxy, amino, alkyle en ($C_1$-$C_6$), halogéno-alkyle en ($C_1$-$C_6$), alcoxy en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyl-alkyle en ($C_1$-$C_3$) pouvant également se présenter sous la forme d'un N-oxyde ;

ou cycloalkyle en ($C_3$-$C_6$), cycloalcényle en ($C_3$-$C_6$), azétidinyle, azolidinyle, azinanyle, oxétanyle, oxolanyle, oxanyle, thiéthanyle, thiolanyle, thianyle, phényle, pyridyle, pyrimidyle, pyridizanyle, pyrazinyle, triazinyle, thiazolyle, thiazadiazolyle, oxazolyle, oxadiazolyle, pyrazolyle, triazolyle, les radicaux susmentionnés pouvant chacun être substitués une fois à trois fois par halogène, cyano, nitro, hydroxy, amino, alkyle en ($C_1$-$C_6$), halogéno-alkyle en ($C_1$-$C_6$), alcoxy en ($C_1$-$C_6$), halogéno-alcoxy en ($C_1$-$C_6$), alkylsulfinyle en ($C_1$-$C_6$), alkylsulfanyle en ($C_1$-$C_6$), alkylsulfonyle en ($C_1$-$C_6$), halo-alkylsulfinyle en ($C_1$-$C_6$), halo-alkylsulfanyle en ($C_1$-$C_6$), halo-alkylsulfonyle en ($C_1$-$C_6$), ou cyclopropyle éventuellement substitué par méthyle, fluor, chlore, cyano, et l'atome N présent dans le pyridyle pouvant également se présenter sous la forme d'un N-oxyde ;

$R^4$ et $R^5$ représentent indépendament l'un de l'autre

hydrogène, halogène, alkyle en ($C_1$-$C_4$), halogénoalkyle en ($C_1$-$C_4$), cyano-alkyle en ($C_1$-$C_6$), cycloalkyle en ($C_3$-$C_6$), cyano ou carboxyle ;

W représente hydrogène ou fluor ;

n représente le nombre 0 ou 1 ;
X et Y représentent indépendamment l'un de l'autre
hydrogène, fluor, chlore, brome, méthyle, éthyle, trifluorométhyle, difluorométhyle, difluorométhoxy, trifluorométhoxy, cyclopropyle, cyano, amino, hydroxy ou nitro ;
Z représente hydrogène.

**5.** Formulation, notamment formulation agrochimique, comprenant au moins un composé de formule (I) selon l'une quelconque des revendications 1 à 4.

**6.** Formulation selon la revendication 5, comprenant en outre au moins un extendeur et/ou au moins une substance tensioactive.

**7.** Formulation selon la revendication 5 ou 6, **caractérisée en ce que** le composé de formule (I) se présente en mélange avec au moins un autre agent actif.

**8.** Procédé de lutte contre des nuisibles, notamment des animaux nuisibles, **caractérisé en ce qu'**un composé de formule (I) selon l'une quelconque des revendications 1 à 4 ou une formulation selon l'une quelconque des revendications 5 à 7 est laissé agir sur les nuisibles et/ou leur habitat.

**9.** Procédé selon la revendication 8, **caractérisé en ce que** le nuisible est un animal nuisible, et comprend un insecte, un acarien ou un nématode, ou **en ce que** le nuisible est un insecte, un acarien ou un nématode.

**10.** Utilisation d'un composé de formule (I) selon l'une quelconque des revendications 1 à 4 ou d'une formulation selon l'une quelconque des revendications 5 à 7 pour lutter contre des animaux nuisibles.

**11.** Utilisation selon la revendication 10, **caractérisée en ce que** l'animal nuisible comprend un insecte, un acarien ou un nématode, ou **en ce que** l'animal nuisible est un insecte, un acarien ou un nématode.

**12.** Utilisation selon la revendication 10 ou 11, dans la protection des plantes.

**13.** Utilisation selon la revendication 10 ou 11, dans le domaine de la santé animale.

**14.** Procédé de protection d'une semence ou d'une plante germée contre des nuisibles, notamment des animaux nuisibles, comprenant une étape de procédé lors de laquelle la semence est mise en contact avec un composé de formule (I) selon l'une quelconque des revendications 1 à 4 ou avec une formulation selon l'une quelconque des revendications 5 à 7.

**15.** Semence, obtenue par un procédé selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 8600072 A
- WO 9730980 A
- WO 2005112941 A
- EP 1803712 A1
- WO 9959983 A
- DE 2543497
- DE 2724673
- GB 2021098 A
- DE 19528305
- DE 19536842
- DE 4431218
- WO 2009012275 A
- WO 2008155034 A
- WO 2007131680 A
- WO 2008086226 A
- WO 2006117657 A1
- US 201099725 A1
- WO 201047956 A1
- US 6673810 B
- WO 9800072 A
- US 7015230 B
- WO 2003042191 A
- WO 2003011841 A
- US 20040072746 A
- WO 2009039127 A
- WO 2013074633 A
- US 3007927 A
- DE 2554866
- WO 2000026194 A
- JP 2007284356 A
- JP 2011042611 A
- WO 2007034755 A
- JP 2007081019 A
- JP 2007284385 A
- JP 2008260706 A
- JP 2008308448 A
- JP 2009023910 A
- WO 2012176856 A
- WO 2006043635 A
- DE 1248665
- WO 9401102 A
- DE 2725148
- WO 2005077934 A
- WO 2007115644 A
- WO 2007115643 A
- WO 2007115646 A
- EP 0539588 A
- WO 2007149134 A
- WO 2010074747 A
- WO 2010074751 A
- WO 2006089633 A
- WO 2008067911 A
- WO 2008066153 A
- WO 2006056433 A
- WO 2006100288 A
- WO 2005035486 A
- WO 2007057407 A
- WO 2008104503 A
- WO 2003106457 A
- WO 2009049851 A
- WO 2004099160 A
- WO 2005063094 A
- WO 2007040280 A
- JP 2010018586 A
- WO 2007075459 A
- WO 2005085216 A
- WO 2010005692 A
- WO 2002096882 A
- WO 2007101369 A
- WO 2010006713 A
- WO 2010069502 A
- WO 2008009360 A
- CN 102057925
- WO 2011049233 A
- WO 2012034472 A
- US 4272417 A
- US 4245432 A
- US 4808430 A
- US 5876739 A
- US 20030176428 A1
- WO 2002080675 A1
- WO 2002028186 A2
- EP OS0313512 A
- DE 102005045174 A
- DE 102005022994 A
- WO 2006122662 A
- EP 1731037 A
- WO 2006015697 A
- DE 4128828 A
- DE 1905834 A
- DE 19631764 A

**In der Beschreibung aufgeführte Nicht-Patentliteratur**


- *Chem. Sci.,* 2010, vol. 1, 13-31
- *Tetrahedron Lett.,* 2009, vol. 50, 7293-7296
- *Synthesis,* 2001, vol. 6, 829-856
- *Synthesis,* 2011, 829-856
- *Tetrahedron,* 2012, vol. 68, 7735-7754
- *Monatshefte Chem.,* 1968, vol. 99 (3), 1009-1013
- *Coll. Czech. Chem. Comm.,* 1979, vol. 44 (8), 2438-2442
- *Pharmazie,* 1980, vol. 35 (12), 744-745
- *Heterocycles,* 1978, vol. 9 (10), 1387-1390
- *J. Med. Chem.,* 1979, vol. 22 (12), 1483-1487
- *Liebigs Ann. Chem.,* 1982, vol. 1, 182-185
- *Tetrahedron Lett.,* 1970, vol. 45, 3957-3960
- *Synthesis,* 2001, vol. 2, 239-242
- *Synthesis,* 1993, 1079-1080
- *J. Amer. Chem. Soc.,* 2011, vol. 133 (41), 16418-16421
- *J. Heterocyclic Chem.,* 1999, vol. 36, 293-295
- *Angew. Chem.,* 1977, vol. 89, 789-796
- *Tetrahedron,* 2006, vol. 62, 906-914
- *Nucleosides Nucleotides,* 1995, vol. 14, 2039-2049
- *J. Org. Chem.,* 2005, vol. 70 (20), 7925-7335
- *Tetrahedron,* 2007, vol. 63, 2859-2864
- *J. Amer. Chem. Soc.,* 1956, 1938-1941
- *Chem. Pharm. Bull.,* 1962, vol. 10, 647-652
- **A.R. MAGUIRE.** *ARKIVOC,* 2011, 1-110
- **H. SUZUKI.** *Chem. Let.,* 1985, vol. 3, 411-412
- **S. L. BUCHWALD.** *J. Amer. Chem. Soc.,* 2002, vol. 124 (50), 14844-14845
- **E. B. MERKUSHEV.** *Synthesis,* 1988, vol. 12, 923-937
- *J. Med. Chem.,* 2003, vol. 46, 4405-4418
- *Chem. Berichte,* 1947, vol. 180, 494-502
- *Bull. Soc. Chim. Fr.,* 1959, 1793-1798
- *Coll. Czech. Chem. Comm.,* 1961, vol. 26, 986-997
- *J. Amer. Chem. Soc.,* 1938-1941
- *Chem. Pharm. Bull,* 1962, vol. 10, 647-652
- *J. Biol. Chem.,* 1925, vol. 65, 469-477
- *Compt. Rend.,* 1959, vol. 248, 3444-3446
- *J. Org. Chem.,* 1972, vol. 11, 11738-11742
- *J. Org. Chem.,* 1977, vol. 12, 2185-2187
- *J. Heterocycl. Chem.,* 1972, vol. 9 (5), 1175-1176
- *J. Heterocycl. Chem.,* 1972, vol. 9 (6), 1423
- *Can. J. Chem.,* 1978, vol. 56 (5), 725-729
- *Synthese nach J. Amer. Chem. Soc.,* 1963, vol. 85, 749-754
- *Synthese nach Phosp., Sulf., Sil. and rel. Elem.,* 1996, vol. 119, 161-168
- Manual on development and use of FAO and WHO specifications for pesticides, FAO Plant Production and Protection Papers - 173, prepared by the FAO/WHO Joint Meeting on Pesticide Specifications. Crop Life International und in Pesticide Specifications, 2004
- **BAUR et al.** *Pesticide Science,* 1997, vol. 51, 131-152
- The Pesticide Manual. British Crop Protection Council, 2006
- *CHEMICAL ABSTRACTS,* 1204776-60-2
- **R. WEGLER.** Chemie der Pflanzenschutz- und Schädlingsbekämpfungsmittel. Springer Verlag, 1970, vol. 2, 401-412
- **HANS W. HELDT.** Pflanzenbiochemie. Spektrum Akademischer Verlag, 1996, 393-462
- Biochemistry and Molecular Biology of Plants. American Society of Plant Physiologists, 2000, 1102-1203
- **DANGL ; JONES.** *Nature,* 2001, vol. 411, 826-833
- **KESSLER ; BALDWIN.** *Annual Review of Plant Biology,* 2003, vol. 53, 299-328
- **PENNAZIO.** *New Microbiol,* 1995, vol. 18, 229-240
- **RYALS et al.** *The Plant Cell,* 1996, vol. 8, 1809-1819
- Biochemistry and Molecular Biology of Plants. American Society of Plant Physiologists, 2000, 850-929
- **SEMBDNER ; PARTHIER.** *Ann. Rev. Plant Physiol. Plant Mol. Biol.,* 1993, vol. 44, 569-589
- **ACHUO et al.** *Plant Pathology,* 2004, vol. 53 (1), 65-72
- **TAMBLYN et al.** *Pesticide Science,* 1999, vol. 55 (6), 676-677
- **WALLING.** *J. Plant Growth Regul.,* 2000, vol. 19, 195-216
- **BROWN et al.** *Beltwide Cotton Conference Proceedings,* 2004, 2231-2237
- **GONIAS et al.** *Beltwide Cotton Conference Proceedings,* 2004, 2225-2229
- *Crop Protection,* 2000, vol. 19 (5), 349-354
- *Journal of Entomological Science,* 2002, vol. 37 (1), 101-112
- *Annals of Biology,* 2003, vol. 19 (2), 179-181
- *Thielert Pflanzenschutz-Nachrichten Bayer,* 2006, vol. 59 (1), 73-86
- **FRANCIS et al.** *European Journal of Plant Pathology,* 23. Januar 2009
- **LEAL, R. S.** The use of Confidor S in the float , a new tobacco seedlings production system in the South of Brazil. *Pflanzenschutz-Nachrichten Bayer,* 2001, vol. 54 (3), 337-352

- **RUDOLPH, R. D. ; ROGERS, W. D.** The efficacy of imidacloprid treatment for reduction in the severity of insect vectored virus diseases of tobacco. *Pflanzenschutz-Nachrichten Bayer,* 2001, vol. 54 (3), 311-336
- Ullmann's Encyclopedia of Industrial Chemistry. Verlagsgesellschaft, 1987, vol. A 10, 323-431
- Ullmann's Encyclopedia of Industrial Chemistry. 1987, vol. A 10, 363-401
- *CHEMICAL ABSTRACTS,* 925982-34-9